# FASCICULE DE BREVET EUROPEEN

(11) **EP 2 111 454 B1**
(45) Date de publication et mention de la délivrance du brevet: **16.09.2015**
(21) Numéro de dépôt: 08708214.5
(22) Date de dépôt: 25.01.2008
(51) Int. Cl.: C12N 15/81

(54) **LEVURES GÉNÉTIQUEMENT MODIFIÉES POUR LA PRODUCTION DE GLYCOPROTÉINES HOMOGÈNES**
GENTECHNISCH VERÄNDERTE HEFEN ZUR HERSTELLUNG HOMOGENER GLYKOPROTEINE
GENETICALLY MODIFIED YEASTS FOR THE PRODUCTION OF HOMOGENEOUS GLYCOPROTEINS

(30) Priorité: 02.02.2007 FR 0753050
(43) Date de publication de la demande: 28.10.2009
(73) Titulaire: Société Industrielle Limousine d'Application Biologique (SILAB), 19130 Objat (FR)
(72) Inventeur: JAVAUD, Christophe, 19100 BRIVE (FR); CARRE, Vincent, F-87370 Jabreilles-les-Bordes (FR)
(74) Mandataire: Aquinov
(86) Numéro de dépôt international: PCT/EP2008/050888
(87) Numéro de publication internationale: WO 2008/095797

(56) Documents cités:
- WO-A-2004/003194
- WO-A-2005/049807
- VERVECKEN W ET AL: "IN VIVO SYNTHESIS OF MAMMALIAN-LIKE, HYBRID-TYPE N-GLYCANS IN PICHIA PASTORIS" APPLIED AND ENVIRONMENTAL MICROBIOLOGY, WASHINGTON,DC, US, vol. 70, no. 5, mai 2004 (2004-05), pages 2639-2646, XP008032109 ISSN: 0099-2240
- CHIBA YASUNORI ET AL: "Production of human compatible high mannose-type (Man5GlcNAc2) sugar chains in Saccharomyces cerevisiae" JOURNAL OF BIOLOGICAL CHEMISTRY, AMERICAN SOCIETY OF BIOLOCHEMICAL BIOLOGISTS, BIRMINGHAM,, US, vol. 273, no. 41, 9 octobre 1998 (1998-10-09), pages 26298-26304, XP002202331 ISSN: 0021-9258
- HAMILTON STEPHEN R ET AL: "Humanization of yeast to produce complex terminally sialylated glycoproteins" SCIENCE, AMERICAN ASSOCIATION FOR THE ADVANCEMENT OF SCIENCE,, US, vol. 313, no. 5792, septembre 2006 (2006-09), pages 1441-1443, XP002457470 ISSN: 0036-8075
- HAMILTON S R ET AL: "Production of complex human glycoproteins in yeast" SCIENCE, AMERICAN ASSOCIATION FOR THE ADVANCEMENT OF SCIENCE,, US, vol. 301, no. 5637, 29 août 2003 (2003-08-29), pages 1244-1246, XP002267832 ISSN: 0036-8075
- SUIHKO M-L ET AL: "CONSTRUCTION AND ANALYSIS OF RECOMBINANT GLUCANOLYTIC BREWER'S YEAST STRAINS" APPLIED MICROBIOLOGY AND BIOTECHNOLOGY, SPRINGER VERLAG, BERLIN, DE, vol. 35, no. 6, septembre 1991 (1991-09), pages 781-787, XP000982895 ISSN: 0175-7598
- TAXIS CHRISTOF; KNOP MICHAEL: "System of centromeric, episomal, and integrative vectors based on drug resistance markers for Saccharomyces cerevisiae" BIOTECHNIQUES, vol. 40, no. 1, janvier 2006 (2006-01), pages 73-78, XP001537899

## Description

La présente invention concerne des levures *Saccharomyces cerevisiae* et *Schizosaccharomyces pombe* génétiquement modifiées pour la production de glycoprotéines possédant des structures glycaniques optimisées et homogènes. Ces levures comprennent une inactivation du gène Ochl, l'intégration par recombinaison homologue dans un marqueur d'auxotrophie d'une cassette d'expression comprenant un premier promoteur, et une phase ouverte de lecture comprenant la séquence codante pour une α-1-2 mannosidase I, et l'intégration d'une cassette comprenant un deuxième promoteur différent dudit premier promoteur et la séquence codante d'une glycoprotéine exogène. Ces levures permettent de produire de l'EPO avec une glycosylation optimisée et homogène à 98%.

La production de glycoprotéines ou glycopeptides présentant des glycanes du type complexe, c'est à dire des structures identiques aux oligosaccharides ajoutés lors des modifications post-traductionnelles chez l'homme, est un objectif recherché depuis quelques années dans l'industrie pharmaceutique. En effet, de nombreuses études ont montré l'importance des oligosaccharides pour optimiser l'activité des glycoprotéines thérapeutiques ou encore pour améliorer leur temps de demi-vie une fois administrées.

Par exemple, l'érythropoiétine humaine (HuEPO) est une glycoprotéine de 166 acides aminés contenant trois sites de N-glycosylation aux positions Asn-24, Asn-38 et Asn-83 et un site de 0-glycosylation de type mucine à la position Ser-126. L'EPO est un modèle particulièrement pertinent pour l'étude de la N-glycosylation de part ses structures glycosylées représentant 40% de sa masse moléculaire. La molécule d'EPO considérée comme naturelle est la forme urinaire (uHuEPO) (Takeuchi et al 1988, Tsuda 1988 Rahbek-nielsen 1997). L'EPO recombinante (rHuEPO) est actuellement produite dans les cellules CHO (sasaki H et al, takeuchi et al, 1988) ou dans les cellules BHK (nimtz et al 1993). La rHuEPO exprimées dans les lignées cellulaires présentent des structures N-glycaniques différentes des structures retrouvées dans la uHuEPO. Ces différences peuvent avoir une répercussion *in vitro* (Higuchi et al, 1992 ; Takeuchi et al, 1990) mais semblent plus sensibles *in vivo* par une perte d'activité drastique pour les formes déglycosylées et une augmentation d'activité corrélée au nombre d'acides sialiques présents sur la structure (Higuchi M et al, 1992).

Pour produire des glycoprotéines présentant une N- ou O-glycosylation optimale, de nombreuses solutions techniques ont été proposées. On peut citer la modification in vitro des structures glycaniques par addition de sucres tel le galactose, le glucose, le fucose ou encore l'acide sialique au moyen de différentes glycosyl-transférases ou par suppression de certains sucres tel le mannose par des mannosidases. Cette technique est décrite dans WO 03/031464 (Neose). On peut cependant se demander comment une telle technique peut être mise en oeuvre à grande échelle car cela implique de nombreuses étapes pour une modification séquentielle de plusieurs oligosaccharides donnés présents sur une même glycoprotéine. A chaque étape, un contrôle strict de la réaction doit être effectué et il faut s'assurer d'une production de structures glycaniques homogènes. Or, dans le cas où il faut modifier plusieurs oligosaccharides sur une glycoprotéine, une réaction séquentielle peut aboutir à des modifications non souhaitées et hétérogènes. Cette technique n'est donc pas compatible avec la préparation de biomédicaments. En outre, l'utilisation d'enzymes purifiés pour une production à l'échelle industrielle n'apparaît pas représenter une alternative économique viable.

Il en est de même avec les techniques de couplage chimiques, comme celles décrites dans les documents WO 2006/106348 et WO 2005/000862. Ces techniques de couplage chimique impliquent des réactions fastidieuses, des étapes de protection / dé-protection, de multiples contrôles. Dans le cas où il faut modifier plusieurs oligosaccharides sur une glycoprotéine donnée, une réaction séquentielle peut également aboutir à des modifications non souhaitées et hétérogènes.

D'autres technologies utilisant des lignées cellulaires de mammifères telles que YB2/0 décrites dans WO 01/77181 (LFB) ou encore CHO génétiquement modifié dans WO 03/055993 (Kyowa) ont démontré qu'une une faible fucosylation de la région Fc des anticorps améliore l'activité ADCC d'un facteur 100. Cependant, ces technologies se rapportent spécifiquement à la production d'anticorps.

Enfin, il a été proposé de produire des glycoprotéines chez la levure ou les champignons filamenteux par transformation de ces micro-organismes avec des plasmides permettant l'expression de mannosidases et de différentes glycosyl transférases. Cette approche a été décrite dans WO 02/00879 (Glycofi) et dans WO 2005/049807 (Research Corporation. Technologies). Cependant, à ce jour, il n'a pas été démontré que ces micro-organismes sont stables dans le temps en fermenteur à haute capacité pour la fabrication de lots cliniques. De même, il n'est pas montré que cette transformation permet de produire des glycoprotéines avec les glycanes souhaités et homogènes.

Dans le but de produire de la rHuEPO possédant des structures N-glycaniques permettant d'obtenir l'activité optimale *in vivo,* nous avons exprimé une rHuEPO dans des levures S.cerevisiae et S.pombe génétiquement modifiées. Ces levures ont montrées une forte expression de la rHuEPO présentant des motifs de N-glycosylation homogènes et bien caractérisés. Dans un second temps, nous sommes partie de ces levures génétiquement modifiées et nous avons incorporées d'autres modifications de sorte à produire des motifs N-glycaniques plus complexes suivant leurs degrés de sialylation. Le système levure est connu pour sa capacité à produire rapidement de grande quantité de protéines mais les levures modifiées décrites ci-après sont capables également de *N*-glycosyler de façon « humanisée » et homogène les protéines produites. En outre, ces levures se sont avérées stables en condition de production à grande échelle. Enfin, en cas de mutations conduisant à une réversion génotypique, ces levures sont construites de sorte à permettre une restauration à l'identique, ce qui est requis dans le cadre de la production de lots cliniques.

Il s'agit donc du premier exemple illustrant les méthodes d'intégration ciblées dans les locus particuliers qui ont été utilisées pour l'ensemble de l'invention, méthodes permettant un contrôle au nucléotide près de régions génomiques interrompues et sélectionnées, et autorisant par conséquent une restauration de l'interruption en cas de réversion génomique spontanée. Cette méthode est à opposer à celle décrite dans WO 02/00879, consistant à transformer une souche de levure avec une banque de séquences et à sélectionner par la suite le meilleur clone sans aucune caractérisation génomique.

En effet, dans WO 02/00879 les intégrations sont aléatoires et les clones sont sélectionnés uniquement sur la base du profil de structures N-glycanniques des protéines produites, ce qui implique en cas de mutation, réversion ou tout autre modification génétique la perte pure et simple du clone d'intérêt. L'avantage de la technologie selon l'invention, est de procurer une sécurité accrue à l'utilisateur, en lui fournissant une garantie de contrôle, de traçabilité des modifications génétiques, et surtout la possibilité de reconstruire un clone qui possèdera rigoureusement les mêmes aptitudes.

En outre, nous apportons pour la première fois une technologie de "Glycan-on-Demand" à partir de la souche Amélie décrite ci-après. Dans ces conditions l'homogénéité des structures est plus importantes que dans des systèmes CHO qui glycosylent comme les mammifères. En effet, les résultats obtenus (voir spectre EPO), font état d'une structure glycannique du type Man5GlcNAc2 représentant environ 98% des N-glycannes présents sur la protéine. Le système est donc conçu pour forcer la glycosylation afin d'obtenir un motif désiré en très grande proportion.

La souche Amélie est le clone servant de base à l'élaboration de toute autre souche destinée à produire des glycannes humanisés, hybrides ou complexes, que l'on souhaite obtenir. L'avantage de cette souche est de constituer un point de départ, qui a été démontré comme étant système stable et homogène produisant 98% de glyprotéines Man5GlcNAc2, pouvant être repris pour des modifications supplémentaires comme l'introduction d'une GIcNAc transférase, d'une fucosyltransférase, d'une galactosyl et/ou sialyl transférase, à la demande, rapidement, selon les structures finales souhaitées.

### Description de l'invention

La construction d'une cassette d'expression s'effectue en intégrant une séquence promotrice en 5' et une séquence terminale en 3' de l'ORF. D'autre part, l'intégration de ces cassettes dans le génome des levures est contrôlée par l'ajout aux extrémités de séquences homologues au locus cible dans le but d'une intégration par recombinaison homologue.

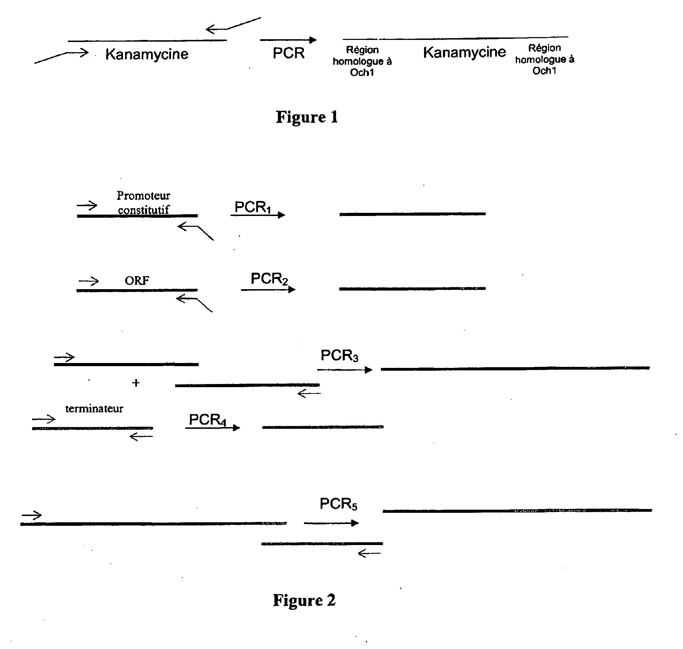

Pour chaque souche et pour chaque ORF, les séquences promotrices ainsi que les séquences d'intégration ont été déterminées dans le but d'obtenir une expression stable et optimale des différentes enzymes permettant un glycosylation homogène des glycoprotéines. La construction d'une cassette d'expression se fait en plusieurs étapes successives de PCR, suivant ce modèle général montré à la figure 2 *(PCR d'assemblage pour construction des cassettes d'expression des ORFs*).

Certaines séquences d'ORF ont été partiellement modifiées en intégrant des signaux de localisation sub-cellulaire afin d'exprimer (d'adresser) la protéine dans un compartiment où son activité sera optimale (environnement, présence du donneur et des substrats etc...).

Ainsi, dans un premier aspect, la présente invention concerne des levures *Saccharomyces cerevisiae* et *Schizosaccharomyces pombe* génétiquement modifiées, capables de produire des glycoprotéines présentant des glycanes homogènes présentant la strucuture Man5GlcNAc2, lesdites levures comprenant les modifications suivantes:
a) inactivation du gène Ochl codant pour l'a1,6 mannosyltransférase par insertion par recombinaison homologue d'une séquence hétérologue codant pour un gène de résistance à une antibiotique (la Kanamycine) (souche delta-Ochl),
b) Intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, une phase ouverte de lecture comprenant la séquence codante pour une α-1-2 mannosidase I comprenant une séquence de ciblage dans le réticulum endoplasmique ou l'appareil de Golgi et un terminateur de la transcription,
c) Intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae ou S. pombe, et ADH2 possédant la séquence SEQ ID No 16-26 respectivement, ledit promoteur en c) étant différent du promoteur en b); une phase ouverte de lecture comprenant la séquence codante pour glycoprotéine exogène à produire et un terminateur de la transcription.

De préférence, les levures décrites ci-dessus ont intégré l'α-1-2 mannosidase I de C. Elegans, notamment une séquence comprenant la SEQ ID No 1. Ces levures se sont montrées capables de produire des glycoprotéines possédant 98% de glycanes **Man5GlcNac2 :**

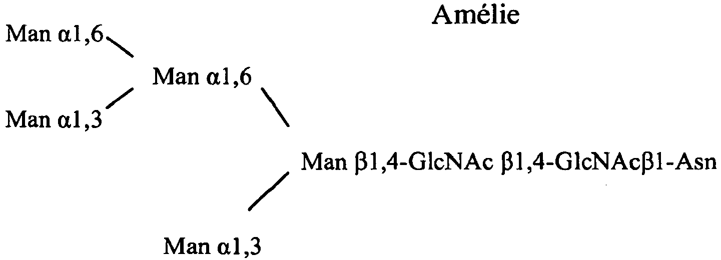

Avantageusement, la α-1-2 mannosidase I est exprimée sous le contrôle du promoteur pGAP et la glycoprotéine protéine exogène est exprimée sous le contrôle du promoteur pGAL1.

Pour la suite de la description il sera fait référence aux abréviations employées dans l'état de la technique Man = mannose, GlcNac = N-acétyl-glucosamine, Gal = galactose, Fuc = fucose et NANA désignant l'acide sialique ou encore le N-acétyl-neuraminique acide.

Dans un deuxième aspect, les levures de l'invention comportent des modifications supplémentaires en vue de produire des glycoprotéines présentant plus de 75%, voir 80% ou encore 95% ou 98% de la structure **GlcNacMan5GlcNAc2 :**

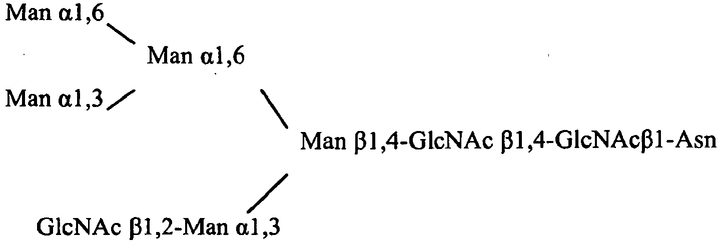

A cet effet, les souches ci-dessus comprennent en outre l'intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, une phase ouverte de lecture comprenant la séquence codante pour la N-acétyl-glucosaminyl transférase I humaine comprenant une séquence de ciblage dans le reticulum endoplasmique ou l'appareil de Golgi et un terminateur de la transcription. De préférence, la N-acétyl-glucosaminyl transférase I humaine comprend la séquence SEQ ID No 2 sans la partie cytoplasmique de l'enzyme qui est remplacée par la partie cytoplasmique de Mnn9 pour une localisation golgienne de la protéine. Cette souche est désignée « **Arielle »** Arielle doit aussi contenir la cassette Transporteur UDP GIcNAc (décrite plus bas) pour synthétiser ce type de glycanne. Avantageusement, on utilise le promoteur pGAP.
Mnn9
**Atgtcactttctcttgtatcg**taccgcctaa**gaaagaacccgtgggttaac**: **partie cytoplasmique (SEQ ID No 13).**

La souche Amélie ci-dessus peut comprendre en outre l'intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, une phase ouverte de lecture comprenant la séquence codante pour la pour le transporteur d'UDP-GlcNAc humain et un terminateur de la transcription. De préférence, le transporteur d'UDP-GIcNAc humain comprend la séquence SEQ ID No 3. De préférence, le promoteur est PGK. Cette souche est désignée ci-après **"Agathe".**

Dans un troisième aspect, les levures de l'invention comportent des modifications supplémentaires en vue de produire des glycoprotéines présentant plus de 75%, voir 80% ou encore 95% ou 98% de la structure **GlcNacMan3GlcNAc2 :**

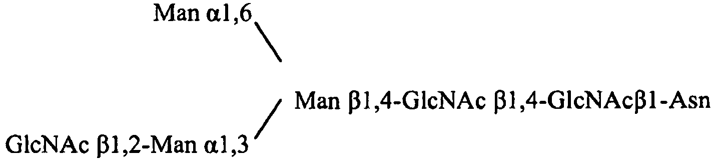

A ce titre, les levures Arielle mentionnées ci-dessus comprennent une intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, une phase ouverte de lecture comprenant la séquence codante pour une mannosidase II comprenant une séquence de ciblage dans le reticulum endoplasmique ou l'appareil de Golgi et un terminateur de la transcription De préférence, la mannosidase II est celle de la souris, notamment une séquence comprenant la SEQ ID No 4. De préférence, le promoteur est TEF. Cette souche est désignée ci-après **"Anaïs".**

Dans un quatrième aspect, les levures de l'invention comportent des modifications supplémentaires en vue de produire des glycoprotéines présentant plus de 75%, voir 80% ou encore 95% ou 98% de la structure GlcNac2Man3GlcNAc2 :

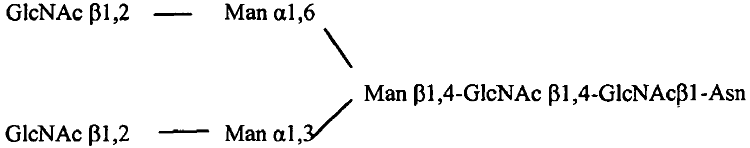

Dans ce cas, les levures Anaïs mentionnées ci-dessus comprennent une intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, une phase ouverte de lecture comprenant la séquence codante pour une *N*-acetyl-glucosaminyl transférase II, comprenant une séquence de ciblage dans le reticulum endoplasmique ou l'appareil de Golgi et un terminateur de la transcription. De préférence, la *N*-acetyl-glucosaminyl transférase II est humaine, notamment une séquence comprenant la SEQ ID No 5. De préférence, le promoteur est PMA1. Cette souche est désignée ci-après **"Alice".**

Dans un autre mode de réalisation, les levures Alice de l'invention comportent des modifications supplémentaires en vue de produire des glycoprotéines présentant plus de 75%, voir 80% ou encore 95% ou 98% de la structure Gal2GlcNac2Man3GlcNAc2 :

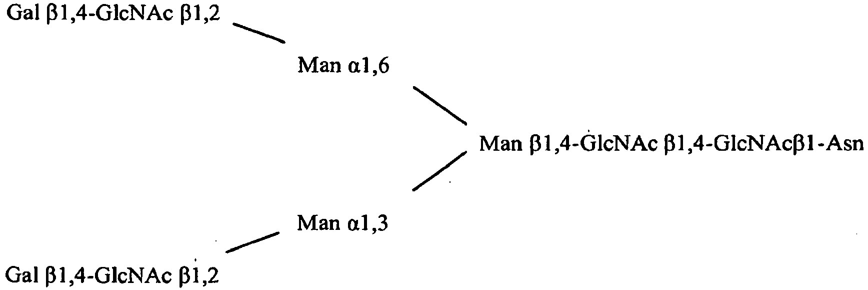

Dans ce cas, les levures Alice mentionnées ci-dessus comprennent une intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement, de préférence le promoteur CaMV, une phase ouverte de lecture comprenant la séquence codante pour une galactosyl transférase I, comprenant une séquence de ciblage dans le reticulum endoplasmique ou l'appareil de Golgi et un terminateur de la transcription. De préférence, la galactosyl transférase I est humaine, notamment une séquence comprenant la SEQ ID No 6, qui est dépourvue de la séquence de ciblage humaine. Cette souche est désignée Athena.

Avantageusement, l'intégration des cassettes d'expression précitées est effectuée dans un marqueur (d'auxotrophie sélectionné parmi) d'intégration sélectionné parmi URA3, ADE2, LYS2, LEU2, TRP1, CAN1, ADO1, HIS5, HIS3, ARG3, MET17, LEM3, Mnn1, Mnn9, gma12. De manière encore plus avantageuse, la cassette d'expression de la α-1-2 mannosidase I est intégrée dans le gène URA3, la cassette d'expression de la N-acétyl-glucosaminyl transférase I est intégrée dans le gène ADE1 ou ADE2, la cassette d'expression du transporteur d'UDP-GIcNAc est intégrée dans le gène LYS2, la cassette d'expression de l'α-mannosidase II est intégrée dans le gène LEU2, et la cassette d'expression de la N-acetyl-glucosaminyl transférase II est intégrée dans le gène Lem3 ou TRP1. La cassette d'expression de l'β1,4 galactosyltransférase I est intègrée dans TRP1 ou MET 17.

De plus, on utilise de préférence dans les constructions une séquence de ciblage dans le reticulum endoplasmique ou l'appareil de Golgi dérivée de la séquence de localisation du gène Mnt1 qui comprend la séquence SEQ ID No 14 et le terminateur CYC1 comprenant la SEQ ID No 15.

Dans un autre mode de réalisation, les levures Alice et Athena, décrites ci-dessus de l'invention comportent des modifications supplémentaires en vue de produire des glycoprotéines présentant plus de 75%, voir 80% ou encore 95% ou 98% d'une structure choisie parmi
GlcNac2Man3GlcNAc2,
Gal2GlcNac2Man3GlcNAc2,
et
GlcNac2Man3(Fuc)GlcNAc2, souche **Ashley**
Gal2GlcNac2Man3(Fuc)GlcNAc2, Souche **Aurel**

Dans ce cas, les levures mentionnées ci-dessus comprennent une intégration par recombinaison homologue dans un marqueur d'auxotrophie d'une cassette d'expression comprenant un promoteur selectionné parmi pGAP, pGAL1, PGK, TEF, adh1, nmt1, SV40, PMA1, CaMV, pet56 de S. cerevisiae or S. pombe, et ADH2 possédant la séquence SEQ ID No 16-26 respectivement ou le promoteur du gène nmt1, une phase ouverte de lecture comprenant la séquence codante pour une α-1,6-fucosyltransférase FUT8, comprenant une séquence de ciblage dans le reticulum endoplasmique ou l'appareil de Golgi et un terminateur de la transcription, en particulier le terminateur dérivé du gène CYC1. Ces souches peuvent avantageusement contenir la cassette correspondant au transporteur de GDP-fucose décrite plus bas. Cette cassette peut être intégrée dans CAN1 ou HIS5.

De préférence, la α-1,6-fucosyltransférase FUT8 est humaine, notamment une séquence comprenant la SEQ ID No 7.
En outre, cette souche, doit comprendre une intégration par recombinaison homologue dans un marqueur d'auxotrophie d'une cassette d'expression comprenant le promoteur SV40, une phase ouverte de lecture comprenant la séquence codante pour un transporteur de GDP-fucose, notamment une séquence comprenant la SEQ ID No 8. Cette cassette peut être intégrée dans TRP1, ARG3 ou gma12.

Dans un autre mode de réalisation, les levures Gal2GlcNac2Man3GlcNAc2 **(Athena)** et Gal2GlcNac2Man3(Fuc)GlcNAc2 **(Aurel)** décrites ci-dessus de l'invention comportent des modifications supplémentaires en vue de produire des glycoprotéines présentant plus de 75%, voir 80% ou encore 95% ou 98% d'une structure choisie parmi
NANA2Gal2GlcNac2Man3GlcNAc2 souche Aeron
NANA2Gal2GlcNac2Man3(Fuc)GlcNAc2 souche Avrel

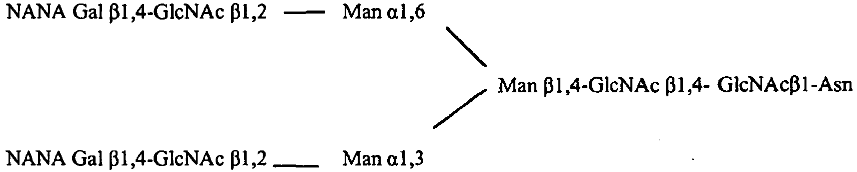

Dans ce mode de réalisation, les levures mentionnées ci-dessus comprennent une intégration par recombinaison homologue dans un marqueur d'auxotrophie d'une cassette d'expression comprenant un promoteur parmi ceux cités ci-dessus ou le promoteur de la thymidine kinase du virus de l'herpès comprenant la séquence SEQ ID No 9.

Une phase ouverte de lecture comprenant la séquence codante pour une α-2,3 sialyltransférase (gène ST3GAL4) et un terminateur de la transcription, en particulier le terminateur dérivé du gène CYC1 comprenant la séquence SEQ ID No 15. De préférence, la sialyltransférase est humaine (NM_006278), notamment une séquence comprenant la SEQ ID No 10.

Dans un autre mode de réalisation, les levures
Gal2GlcNac2Man3GlcNAc2 **(Athena)** et NANA2Gal2GlcNac2Man3GlcNAc2 **(Aeron)**
décrites ci-dessus de l'invention comportent des modifications supplémentaires en vue de produire des glycoprotéines présentant plus de 75%, voir 80% ou encore 95% ou 98% d'une structure choisie parmi
Gal2GlcNac3Man3GlcNAc2 souche Azalée
NANA2Gal2GlcNac3Man3GlcNAc2 souche A

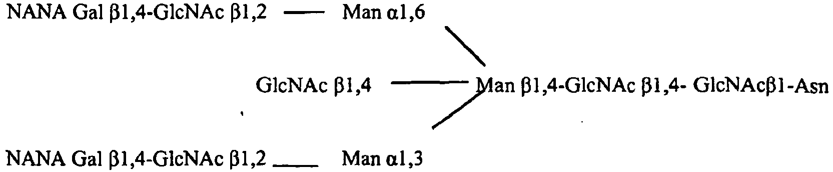

Dans ce mode de réalisation, les levures mentionnées ci-dessus comprennent une intégration par recombinaison homologue dans un marqueur d'auxotrophie d'une cassette d'expression comprenant un promoteur parmi ceux cités ci-dessus.

Une phase ouverte de lecture comprenant la séquence codante pour une β-1,4 N-acetylglucosaminyltransférase III et un terminateur de la transcription, en particulier le terminateur dérivé du gène CYC1 comprenant la séquence SEQ ID No 15. De préférence, la GNTIII est murine, notamment une séquence comprenant la SEQ ID No 27.

Comme indiqué ci-dessus, les levures selon l'invention ont intégrées une cassette pour exprimer une glycoprotéine ou un glycopeptide exogène. La glycoprotéine peut être choisie parmi les glycoprotéines à usage thérapeutique telles que des cytokines, des interleukines, des hormones de croissance, facteurs de croissance, des enzymes, et des anticorps monoclonaux, protéines vaccinales, récepteurs solubles et tous types de protéines recombinantes. Il peut s'agir d'une séquence codant pour l'EPO, notamment une cassette comprenant la SEQ ID No 11 codant pour une EPO ayant la SEQ ID No 12 comprenant l'épitope V5 et un motif poly-HIS en N-terminal pour purification.

L'invention concerne également une composition pharmaceutique comprenant une glycoprotéine possédant des structures glycanniques homogènes à plus de 75%, 90%, 95% ou encore 98% de la structure :
Man5GlcNAc2,
GlcNacMan5GlcNAc2,
GlcNacMan3GlcNAc2,
GlcNac2Man3GlcNAc2.
Gal2GlcNac2Man3GlcNAc2
NANA2Gal2GlcNac2Man3GlcNAc2
GlcNac2Man3(Fuc)GlcNAc2
Gal2GlcNac2Man3(Fuc)GlcNAc2
NANA2Gal2GlcNac2Man3(Fuc)GlcNAc2
Gal2GlcNac3Man3GlcNAc2
NANA2Gal2GlcNac3Man3GlcNAc2

L'invention concerne également une composition pharmaceutique comprenant de l'EPO à titre de principe actif, ledit EPO présentant plus de 75%, 90%, 95% ou encore 98% de la structure
NANA2Gal2GlcNac2Man3GlcNAc2 ou
NANA2Gal2GlcNac2Man3(Fuc)GlcNAc2

L'invention se rapporte également à une culture en fermenteur comprenant un milieu de culture de base des milieux de culture pour levures et une levure décrite ci-dessus.

Dans encore un autre aspect, l'invention porte sur un procédé de production d'une glycoprotéine possédant des structures glycanniques homogènes à plus de 75%, 90%, 95% ou encore 98%, de la structure :
Man5GlcNAc2,
GlcNacMan5GlcNAc2,
GlcNacMan3GlcNAc2,
GlcNac2Man3GlcNAc2.
Gal2GlcNac2Man3GlcNAc2
NANA2Gal2GlcNac2Man3GlcNAc2
GlcNac2Man3(Fuc)GlcNAc2
Gal2GlcNac2Man3(Fuc)GlcNAc2
NANA2Gal2GlcNac2Man3(Fuc)GlcNAc2
Gal2GlcNac3Man3GlcNAc2
NANA2Gal2GlcNac3Man3GlcNAc2
comprenant la mise en culture d'une levure décrite dessus dans un fermenteur, et l'extraction de ladite glycoprotéine du milieu de culture. Ce procédé peut comprendre en outre une étape de purification.

Enfin, l'invention porte également sur l'utilisation d'une levure décrite ci-dessus pour la production en fermenteur d'une glycoprotéine possédant des structures glycanniques homogènes à plus de 75%, 90%, 95% ou encore 98% de la structure :
Man5GlcNAc2,
GlcNacMan5GlcNAc2,
GlcNacMan3GlcNAc2,
GlcNac2Man3GlcNAc2.
Gal2GlcNac2Man3GlcNAc2
NANA2Gal2GlcNac2Man3GlcNAc2
GlcNac2Man3(Fuc)GlcNAc2
Gal2GlcNac2Man3(Fuc)GlcNAc2
NANA2Gal2GlcNac2Man3(Fuc)GlcNAc2
Gal2GlcNac3Man3GlcNAc2
NANA2Gal2GlcNac3Man3GlcNAc2

### Exemple 1 : Création des souches mutées sur le gène Och1 codant pour l'α-1,6 mannosyl transférase (souche delta-Och1).

Le gène de résistance de la kanamycine a été amplifié par PCR et des régions flanquantes homologues au gène Och1 ont été ajoutées en ces deux extrémités **(****Figure1****),** régions spécifiques de chaque souche de levure S.cerevisae ou S.pombe. Le gène Och1 est rendu non-fonctionnel par l'insertion de ce gène de résistance à un antibiotique, la Kanamycine. L'intégration du gène dans le génome de la levure se fait par électroporation et le gène d'intérêt s'intègre ensuite par recombinaison homologue. Les régions flanquantes font une quarantaine de bases et permettent l'intégration du gène de la kanamycine au sein du gène Och1 dans le génome de la levure.

Les souches ayant intégrées le gène de résistance à la kanamycine sont sélectionnées sur un milieu contenant 50µg/ml de kanamycine. Nous avons ensuite vérifié par PCR l'intégration du gène de résistance à la kanamycine dans le gène Och1. L'ADN génomique des clones ayant résistés à la présence de kanamycine dans le milieu, a été extrait. Des oligonucléotides ont été choisis de façon à vérifier d'une part la présence du gène de résistance à la kanamycine et d'autre part que ce gène a bien été intégré dans le gène Och1. L'ADN génomique des souches sauvages a également été testé, nous avons amplifié le gène Och1 de ces souches. Ce gène fait 1100pb. Chez les souches ayant intégrées la cassette kanamycine, l'amplification observée du gène Och1 est plus longue (1500pb).

### Exemple 2: Tests d'activités

### 2.1 Activité mannosyltransférase och1 sur les souches de levures mutées

Autre niveau de validation : pour chaque gène intégré au génome des souches de levure, un control systématique d'activité enzymatique est réalisé, afin de suivre constamment les fluctuations éventuelles des niveaux d'activité, dû la plupart du temps à des mutations spontanées, et nécessitant alors la sélection de nouveaux clones.

L'activité de l'enzyme Och1 peut être mise en évidence par un dosage *in vitro.* Des études antérieures ont montré que le meilleur accepteur pour le transfert d'un mannose par l'enzyme Och1 est le Man₈GlcNAc₂. A partir des fractions microsomales des levures (100 µg de protéines) ou d'un lysat de protéines totales (200 µg), on mesure l'activité de transfert d'un mannose en position alpha-1,6 sur une structure Man₈GlcNAc₂. Pour cela, le Man₈GlcNAc₂ couplé à un groupement d'amino-pyridine (M₈GN₂-AP) est utilisé comme accepteur et le GDP-mannose marqué au [¹⁴C]-mannose comme molécule donneuse de mannose radioactif. Les microsomes ou les protéines sont incubés avec le donneur (GDP-mannose radioactif), l'accepteur (Man₈GlcNAc₂-AP) et du déoxymannojirimycine (inhibiteur de la mannosidase I) dans un milieu tamponné au pH contrôlé. Après 30 minutes d'incubation à 30°C, du chloroforme et du méthanol sont ajoutés au milieu réactionnel afin d'obtenir une proportion de CHCl₃-MeOH-H₂O de 3 :2 :1 (v/v/v). La phase supérieure correspondant à la phase aqueuse, contient le Man₈GlcNAc₂-AP, le Man₉GlcNAc₂-AP radioactif et le GDP-[¹⁴C]-mannose. Une fois séchés, les échantillons sont repris dans 100 µl d'H₂O-1% acide acétique et passés sur une colonne de Sep-Pak C18 (waters), conditionnée au préalable, afin de séparer le GDP-mannose du Man₉GlcNAc₂-AP radioactif formé (le groupement AP permet de retenir ce composé sur les colonne de C18). L'élution par l'H₂O-1% acide acétique (20 mL) puis par 20% méthanol-1% acide acétique (4 mL) permet de récupérer les différentes fractions et de les compter au compteur à scintillation.

### 2.2 Activité mannosidase

L'activité mannosidase est mesurée en incubant 4 heures à 37°C 4 mM de *p-*nitrophenyl-α-D-mannopyranoside avec 100-200 µg de protéines (provenant de protéines totales ou fractions subcellulaires) dans 0.1 M de PBS pH 6.5 +/- 120 µM DMJ (inhibiteur alpha-1,2-mannosidase I) +/- 12µM SW (inhibiteur spécifique de la mannosidase II). L'absorbance est mesurée à 405 nm.

### 2.3 Activité N-acétylglucosaminyl transférase

L'activité GlcNAcTransférase est mesurée sur des fractions microsomales de levures. 50 µg de microsomes (dosage BCA) sont incubés dans 50 µL final 25 minutes à 30°C avec 0.01 µCi de donneur (UDP-GIcNAc radioactif), 0.5 mM d'accepteur (3-O-α-D-mannopyranosyl-D-mannopyranoside) dans un milieu 50 mM HEPES, 10 mM MnCl₂, 0.1% TritonX-100. La réaction est arrêtée par 400 µL de 10 mM EDTA puis les échantillons sont passés sur des colonnes de Dowex AG-1X2. L'accepteur radioactif est ensuite élué des colonnes par de l'acide formique 3M et la radioactivité mesurée au compteur à scintillation.

### Exemple 3 : Cassette d'expression pour l'α-1-2 mannosidase I de C. elegans

Schéma explicatif pour la construction des cassettes d'expression : **figure 2**

### 3.1 Etape 1 : obtention de l'ORF

α-mannosidase I : PCR à partir de clones bactériens possédant le plasmide pDONR201 (Open Biosystem)
Programme :
8 minutes à 94 °C

| | |
|---|---|
| 35 cycles : | 20 sec à 94°C |
| | 30 sec à 65°C |
| | 2 min à 72°C |

10 minutes à 72°C
Amplification d'un fragment à 1644 pb

L'amplification PCR a été extraite et purifiée du gel d'agarose par le kit QBIOgene et a été introduite dans un vecteur TOPO2.1. Des bactéries compétentes (TOP 10, Invitrogen) ont été transformées avec ce vecteur. La transformation a été vérifiée par PCR et l'insertion de l'amplification PCR dans le vecteur par séquençage (plasmide pGLY02.001).

### 3.2 Etape 2 : assemblage de la cassette d'expression :

L'intégration de la cassette d'expression de la mannosidase I va être localisée dans le marqueur d'auxotrophie URA3 pour les deux souches. L'invalidation de ce gène induit une résistance à un agent toxique, le 5-fluorouracil. Les levures modifiées par cette cassette deviendront donc résistantes à cette drogue mais également auxotrophe pour l'uracile.

### Cassette d'expression pour S.cerevisiae :

- Amplification du promoteur pGAP à partir d'ADN génomique de S.cerevisiae sauvage BS16 (forward) et BS17' (reverse)
- Assemblage du promoteur pGAP (produit de PCR) et de l'ORF (pGLY02.001)
   BS16 (forward) et BS19' (reverse)
- Amplification du terminateur CYC1 à partir du plasmide pYES 2.1
   BS40b (forward) et BS41 (reverse)
- Assemblage de l'ORF (plasmide pGLY02.001) et du terminateur CYC1 (produit de PCR)
   BS18 (forward) et BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY02.002).
- assemblage promoteur-ORF (produit de PCR) et ORF-terminateur (pGLY02.002) avec les régions homologues à URA3 (provenant des amorces)
   BS42 (forward)
   BS43 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY02.004).

### Cassette d'expression pour S.pombe

- Amplification du promoteur adh1 à partir d'ADN génomique de S.pombe sauvage BS25 et BS26' (reverse)
- Assemblage du promoteur adh1 (produit de PCR) et de l'ORF (pGLY02.001) BS25 (forward) et BS20 (reverse)
   L'amplification PCR a été extraite et purifiée du gel d'agarose par le kit Qiagen et a été introduite dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage (pGLY02.009).
- Assemblage du produit promoteur-ORF (pGLY02.009) avec ORF- CYC1 (produit PCR)
   BS25 (forward) et BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY02.011).
- Amplification de la cassette (pGLY02.011) avec les régions flanquantes homologues à URA3
   BS76 (forward) et BS77 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage.

### 3.3 Etape 3 : transformation des levures

### -Préparation des levures compétentes :

S.cerevisiae Adèle :
   Protocole : Ensemencer 500 mL de levures à DO=0,1 et les incuber à 30°C jusqu'à 5.5<DO<6.5.
   Centrifuger les cellules à 1500g 5 min à 4°C et les re-suspendre dans 500 mL d'eau stérile froide.
   Centrifuger les cellules et les re-suspendre dans 250 mL d'eau stérile froide. Centrifuger les cellules et les re-suspendre dans 20 mL de sorbitol 1M. Centrifuger les cellules et les re-suspendre dans 1 mL de sorbitol 1 M. Aliquoter par 80 µL et les stocker à -80°C.
S.pombe Edgar:
   Protocole : Ensemencer 200 mL de levures à DO=0,5 et les incuber à 30°C jusqu'à DO=1.5.

Centrifuger à 3000 rpm pendant 5 min à 20°C. Laver les cellules dans de l'eau froide stérile et centrifuger, laver une seconde fois avec du sorbitol 1 M. Incuber 15 min en ajoutant du DTT pour arriver à 25 mM final (pour augmenter l'électrocompétence). Reprendre en suspension finale dans du sorbitol 1 M froid (densité 1 à 5.109 cells/mL: env. 5 mL). Aliquoter en 40 µL et stocker env 10 fioles à -80°C.

### -Transformation des levures par électroporation :

Pour chaque cassette d'expression, l'ADN utilisé pour transformer les levures provient soit d'une digestion du plasmide mentionné avec des enzymes de restriction sélectionnées, soit directement du produit PCR obtenu et purifié après l'assemblage complet.

Cassette S.cerevisiae : pGLY02.004 est digéré par les enzymes de restriction BamHI et SmaI.
Les levures compétentes sont transformées avec 1 µg d'ADN: incuber 5 min dans la glace. Donner un pulse à V=1500 V. Ajouter immédiatement 1 mL de sorbitol 1M stéril glacé et transférer les cellules avec une pipette pasteur dans un tube eppendorf puis laisser relaxer au moins 1 heure dans l'infors à 30 °C. Étaler les levures sur une boite de milieux de sélection (YPD contenant 10 mM 5-fluorouracile, 5-FU). Les transformants apparaissent dans les 4 à 6 jours.

Cassette S.pombe : les levures compétentes sont transformées avec 100 ng d'ADN (produit de PCR): incuber 5 min dans la glace. Les cellules et l'ADN sont transférées dans une cuve à électroporation. Donner un pulse à V=1500V et ajouter immédiatement 0.9 mL de 1M sorbitol froid. Les cellules sont étalées aussi rapidement que possible sur le milieu approprié (YPD contenant 10 mM 5-FU). Les transformants apparaissent dans les 4 à 6 jours.

### Exemple 4: Souches Amélie et Emma + Cassette d'expression pour la N-acétyl-glucosaminyl transférase I humaine

### 4.1 Etape 1: Obtention de l'ORF

PCR à partir d'un plasmide commercial Biovalley (Human ORF clone V1.1) Programme :
5 minutes à 94 °C

| | |
|---|---|
| 30 cycles : | 60 sec à 94°C |
| | 60 sec à 56°C |
| | 2 min à 72°C |

5 minutes à 72°C

Amplification d'un fragment 1327 pb sans la partie cytoplasmique de l'enzyme : elle sera remplacée par la partie cytoplasmique de Mnn9 pour une localisation golgienne de la protéine.

**Région cytoplasmique de Mnn9 :** PCR à partir d'ADN génomique de S. cerevisiae sauvage
8 minutes à 95°C

| | |
|---|---|
| 30 cycles : | 20 sec à 94°C |
| | 30 sec à 58°C |
| | 1 min à 72°C |

10 minutes à 72°C Amplification d'un fragment de 51 pb (partie cytoplasmique de mmn9)
**Atgtcactttctcttgtatcg**taccgcctaa**gaaagaacccgtgggttaac** (SEQ ID No 13)

A partir de ces deux amplifications de PCR :
Obtention d'un seul fragment :

Le produit d'amplification de la PCR d'assemblage a été purifié du gel d'agarose par le kit QIAGEN et a été introduit dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage (pGLY03.001).

### 4.2 Etape 2 : assemblage de la cassette d'expression

### Cassette d'expression pour S.cerevisiae

L'intégration de la cassette d'expression de la GlcNAc transférase I pour la levure S.cerevisiae va être localisée dans le marqueur d'auxotrophie ADE2. L'invalidation de ce gène induit un changement de couleur des levures qui deviennent rouges et également une auxotrophie pour l'adénine.
- Amplification du promoteur adh1 à partir d'ADN génomique de S.cerevisiae BS29 (forward) et BS30 (reverse)
- Assemblage du promoteur adh1 (produit de PCR) et de l'ORF (pGLY03.001): BS29 (forward) et BS59

L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.002).
- Assemblage de l'ORF (pGLY03.001) avec le terminateur CYC1 (produit de PCR) : CA005 (forward)
   BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.011).
- Assemblage du promoteur-ORF(pGLY03.002) et de l'ORF-terminateur (produit de PCR) avec les extensions homologues à ADE2 :
   BS67 (forward) et BS68 (reverse)

   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.010).

### Cassette d'expression pour S.pombe

L'intégration de la cassette d'expression de la GlcNAc transférase I pour la levure S.pombe va être localisée dans le marqueur d'auxotrophie ADE1. L'invalidation de ce gène induit un changement de couleur des levures qui deviennent rouges et également une auxotrophie pour l'adénine.
- Amplification du promoteur hCMV à partir du plasmide pCDNA 3.1
   BS62 (forward) et BS58 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.004).
- Assemblage hCMV-ORF (pGLY03.004) avec le terminateur CYC1 (produit de PCR) BS62 (forward) et BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.005).
- Assemblage de la cassette d'expression (pGLY03.005) avec les extensions homologues à ADE1 :
   BS69 (forward) et BS70 (reverse)
      L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY03.007).

### 4.3 Etape 3 : Transformation des levures

- Préparation de levures compétentes :
   Les souches Amélie et Emma ont été préparées comme indiqué ci-dessus afin de les rendre compétentes.
- Electroporation des levures S.cerevisiae et S.pombe
   Protocole :
   20 µg des plasmides contenant les cassettes d'expression pour *S.cerevisiae* et *S*.*pombe* ont été digéré par l'enzyme de restriction EcoRI. La cassette linéarisée a été introduite dans les levures Amélie et Emma par électroporation. Les levures sont sélectionnées sur un milieu YNB contenant les acides aminés nécessaires.

### Exemple 5 : souches Agathe et Egée + Cassette d'expression pour le transporteur d'UDP-GlcNAc

### 5.1 Etape 1 : Obtention de l'ORF

### PCR à partir de cDNA de pancréas humain

Programme :
3 minutes à 94 °C

| | |
|---|---|
| 30 cycles : | 20 sec à 94°C |
| | 30 sec à 58°C |
| | 2 min à 72°C |

10 minutes à 72°C Amplification d'un fragment de 916 pb

L'amplification PCR a été extraite et purifiée du gel d'agarose par le kit QIAGEN et a été introduite dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage (pGLY04.001).

### 5.2 Etape 2 : Assemblage de la cassette d'expression :

### Cassette pour S.cerevisiae et pour S.pombe :

L'intégration de la cassette d'expression du transporteur d'UDP-GlcNAc va être localisée dans le marqueur d'auxotrophie LYS2 pour les deux souches S.cerevisiae et S.pombe. L'invalidation de ce gène induit une résistance à un agent toxique, l'acide alpha-aminoadipique. Les levures modifiées par cette cassette deviendront donc résistantes à cette drogue mais également auxotrophe pour la lysine.
- Amplification du promoteur PGK à partir du plasmide pFL61
   BS95 (forward) et BS96 (reverse)
- Assemblage ORF (pGLY04.001) avec le terminateur CYC1 (produit PCR)
   CA017 (forward) et BS41 (reverse)
   L'amplification PCR a été extraite et purifiée du gel d'agarose par le kit QIAGEN et a été introduite dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage (pGly04.002).
- Assemblage du promoteur PGK (produit de PCR) avec le fragment ORF-terminateur CYC1 (pGLY04.002):
   BS95 (forward) et BS41 (reverse)
- Assemblage de la cassette d'expression avec les extensions homologues à LYS2 S.Cerevisiae :
   BS97 (forward) et BS98 (reverse)
   S.Pombe
   BS99 (forward)
   BS100 (reverse)
      Les amplifications PCR ont été extraites et purifiées du gel d'agarose par le kit Qiagen et ont été introduites dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGly04.006 pour la cassette S.cerevisiae et pGly04.005 pour la cassette S.pombe).

### 5.3 Etape 3 : Modification des levures

- Préparation de levures compétentes :
   Les souches Agathe et Egée ont été préparées comme indiqué ci-dessus afin de les rendre compétentes.
- Electroporation des levures :
   20 µg des plasmides contenant les cassettes d'expression pour *S.cerevisiae, S.pombe* ont été digéré par l'enzyme de restriction EcoRI. La cassette linéarisée a été introduite dans les levures Agathe et Egée par électroporation. Les levures sont sélectionnées sur un milieu YNB contenant les acides aminés nécessaires et de l'acide alpha-aminoadipique.

### Exemple 6: souches Arielle et Erika + Cassette d'expression pour l'α-mannosidase II

### 6.1 Etape 1 : obtention de l'ORF

PCR à partir de cDNA de foie de souris
Programme :
3 minutes à 94 °C

| | |
|---|---|
| 35 cycles : | 20 sec à 94°C |
| | 30 sec à 58°C |
| | 4 min à 72°C |

10 minutes à 72°C Amplification d'un fragment de 3453 pb

L'amplification de cet ORF a été obtenu par une PCR nichée (3453 pb) sur d'ADNc de foie de souris puis purifiée par la méthode phénol/chloroforme. Le produit PCR a été introduit dans un vecteur TOPO-XL. Des bactéries compétentes (TOP10, Invitrogen) ont été transformées avec ce vecteur. La transformation a été vérifiée par PCR et l'insertion de l'amplification PCR dans le vecteur par séquençage (pGLY05.001).

### 6.2 Etape 2 : assemblage de la cassette d'expression

### Cassette S. cerevisiae et S. pombe:

L'intégration de la cassette d'expression de la mannosidase II va être localisée dans le marqueur d'auxotrophie LEU2 pour les deux souches. L'invalidation de ce gène induit une résistance à un agent toxique, la trifluoroleucine. Les levures modifiées par cette cassette deviendront donc résistantes à cette drogue mais également auxotrophe pour la leucine.
- Amplification du promoteur TEF à partir d'ADN génomique de S.cerevisiae :
   BS83 (forward) et BS84 (reverse)
- assemblage promoteur TEF (produit de PCR) avec l'ORF (produit de PCR) et le terminateur (produit de PCR)
   Pour S.cerevisiae marqueur LEU2
   BS 111 (forward) et BS112 (reverse)
   Pour S.pombe marqueur LEU2
   BS113 (forward) et BS114 (reverse)

Les amplifications PCR ont été extraites et purifiées du gel d'agarose par le kit Qiagen et ont été introduites dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGly05.008 pour la cassette S.cerevisiae, pGly05.009 pour la cassette S.pombe).

### 6.3 Etape 3 : modification des levures

- Préparation de levures compétentes :
   Les souches Arielle et Erika ont été préparées comme indiqué ci-dessus afin de les rendre compétentes.
- Electroporation des levures
   Protocole : 20 µg des plasmides contenant les cassettes d'expression pour S.cerevisiae et S.pombe ont été digéré par l'enzyme de restriction EcoRI. La cassette linéarisée a été introduite dans les levures Arielle et Erika par électroporation. Les levures sont sélectionnées sur un milieu YNB contenant les acides aminés nécessaires ainsi que de la tri-fluoroleucine (TFL) pour S.cerevisiae et S.pombe.

### Exemple 7 : souches Anaïs et Enrique + Cassette d'expression de la N-acetyl-glucosaminyl transférase II

### 6.1 Etape 1 : obtention de l'ORF :

PCR à partir d'ADN complémentaire de fibroblastes humains - Utilisation de la Taq polymérase Isis™ (Q-Biogene)
Programme:
3 min à 94°C

| | |
|---|---|
| 30 cycles: | 30 sec à 94°C |
| | 30 sec à 58°C |
| | 1.30 min à 68°C |

Amplification d'un fragment de 1344 pb

L'amplification PCR a été purifiée par la méthode phénol/chloroforme et introduite dans le vecteur pTarget (Promega) Des bactéries compétentes (JM109, Promega) ont été transformées avec ce vecteur. La transformation a été vérifiée par PCR et l'insertion de l'amplification PCR dans le vecteur par séquençage (pGLY08.002).

**Région cytoplasmique de mmn9 :** PCR à partir d'ADN génomique de S. cerevisiae sauvage
8 minutes à 95°C

| | |
|---|---|
| 30 cycles : | 20 sec à 94°C |
| | 30 sec à 58°C |
| | 1 min à 72°C |

10 minutes à 72°C
Amplification d'un fragment de 51 pb (partie cytoplasmique de mmn9) + homologie à GNTII
**Atgtcactttctcttgtatcg**taccgcctaag**aaagaacccgtgggttaacaggttccgcatctac**

**Assemblage de Mnn9 (produit PCR) et de l'ORF (pGLY08.002)** avec la Taq Platinium
CA005 (forward) et CD005 (reverse)
programme
2 min à 95°C

| | |
|---|---|
| 30 cycles: | 45 sec à 95°C |
| | 45 sec à 54°C |
| | 2 min à 72°C |

10 min à 72°C

L'amplification PCR a été purifiée par la méthode phénol/chloroforme et introduite dans le vecteur pTarget (Promega). Des bactéries compétentes (JM109, Promega) ont été transformées avec ce vecteur. La transformation a été vérifiée par PCR et l'insertion de l'amplification PCR dans le vecteur par séquençage (pGLY08.007)

### 7.2 Etape 2 : assemblage des cassettes d'expression pour les souches S.cerevisiae et S. pombe.

L'intégration de la cassette d'expression de la GlcNAc-transférase II va être inséré dans le marqueur Lem3 pour *S.cerevisiae* et TRP1 pour *S.pombe.* L'invalidation du gène Lem3 induit une résistance à un agent toxique, la miltefosine. Les levures modifiées par cette cassette deviendront donc résistantes à cette drogue. L'invalidation du gène TRP1 induit une résistance à un agent toxique, l'acide 5-fluoro-anthranilique. Les levures modifiées par cette cassette deviendront donc résistantes à cette drogue mais également auxotrophe pour le tryptophane.
- Amplification du promoteur PMA1
CD001 (forward) aagcttcctgaaacggag
CD008 (reverse) acgatacaagagaaagtgacatattgatattgtttgataattaaat
PCR à partir d'ADN génomique de S.cerevisiae Tyr-
Programme:
2 min à 95°C

| | |
|---|---|
| 30 cycles: | 45 sec à 95°C |
| | 45 sec à 54°C |
| | 2 min à 72°C |

5 min à 72°C Assemblage du promoteur PMA1 (produit PCR) avec Mnn9-homologie ORF (pGLY08.007) avec la Taq Expand (Roche)
CD007 cgtttgtagatgcggaacctgttaacccacgggttcttt
CD001 aagcttcctgaaacggag
2 min à 94°C

| | |
|---|---|
| 30 cycles: | 45 sec à 94°C |
| | 45 sec à 55°C |
| | 1.15 min à 68°C |

5 min à 68°C
L'amplification PCR a été purifiée par la méthode phénol/chloroforme et introduite dans le vecteur TOPO2.1 (Invitrogen). Des bactéries compétentes (TOP10, Invitrogen) ont été transformées avec ce vecteur. La transformation a été vérifiée par PCR et l'insertion de l'amplification PCR dans le vecteur par séquençage (pGLY08.005).
- Assemblage Mnn9-ORF (pGLY08.007) avec le terminateur CYC1 (produit PCR) avec la Taq polymérase Phusion™ (Ozyme)
Programme
2 min à 98°C

| | |
|---|---|
| 3 cycles | 10 sec à 98°C |
| | 30 sec à 52°C |
| | 40 sec à 72°C |

ajout des primers puis

| | |
|---|---|
| 30 cycles | 10 sec à 98°C |
| | 30 sec à 61 °C |
| | 40 sec à 72°C |

5 min à 72°C
L'amplification PCR a été purifiée par la méthode phénol/chloroforme et introduite dans le vecteur pTarget (Promega). Des bactéries compétentes (JM109, Promega) ont été transformées avec ce vecteur. La transformation a été vérifiée par PCR et l'insertion de l'amplification PCR dans le vecteur par séquençage (pGLY08.04)
- Assemblage du promoteur PMA1-Mnn9 (pGLY08.009) avec Mnn9-ORF-terminateur CYC1 (produit PCR) avec les extrémités homologues au marqueur Lem3 pour *S.cerevisiae* avec la Taq polymérase Phusion™ (Ozyme)
CB053: Atggtaaatttcgatttgggccaagttggtgaagtattccaagcttcctgaaacggag (forward)
CB070: Ttctaccgccgaagagccaaaacgttaataatatcaatggcagcttgcaaattaaagc (reverse) Programme
2 min à 98°C

| | |
|---|---|
| 3 cycles | 10 sec à 98°C |
| | 30 sec à 52°C |
| | 1 min à 72°C |

ajout des primers puis

| | |
|---|---|
| 30 cycles | 10 sec à 98°C |
| | 30 sec à 61 °C |
| | 1 min à 72°C |

5 min à 72°C
L'amplification PCR a été purifiée par la méthode phénol/chloroforme et introduite dans le vecteur pTarget (Promega). Des bactéries compétentes (JM109, Promega) ont été transformées avec ce vecteur. La transformation a été vérifiée par PCR et l'insertion de l'amplification PCR dans le vecteur par séquençage **(pGLY08.)**
- Assemblage des extrémités homologues au marqueur TRP1 pour *S.pombe* à partir de pGLY08.012
CD009 (forward) taaagttgattccgctggtgaaatcatacatggaaaagtttaagcttcctgaaacggag
CD010 (reverse) atgtgaaatttccttggccacggacaagtccacttttcgtttggcagcttgcaaattaaagc

### 7.3 Etape 3 : modification des levures

- Préparation de levures compétentes :
   Les souches Anaïs et Enrique ont été préparées comme indiqué ci-dessus afin de les rendre compétentes.
- Electroporation des levures
   Protocole : 20 µg des plasmides contenant les cassettes d'expression pour S.cerevisiae et S.pombe ont été digéré par l'enzyme de restriction EcoRI. La cassette linéarisée a été introduite dans les levures Anaïs et Enrique par électroporation. Les levures sont ensuite étalées sur un milieu YPD gélosé contenant la drogue de sélection nécessaire.

### Exemple 8: souches Alice et Elga + Cassette d'expression de la galactosyl transférase I

### 8.1 :Etape 1 : obtention de l'ORF sans la séquence de localisation humaine

PCR à partir de cDNA de lymphoblastes humains
CD025 (forward) CD026 (reverse)
2 minutes à 94 °C

| | |
|---|---|
| 30 cycles : | 45 sec à 94°C |
| | 45 sec à 58°C |
| | 1.15 min à 72°C |

5 minutes à 72°C
Amplification d'un fragment de 1047 pb

L'amplification PCR a été purifiée par la méthode phénol/chloroforme et introduite dans le vecteur pTarget (Promega) Des bactéries compétentes (JM109, Promega) ont été transformées avec ce vecteur. La transformation a été vérifiée par PCR et l'insertion de l'amplification PCR dans le vecteur par séquençage (pGLY11.003).

### Localisation Mnt1 : PCR à partir d'ADNg de S.cerevisiae

3 minutes à 94 °C

| | |
|---|---|
| 30 cycles : | 20 sec à 94°C |
| | 30 sec à 58°C |
| | 45sec à 72°C |

10 minutes à 72°C Amplification d'un fragment de 246pb - SEQ ID No 14

L'amplification PCR a été purifiée par la méthode phénol/chloroforme et introduite dans le vecteur pTarget (Promega) Des bactéries compétentes (JM109, Promega) ont été transformées avec ce vecteur. La transformation a été vérifiée par PCR et l'insertion de l'amplification PCR dans le vecteur par séquençage.

### 8.2 : Etape 2 : assemblage des cassettes d'expression pour les souches S.cerevisiae et S. pombe.

L'intégration de la cassette d'expression de la Galactosyltransférase I va être localisée dans le marqueur TRP1 pour *S.cerevisiae* Alice. L'invalidation de ce gène induit une résistance à un agent toxique, l'acide fluoroanthranilique. Les levures modifiées par cette cassette deviendront donc résistantes à cette drogue. L'intégration de la cassette d'expression de la Galactosyltransférase I va être localisée dans le marqueur Met17 pour *S.cerevisiae* Ashley.
- Amplification du promoteur CaMV à partir du plasmide pMDC :
CD035 (forward)
CD037 (reverse)
programme
2 min à 94°C

| | |
|---|---|
| 30 cycles | 45 sec à 94°C |
| | 45 sec à 59°C |
| | 2 min 30 à 72°C |

5 min à 72°C L'amplification PCR a été extraite et purifiée du gel d'agarose par le kit Qiagen et a été introduite dans un vecteur pTarget. Des bactéries compétentes (JM109, Promega) ont été transformées avec ce vecteur. La transformation a été vérifiée par PCR et l'insertion de l'amplification PCR dans le vecteur par séquençage (pGLY11.001).
- Assemblage du promoteur CaMV (pGLY11.001) avec la séquence de localisation Mnt1 (produit de PCR) :
CD035 (forward) et CD028 (reverse)
Programme:
2 min à 94°C

| | |
|---|---|
| 30 cycles | 45 sec à 94°C |
| | 45 sec à 59°C |
| | 1 min 15 à 72°C |

3 min à 72°C L'amplification PCR a été extraite et purifiée du gel d'agarose par le kit Qiagen et a été introduite dans un vecteur pTarget. Des bactéries compétentes (JM109, Promega) ont été transformées avec ce vecteur. La transformation a été vérifiée par PCR et l'insertion de l'amplification PCR dans le vecteur par séquençage (pGLY11.002).
- Assemblage du promoteur CaMV-localisation Mnt1 (pGLY011.002) avec l'ORF (produit PCR).
CD035 (forward)
CD029 (reverse)
Programme
2 min à 94°C

| | |
|---|---|
| 30 cycles | 45 sec à 94°C |
| | 45 sec à 56°C |
| | 2 min 30 à 72°C |

3 min à 72°C
L'amplification PCR a été purifiée par la méthode phénol/chloroforme et introduite dans le vecteur pTarget (Promega). Des bactéries compétentes (JM109, Promega) ont été transformées avec ce vecteur. La transformation a été vérifiée par PCR et l'insertion de l'amplification PCR dans le vecteur par séquençage (pGLY011.004).
- Assemblage du promoteur CaMV-localisation Mnt1-ORF (pGLY011.004) avec le terminateur CYC1 (produit PCR) avec la Taq Expand (Roche)
CD035 (forward) BS41 (reverse)
Programme
3 min à 95°C

| | |
|---|---|
| 30 cycles | 30 sec à 95°C |
| | 30 sec à 57°C |
| | 2 min 30 à 68°C |

10 min à 68°C

L'amplification PCR a été purifiée par la méthode phénol/chloroforme et introduite dans le vecteur pTarget (Promega). Des bactéries compétentes (JM109, Promega) ont été transformées avec ce vecteur. La transformation a été vérifiée par PCR et l'insertion de l'amplification PCR dans le vecteur par séquençage (pGLY011.005).

Assemblage de la cassette d'intégration pour S.cerevisiae avec la Taq Expand (Roche)
CD063 (forward) agatgccagaaacaaagcttgttgcaggtggtgctgctcatgcctgcaggtcaacatggt
CD064 (reverse) gtgtcgacgatcttagaagagtccaaaggtttgactggatgcagcttgcaaattaaagcc
Programme
3min à 95°C

| | |
|---|---|
| 30 cycles | 30 sec à 95°C |
| | 30 sec à 57°C |
| | 2 min 30 à 68°C |

10 min à 68°C

L'amplification PCR a été purifiée par la méthode phénol/chloroforme et introduite dans le vecteur TOPO 2.1 (Invitrogen). Des bactéries compétentes (TOP10 Invitrogen) ont été transformées avec ce vecteur. La transformation a été vérifiée par PCR et l'insertion de l'amplification PCR dans le vecteur par séquençage (pGLY011.008).

Pour S.pombe, intégration dans la séquence MET6
Utilisation de la Taq expand (Roche)
CD038 (forward) et CD039 (reverse)
Les produits PCR a été introduit dans un vecteur TOPO-XL. Des bactéries compétentes (TOP10, Invitrogen) ont été transformées avec ce vecteur. La transformation a été vérifiée par PCR et l'insertion de l'amplification PCR dans le vecteur par séquençage (pGLY11.006 pour S.pombe et pGLY11.007 pour S.cerevisiae).

### 8.3 Etape 3 : modification des levures

- Préparation de levures compétentes :
   Les souches Alice et Elga ont été préparées comme indiqué ci-dessus afin de les rendre compétentes.
- Electroporation des levures
   Protocole : 20 µg des plasmides contenant les cassettes d'expression pour S.cerevisiae et S.pombe ont été digéré par les enzymes de restriction KpnI et XhoI. La cassette linéarisée a été introduite dans les levures Alice et Elga par électroporation. Les levures sont ensuite étalées sur un milieu YPD gélosé contenant la sélection nécessaire.

### Exemple 9: Souches Anaïs et Enrique + Cassettes d'expression pour la fucosylation (α-1,6-fucosyltransférase FUT8 et transporteur de GDP-fucose):

### 9.1 Cassette d'expression de FUT8

### 9.1.1 Etape 1 : obtention de l'ORF

PCR à partir de cDNA de pancréas et de poumons humains
Programme :
3 minutes à 94 °C

| | |
|---|---|
| 35 cycles : | 20 sec à 94°C |
| | 30 sec à 58°C |
| | 2 min à 72°C |

10 minutes à 72°C Amplification d'un fragment de 1801 pb

L'amplification PCR (1801 pb à partir d'ADNc de poumon et de pancréas humains) a été extraite et purifiée du gel d'agarose par le kit QBIOgene et a été introduite dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage.

### 9.1.2 Etape 2 : assemblage de la cassette d'expression pour S.cerevisiae

- Amplification du promoteur mnt1 à partir d'ADN génomique de S.pombe BS86 (forward) et BS87 (reverse)
- Assemblage du promoteur mnt1 (produit de PCR) avec l'ORF (pGLY06.001)
   BS86 (forward) et BS88 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY06.003).
- Assemblage de l'ORF (pGLY06.001) avec le terminateur CYC1 (produit de PCR) CA011 (forward) et BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY06.002).
- Assemblage de nmt1-ORF (pGLY06.003) avec ORF-terminateur CYC1 (produit de PCR)
   BS86 (forward) et BS41 (reverse)
- Assemblage des extrémités pour l'intégration dans les marqueurs d'auxotrophie de levures :

### 9.1.2.1 S.cerevisiae:

L'intégration de la cassette d'expression de la FUT8 va être localisée dans le marqueur CAN1 pour la souche S.cerevisiae. L'invalidation du gène CAN1 induit une auxotrophie pour la canavanine.
BS147 (forward) et BS148 (reverse)
Les amplifications PCR ont été extraites et purifiées du gel d'agarose par le kit Qiagen et ont été introduites dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY06.005).

### 9.1.2.2 S.pombe:

La cassette d'expression pour la fucosyltransférase 8 a été réalisée en tandem avec une cassette de résistance à un antibiotique, la phléomycine. Cette double cassette est insérée dans un marqueur d'auxotrophie simple HIS5. L'insertion de cette cassette dans ce locus induira une résistance à la phléomycine ainsi qu'une auxotrophie pour l'histidine.

La cassette d'expression de FUT8 précédement obtenue est assemblée avec une cassette d'expression de la phléomycine comprenant le promoteur de SV40, l'ORF de la résistance à la phléomycine ainsi que le terminateur TEF. Ces cassettes en tandem sont insérées dans le marqueur HIS5.

### 9.1.3 Etape 3 : modification des levures

- Préparation de levures compétentes :
   Les souches Anaïs et Enrique ont été préparées comme indiqué ci-dessus afin de les rendre compétentes.
- Electroporation des levures
   Protocole : 20 µg des plasmides contenant les cassettes d'expression pour S.cerevisiae et S.pombe ont été digéré par l'enzyme de restriction EcoRI. La cassette linéarisée a été introduite dans les levures Anaïs et Enrique par électroporation. Les levures sont étalées en dilution limite sur un milieu YPD gélosé, les marqueurs délétés ne conférents pas de résistance à une drogue. Une fois les clones sortis, des réplicats sur milieu minimum sont établis afin de sélectionner les clones ne pouvant plus pousser sans l'acide aminé nécessaire.

### 9.2 Cassette d'expression du transporteur de GDP-fucose

### 9.2.1 Obtention de l'ORF :

PCR à partir de cDNA de poumons humains
Programme :
3 minutes à 94 °C

| | |
|---|---|
| 35 cycles : | 20 sec à 94°C |
| | 30 sec à 58°C |
| | 2 min à 72°C |

10 minutes à 72°C Amplification d'un fragment de 1136 pb

L'amplification PCR a été extraite et purifiée du gel d'agarose par le kit QIAGEN et a été introduite dans un vecteur TOPO2.1. Des bactéries compétentes (TOP10, Invitrogen) ont été transformées avec ce vecteur. La transformation a été vérifiée par PCR et l'insertion de l'amplification PCR dans le vecteur par séquençage (pGLY07.001).

### 9.2.2 Etape 2 : assemblage de la cassette d'expression pour S.cerevisiae et S.pombe

- Amplification du promoteur SV40 à partir du pTarget :
   BS109 (forward) et BS110 (reverse)
- Assemblage de l'ORF (pGLY07.001) avec le terminateur CYC1 (produit de PCR) CA013 (forward) et BS41 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY07.002).

### 9.2.2.1 S. cerevisiae

L'intégration de la cassette d'expression du transporteur de GDP-fucose va être localisée dans le marqueur d'auxotrophie TRP1 pour la souche S.cerevisiae L'invalidation du gène TRP1 induit une résistance à un agent toxique, l'acide 5-fluoro-anthranilique. Les levures modifiées par cette cassette deviendront donc résistantes à cette drogue mais également auxotrophe pour le tryptophane.
- Assemblage de la cassette promoteur SV40 (produit de PCR), ORF (produit de PCR) et terminateur CYC1 (produit de PCR)
   BS136 (forward) et BS137 (reverse)
   L'amplification PCR a été extraite et purifiée du gel d'agarose par le kit Qiagen et a été introduite dans un vecteur pTarget. Des bactéries compétentes (JM109, promega) ont été transformées avec ce vecteur. La transformation a été vérifiée par PCR et l'insertion de l'amplification PCR dans le vecteur par séquençage (pGLY07.003).

### 9.2.2.2 S. pombe

La cassette d'expression pour le transporteur de GDP-fucose a été réalisée en tandem avec une cassette de résistance à un antibiotique, l'hygromycine. Cette double cassette est insérée dans un gène codant pour une protéine impliquée dans la maturation des N-glycanes de *S.pombe,* GMA12. L'insertion de cette cassette dans ce locus induira une résistance à l'hygromycine mais également la délétion du gène gma12.

La cassette d'expression du transporteur de GDP-fucose précédement obtenue est assemblée avec une cassette d'expression de résistance à l'hygromycine comprenant le promoteur de SV40, l'ORF de la résistance à l'hygromycine ainsi que le terminateur TEF. Ces cassettes en tandem sont insérées dans le marqueur gma12.

### 9.2.3 Etape 3 : modification des levures

- Préparation de levures compétentes :
   Les souches Apoline et Epiphanie ont été préparées comme indiqué ci-dessus afin de les rendre compétentes.
- Electroporation des levures
   Protocole : 20 µg des plasmides contenant les cassettes d'expression pour S.cerevisiae et S.pombe ont été digéré par l'enzyme de restriction EcoRI. La cassette linéarisée a été introduite dans les levures Apoline et Epiphanie par électroporation. Les levures S.cerevisiae sont étalées sur milieu YPD contenant de l'acide 5-fluoro-anthranilique. Les levures S.pombe sont étalées en dilution limite sur un milieu YPD gélosé, le marqueur délété ne conférant pas de résistance à une drogue. Une fois les clones sortis, des réplicats sur milieu minimum sont établis afin de sélectionner les clones ne pouvant plus pousser sans l'acide aminé nécessaire.

### Exemple 10 : souches Athena et Etienne + cassette d'expression de la N-acetylglucosaminyl- transférase III.

### 10.1 Obtention de l'ORF

PCR à partir d'ADN complémentaire de cerveau de souris
CB007 (forward) Atgaagatgagacgctacaa
CB036 (reverse) ctagccctccactgtatc
Programme:
2 min à 94°C
30 cycles: 45 sec à 94°C
   45 sec à 56°C
   2 min à 72°C
5 min à 72°C

Amplification d'un fragment de pb sans la partie cytoplasmique de l'enzyme: elle sera remplacée par la partie cytoplasmique de Mnt1 pour une localisation golgienne de la protéine.

### 10.2 Assemblage de cassette d'expression pour les souches S.cerevisiae et S.pombe

### 10.2.1 Assemblage pour S.cerevisiae

Amplification du promoteur nmt1
CB013: tatagtcgctttgttaaatcatatggccctctttctcagtaa
CB014: agcgaagactccgaagcccacttctttaagaccttatcc
Amplification du terminateur CYC1
Amplification de la cassette d'expression avec les extrémités CAN1
CB030 (forward) : cagaaaatccgttccaagag
CB031 (reverse) : tgccacggtatttcaaagct

### 10.2.2 Assemblage pour S.pombe

Cassette d'expression de la GNTIII en tandem avec une cassette de résistance à l'hygromycine. Insertion dans GMA12.

### 10.3 Transformation des levures.

### Exemple 11 : délétion de gènes impliqués dans l'hypermannosylation chez S.cerevisiae et S.pombe

**11.1 Etape 1**:délétion du gène Mnn1 dans les levures Amélie, Arielle, Anaïs, Alice,Abel, Ashley, Athena, Azalée et Aurel:
11.1.1 Construction et insertion d'une cassette contenant un gène de résistance à l'hygromycine dans le gène Mnn1.
11.1.1.1 Construction de la cassette d'expression
Amplification du promoteur CaMV avec l'extrémité Mnn1 5'
   CB39: TTTATATTAAACCAAAGGTCTTTGAGATCGTGTACCATACTGCCTGCAGGTCAACATG
   CB40: TTCTCGACAGACGTCGCGGTGAGTTCAGGCTTTTTACCCATCCGGGGATCCTCTAGAGTC
Amplification hygromycine-terminateur TEF avec une homologie au promoteur CaMV et l'extrémité Mnn1 3'
CB41: TTTCATTTGGAGAGGACCTCGACTCTAGAGGATCCCCGGATGGGTAAAAAGCCTGAACTC
CB42: GGTGTTATCTTTATTAGCATGTGACCAAACAGTGTTGACATCGACACTGGATGGCGGCGT

Assemblage de la cassette d'insertion pour la délétion du gène Mnn1 chez S.cerevisiae:
CB39: TTTATATTAAACCAAAGGTCTTTGAGATCGTGTACCATACTGCCTGCAGGTCAACATG
CB42: GGTGTTATCTTTATTAGCATGTGACCAAACAGTGTTGACATCGACACTGGATGGCGGCGT

### 11.1.1.2 Transformation des levures

- Préparation de levures compétentes :
   Les souches ont été préparées comme indiqué ci-dessus afin de les rendre compétentes.
- Electroporation des levures S.cerevisiae
   Protocole :
   20 µg des plasmides contenant les cassettes d'expression pour *S.cerevisiae* ont été digéré par enzyme de restriction. La cassette linéarisée a été introduite dans les levures par électroporation. Les levures sont sélectionnées sur un milieu YPD contenant de l'hygromycine.

### 11.1.2 Délétion du gène Mnn9 dans les levures Amélie, Arielle, Anaïs, Alice,Abel, Ashley, Athena, Azalée et Aurel:

### 11.1.2.1 Construction d'une cassette d'intégration dans le gène Mnn9 contenant un gène de résistance à la Phléomycine.

Amplification du promoteur SV40 avec l'extrémité Mnn9 5'
CB46: CAAAGATCTTAACGTCGTCGACCATGTGCTTAAGCACGACGCAGGCAGAAGTATGCAAA
CB47: AAATCTCGTGATGGCAGGTTGGGCGTCGCTTGGTCGGCCATAGCTTTTTGCAAAAGCCTAG

Amplification phléomycine-terminateur TEF avec une homologie au promoteur SV40 CB48:
GCTGTGGAATGTGTGTCAGTTAGGGTGTGGAAAGTCCCCAATGGCCGACCAAGCGACGCCC CB49: GGTGTTATCTTTATTAGCATGTGACCAAACAGTGTTGACATCGACACTGGATGGCGGCGT

### 11.1.2.2 Transformation des levures

- Préparation de levures compétentes :
   Les souches ont été préparées comme indiqué ci-dessus afin de les rendre compétentes.
- Electroporation des levures S.cerevisiae
Protocole :
20 µg des plasmides contenant les cassettes d'expression pour *S.cerevisiae* ont été digéré par enzyme de restriction. La cassette linéarisée a été introduite dans les levures par électroporation. Les levures sont sélectionnées sur un milieu YPD contenant de la phléomycine.
11.2 Etape 2 : Délétion du gène GMA12 dans les levures *S.pombe* Emma, Erika, Enrique, Elga, Etienne
11.2.1 construction de la cassette d'intégration contenant le gène de résistance à l'hygromycine
ext-gma12/prom-CaMV/hph/Tef-term/ext-gma12
CB51 : CAAAGATCTTAACGTCGTCGACCATGTGCTTAAGCACGACTGCCTGCAGGTCAACATG
CB52: ATATGATCCTTTTCTTGAGCAGACATCCAATCGGATCCTTTCGACACTGGATGGCGGCGT

### 11.2.2 Transformation des levures

- Préparation de levures compétentes :
   Les souches ont été préparées comme indiqué ci-dessus afin de les rendre compétentes.
- Electroporation des levures S.cerevisiae
Protocole :
20 µg des plasmides contenant les cassettes d'expression pour *S.pombe* ont été digéré par enzyme de restriction. La cassette linéarisée a été introduite dans les levures par électroporation. Les levures sont sélectionnées sur un milieu YPD contenant de l'hygromycine.

### Exemple 12: souches + cassettes d'expression pour la sialylation des N-glycanes de S.cerevisiae et S.pombe

Dans le but d'obtenir une sialylation effective des N-glycanes des protéines produites chez *S.cerevisiae* et *S.pombe,* il faut tout d'abord introduire la voie de biosynthèse de l'acide sialique dans ces même levures. Pour se faire, nous avons introduit dans le génome des levures la voie de biosynthèse du CMP-acide sialique de N.meningitidis, enzymes localisées dans le cytosol.

Préparation des cassettes en tandem pour la sialylation (à partir des souches Athena Aurel et Azalée):

### 12.1 Cassette S1 :

Construction d'une cassette en tandem conposée du promoteur PET56, de l'ORF de l'acide sialique synthase, du terminateur CYC1 puis du promoteur PET56, de l'ORF du CMP-acide sialique synthase et du terminateur CYC1.
- Obtention des ORF:
Acide sialique synthase (1050 pb) CMP-acide sialique synthase (687 pb):
- Cassette d'expression:
   Promoteur PET56

### 12.2 Cassette S2 :

La cassette d'expression pour le transporteur de CMP-acide sialique a été réalisée en tandem avec une cassette de résistance à un antibiotique, l'hygromycine. Cette double cassette est insérée le gène mnn1. L'insertion de cette cassette dans ce locus induira une résistance à l'hygromycine mais également la délétion du gène mnn1.
- Obtention de l'ORF
   Transporteur du CMP-acide sialique mus musculus (1011 pb)
- Construction de la cassette d'expression:
   La cassette d'expression du transporteur de CMP-acide sialique (promoteur CaMV, ORF, terminateur CYC1) est assemblée avec une cassette d'expression de résistance à l'hygromycine comprenant le promoteur de CaMV, l'ORF de la résistance à l'hygromycine ainsi que le terminateur TEF. Ces cassettes en tandem sont insérées dans le marqueur mnn1.

### 12.3 Cassette S3 :

La cassette d'expression pour la sialyltransférase ST3GAL4 a été réalisée en tandem avec une cassette de résistance à un antibiotique, la phléomycine. Cette double cassette est insérée le gène mnn9. L'insertion de cette cassette dans ce locus induira une résistance à l'hygromycine mais également la délétion du gène mnn9.
- Obtention de l'ORF
   Sialyltransférase ST3GAL4 humaine (990 pb) Sialyltransférase ST3GAL4 souris (1002 pb)

La cassette d'expression du transporteur de CMP-acide sialique (promoteur CaMV, ORF, terminateur CYC1) est assemblée avec une cassette d'expression de résistance à l'hygromycine comprenant le promoteur de CaMV, l'ORF de la résistance à l'hygromycine ainsi que le terminateur TEF. Ces cassettes en tandem sont insérées dans le marqueur mnn1.

### Exemple 13 : production d'EPO glycosylé de façon homogène

### 13.1 Amplification de la séquence nucléotidique de l'érythropoiétine humaine (EPO) :

L'amplification de la séquence nucléotidique de l'EPO humaine a été obtenue à partir d'ADN complémentaire de rein humain avec les amorces appropriées.

### 13.2 Clonage de la séquence de l'EPO dans un vecteur d'expression de S.cerevisiae :

La séquence nucléotidique de l'huEPO humaine tronquée de son codon STOP (585 paires de bases) est intégrée dans un vecteur d'expression de levure S.cerevisiae. La continuité du cadre de lecture entre la séquence introduite et la séquence du plasmide pSC (épitope V5 et tag poly-histidine) a été confirmée par séquençage du plasmide obtenu (pSC-EPO). L'expression de la protéine EPO se trouve sous le control du promoteur pGAL1, promoteur inductible par le galactose pour les souches S.cerevisiae. La sélection des levures possédant le plasmide se fait par un retour de la prototrophie pour l'uracile (présence de la séquence URA3 dans le plasmide).
Séquence obtenue dans le plasmide d'expression : Séquence protéique de l'EPO séquencée dans le plasmide d'expression (SEQ ID No 12)

### 13.3 Extraction des ARN:

Centrifuger les levures 16000g pendant 5 min. Enlever le surnageant et remettre le culot en suspension dans 500µl de tampon TES (TrisHCl pH7.5 10mM, EDTA10mM, SDS 0.5%). Ajouter 200µL de phénol et 200µL de chloroforme puis incuber 20min à 65°C en vortexant 30s toutes les 5min et incuber 1h à -80°C. Centrifuger 20 min à 13200rpm puis récupérer la phase aqueuse et ajouter 335µL de phénol et 67µL de chloroforme. Vortexer et centrifuger 5min à 11000rpm. Récupérer la phase aqueuse et ajouter 300µL de chloroforme. Vortexer et centrifuger 2 min à 13200rpm. Récupérer la phase aqueuse et ajouter 30µL d'acétate de sodium 3M pH5.2 et 600µL d'éthanol absolu. Incuber 1h à -20°C. Centrifuger 15min à 13200rpm.Enlever le surnageant en faisant attention au culot. Laisser sécher, reprendre dans 100µl d'eau DEPC puis les placer dans un tube contenant l'unité de filtration violet Nucleospin (Nucleospin RNAII). Centrifuger 1min à 11000g. Enlever le filtre et ajouter 350µL d'éthanol 70%. Charger la colonne Nucleospin RNAII. Centrifuger 30sec à 8000g. Placer la colonne dans un nouveau tube, ajouter 350µL de Membrane Desalting Buffer. Centrifuger 1min à 11000g. Déposer 95µL de solution DNase au centre de la colonne puis incuber 15 min à température ambiante. Ajouter 200µL de solution RA2 (inactive la DNase) et centrifuger 30s à 8000g. Ajouter 600µL de solution RA3 au centre de la colonne.

Centrifuger 30s à 8000g. Placer la colonne dans un nouveau tube, ajouter 250µL de solution RA3. Centrifuger 2min à 11000g pour sécher la colonne. Placer la colonne dans un tube de 1.5mL et ajouter 50µL d'eau DEPC. Centrifuger 1min à 11000g. Conserver les échantillons à -80°C.

### 13.4 Transcription inverse: Super Script III First-Strand Synthesis System for RT-PCR

| 5µg d'ARN | au maximum 8µL | | | |
|---|---|---|---|---|
| Random hexamer | 50ng/µL | 1µL | | |
| dNTP mix | 10mM | 1µL | | |
| eau DEPC | | qsp10µL | | |
| Incuber 5 min à 65°C | | | | |
| Ajouter 10µL de mix de transcription: | | RT Buffer | 10X | 2µL |
| | | MgCl₂ | 25 mM | 4µL |
| | | DTT | 0,1 M | 2µL |
| | | RNase Out | 40U/µL | 1µL |
| | | SuperScript | 200U/µL | 1µL |
| Incuber | 10min à 25°C | | | |
| | 50min à 50°C | | | |
| | 5min à 85°C | | | |

Récupérer dans la glace et ajouter 1µL de RNaseH. Laisser incuber 20min à 37°C puis conserver les ADNc à -20°C.

### 13.5 Extraction des protéines :

Après une centrifugation à 1500g pendant 5 min à 4°C, le culot cellulaire est repris dans 500 µL d'H₂O stérile puis centrifuger à vitesse maximale pendant 30 secondes à 4°C. Le culot est repris dans 500 µL de tampon de lyse 50 mM sodium phosphate pH 7.4, 5% glycérol,1 mM PMSF, centrifuger 10 min à 1500g à 4°C. Le culot est ensuite repris dans un volume de tampon de lyse nécessaire pour obtenir une DO comprise entre 50 et 100. Les échantillons sont ensuite vortexer pendant 4x30 secondes avec des billes de verre et une centrifugation de 10 min à vitesse maximale est réalisée pour séparer les billes et les débris cellulaires du surnageant protéique. Un dosage BCA est réalisé sur le surnageant.

### 13.6 Purification de l'EPO:

Les protéines totales sont tout d'abord dialysées contre du tampon Tris HCl 10 mM pH 6.0. Après équilibration d'une colonne échangeuse de cations SP séphadex C50 avec 10 mM Tris-Hcl pH 6.0, les protéines totales dialysées sont chargées sur la colonne. Après rinçage de la colonne avec du tampon Tris HCl 10 mM pH 6.0, les protéines sont éluées avec du tampon Tris HCl 10 mM pH 6.0, 250 mM NaCl. On détermine l'absorbance de chaque fraction à 280 nm ainsi que la quantité de protéines éluées par un dosage de Bradford. Les protéines sont ensuite analysées par électrophorèse SDS-PAGE sur gel acrylamide 12%.

### 13.7 Détection de la protéine EPO - Western Blot

Les protéines totales sont transférées sur une membrane de nitrocellulose afin de procéder à une détection par l'anticorps anti-EPO (R&D systems). Après le transfert, la membrane est saturée par une solution de blocage (TBS, 1% blocking solution Roche) pendant 1 heure. La membrane est ensuite mise en contact avec la solution d'anticorps anti-EPO (dilution 1 :500) pendant 1 heure. Après trois rinçages TBS-Tween 20 0.1%, la membrane est mise en contact avec l'anticorps secondaire anti-souris-HRP afin de procéder à la détection par chimioluminescence (solution de détection Roche).

### 141. Résultats :

### 14.1 : Validation des clones ayant intégrés la cassette Kanamycine dans le gène Och1

Pour la suppression de l'activité Och1, la cassette introduite a été intégralement séquencée après son intégration afin de cartographier la région génomique affectée dans le génome de la levure. L'absence de l'activité enzymatique est ensuite réalisée, renforçant le résultat précédent, puis la structure des glycannes est déterminée par spectrométrie de masse.

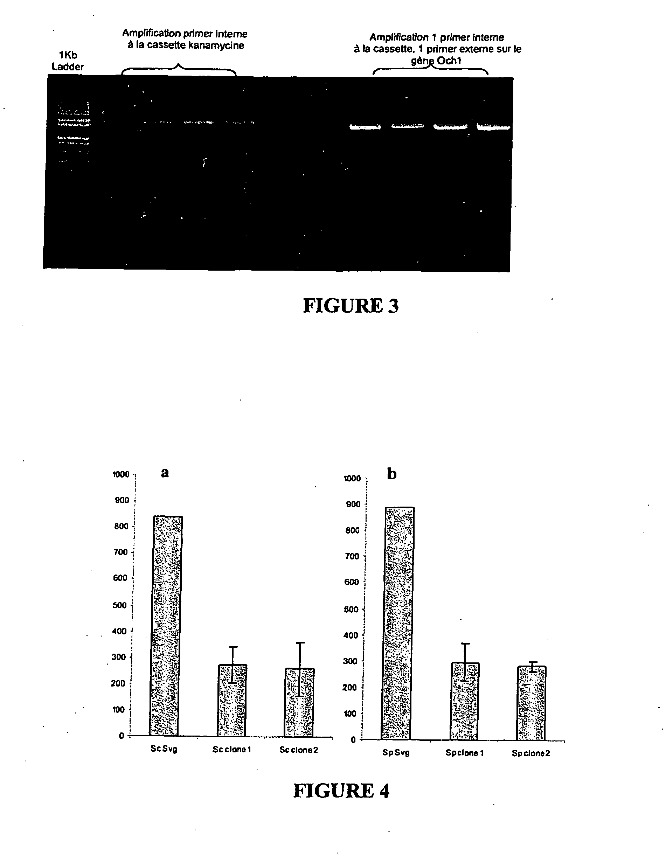

L'analyse sur gel TBE-agarose 1% de la réaction de PCR effectuée partir de l'ADN génomique des clones *S.cerevisiae* ayant résistés à la présence de kanamycine dans le milieu de culture montre un fragment de 2Kb amplifié avec un couple d'oligonucléotides spécifique de la cassette kanamycine. La taille de ce fragment correspond à a taille théorique de la cassette kanamycine. Le deuxième fragment a été amplifié grâce à un oligonucléotide interne à la cassette kanamycine et un oligonucléotide externe à la cassette s'hybridant avec la gène Och1 la taille théorique du fragment attendu est 1.5Kb ce qui correspond à la taille du fragment obtenu. Nous pouvons donc conclure que les clones 1, 2, 3 et 4 ont bien intégré la cassette kanamycine et que celle-ci a été intégrée dans le gène Och1 (voir figure **3****).**

Le même type de réaction de PCR a été effectué sur les souches S. *pombe* ayant résisté à la présence de kanamycine dans le milieu de culture. Ainsi deux clones mutés de chaque souche ont été isolés et testés pour la perte d'activité enzymatique α1,6-mannosyltransférase.

### Test d'activité mannosyltransférase och1 sur les souches de levures mutées

Validation de la perte d'activité Och1 dans les mutants Δoch1 obtenus par recombinaison homologue dans les levures *S. cerevisiae* et *S. pombe:*

Après la validation par PCR de l'insertion de la cassette d'expression de la kanamycine dans les levures *S.cerevisiae* sauvages et *S.pombe* sauvages, les clones positifs à cette insertion sont testés pour leur perte d'activité mannosyltransférase och1. L'activité och1 a été testée sur les microsomes des levures de *S.cerevisiae* et *S.pombe.* La **figure 4** montre le test d'activité och1 sur **a-** la fraction microsomale de la souche sauvage et des clones sélectionnés de S. cerevisiae **b-** la fraction microsomales de la souche sauvage et des clones sélectionnés de S.pombe. D'après la **figure 4****,** nous pouvons observée une perte d'activité de l'enzyme och1 dans les clones sélectionnés de *S.cerevisiae* (a) et de *S pombe (b).*

### Validation des souches par analyses des N-glycannes :

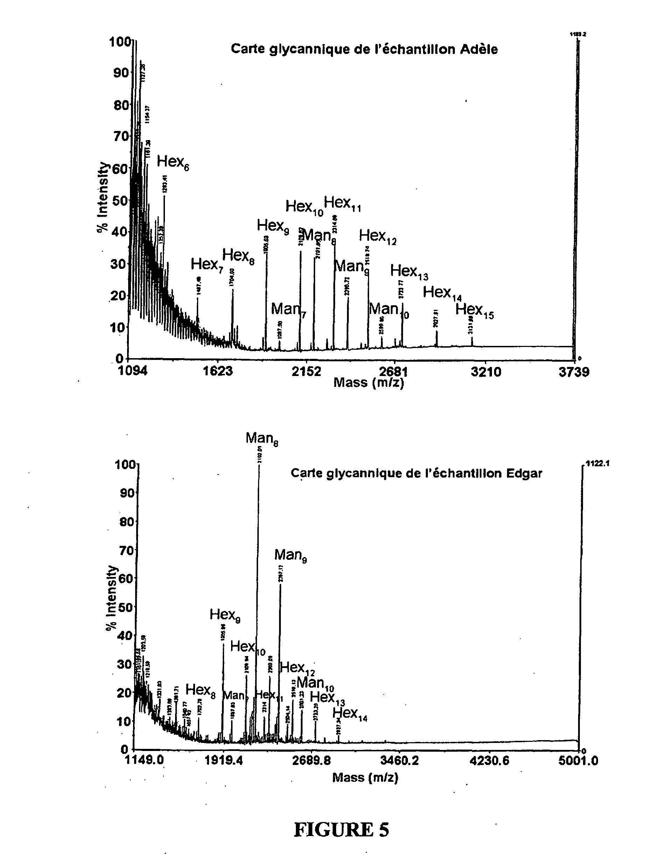

Les protéines totales provenant des deux souches modifiées ont été réduites et alkylées puis digérées par la trypsine. Les polysaccharides libres sont éliminés par passage sur SepPak C18. Les peptides et glycopeptides récupérés sont soumis à la PNGase. Les glycannes sont purifiés sur SepPak C18 puis méthylés avant d'être analysés en spectrométrie de masse en mode Maldi-Tof. La Figure 5 montre le spectre de masse réalisé sur les N-glycannes des souches Adèle et Edgar.
Nomenclature des levures :
S.cerevisiae Δoch1 = Adèle
S.pombe Δoch1 = Edgard

Les deux souches présentent des N-glycannes de formes oligomannosidiques du Man₇ au Man 10, des formes plus courtes que dans la souche sauvage de Saccharomyces cerevisiae indiquant la perte des formes polymannosylées sauvages.
Les structures prédominantes pour la souche Edgar (ΔOCH1) sont Man 9 et Man 8, structures classiquement rencontrées chez les mammifères à l'issue du transit de la protéine néosynthétisée dans le réticulum endoplasmique. Ceci suggère un blocage de la glycosylation du à l'impossibilité d'action des mannosyltransférases golgiennes qui ne greffent du mannose que sur des glycannes sur lesquels l'enzyme Och1 a greffé un mannose lié en α1,6.

### 14.2 : Validation des clones ayant intégrés la cassette Mannosidase I dans le gène URA3.

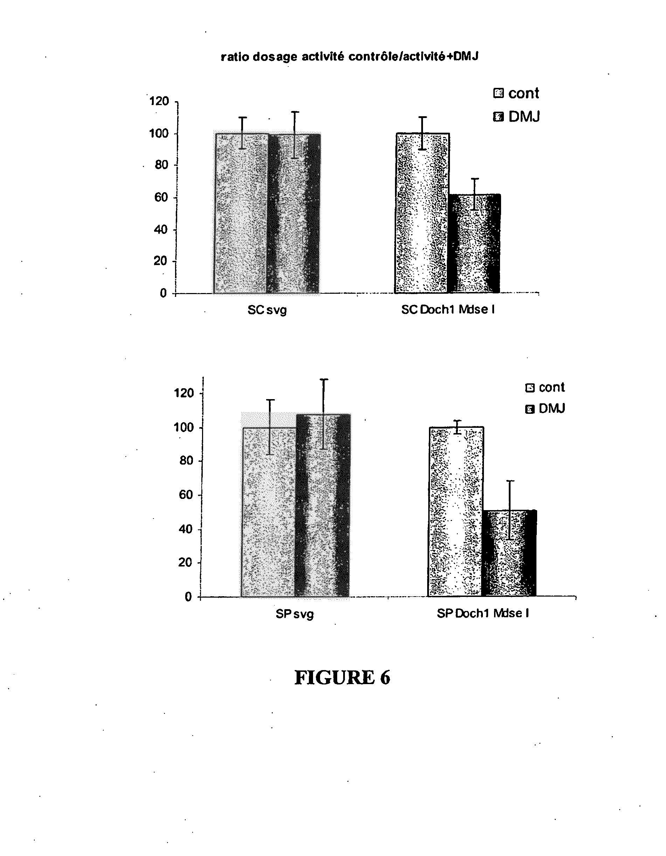

Les levures Adèle et Edgard positives pour l'insertion de la cassette d'expression de la mannosidase I dans le gène URA3 sont testées pour leur activité biochimique mannosidase I. La **Figure 6** montre le dosage d'activité mannosidase dans les microsomes de levures *S.cerevisiae* et *S.pombe.* L'expérience a été réalisée en triplicat. Nous pouvons observer dans les souches *S.cerevisiae* et *S. pombe* sauvages une activité mannosidase non-inhibée par le DMJ. Inversement, les souches sélectionnées présentent une inhibition significative de l'activité mannosidase mesurée lors d'un traitement DMJ. De plus, l'activité mannosidase I mesurée étant présente dans les microsomes de levures, nous pouvons en déduire que cette enzyme est exprimée dans la voie de sécrétion au niveau du réticulum endoplasmique/*cis*-Golgi, indiquant que le signal de rétention HDEL intégré en C-terminal de la protéine est bien reconnu par le système cellulaire.

Nomenclature des levures :
S.cerevisiae Adèle + mannosidase I = Amélie
S.pombe Edgard + mannosidase I =Emma

### 14.3 Validation des clones ayant intégrés la cassette N-acétylglucosaminyl transférase (GlcNAc Transférase I) dans les levures modifiées :

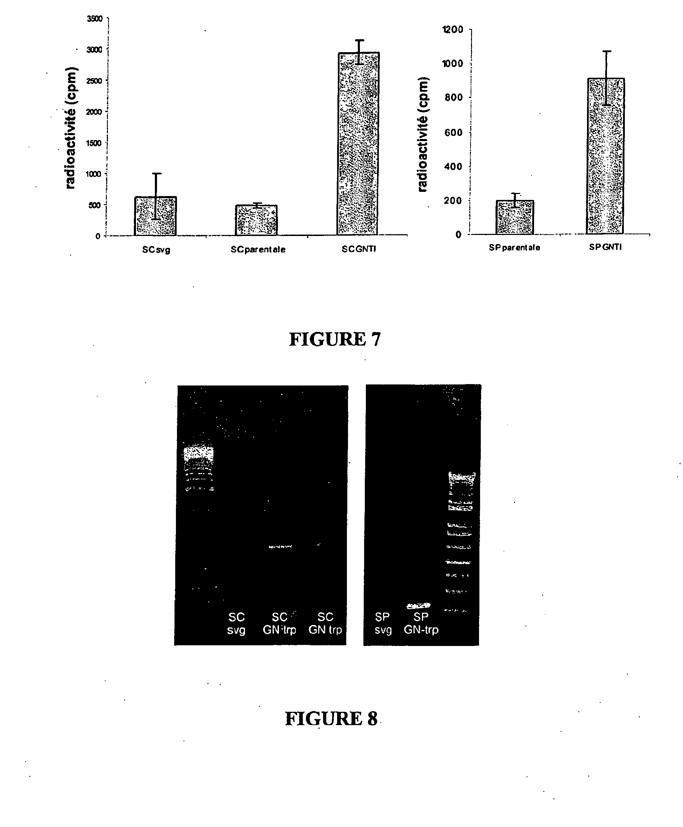

**La** **Figure 7** montre l'activité GIcNAcTransférase I dans les microsomes des levures sauvages et modifiées.
Dans les fractions microsomales des levures Amélie-GlcNacTI et Emma-GlcNacTI, nous observons une augmentation du marquage de l'accepteur par transfert d'un groupement GIcNAc radioactif comparée au marquage observée dans les levures témoins (sauvage et/ou Δoch1-MdseI). Ce transfert implique la présence d'une activité N-acétylglucosaminyltransférase dans les levures modifiées par l'expression de la GIcNAcTI.

Nomenclature des levures modifiées :
S.cerevisiae Amélie + GlcNAcTI = Agathe
S.pombe Emma + GIcNAcTI = Egée

### 14.4 Validation clones ayant intégrés la cassette du transporteur d'UDP-GIcNAc dans les levures modifiées :

L'expression du transporteur d'UDP-GIcNAc a été analysée par RT-PCR sur des cultures de levures parentales ou modifiées. Après une étape de reverse transcription sur les ARN totaux extraits, les cDNA ont été analysés par PCR en utilisant des primers spécifiques du transporteur d'UDP-GlcNAc (PCR nichée). On observe donc dans les levures modifiées par la cassette d'expression du transporteur d'UDP-GIcNAc une expression de l'ARNm de ce transporteur **(****figure 8****).**

Nomenclature des levures modifiées :
S.cerevisiae Agathe + UDP-GlcNAc transporteur = Arielle
S.pombe Egée + UDP-GIcNAc transporteur = Erika

### 14.5 Validation des clones ayant intégrés la cassette de la mannosidase II dans les levures modifiées :

### a- Validation par amplification PCR

Les clones sélectionnés pour *S.cerevisiae* et *S.pombe* ont été testés par PCR pour vérifier la présence des cassettes d'expression de la mannosidase II dans le génome des levures.

### b- Expression de la mannosidase II

L'expression de la mannosidase II a été analysée par RT-PCR sur des cultures de levures parentales ou modifiées. Après une étape de reverse transcription sur les ARN extraits, les cDNA ont été analysés par PCR en utilisant des primers spécifiques de la mannosidase II (PCR nichée). On observe donc dans les levures modifiées par la cassette d'expression de la mannosidase II une expression de l'ARNm de cette protéine.

### c- Mesure de l'activité de la mannosidase II

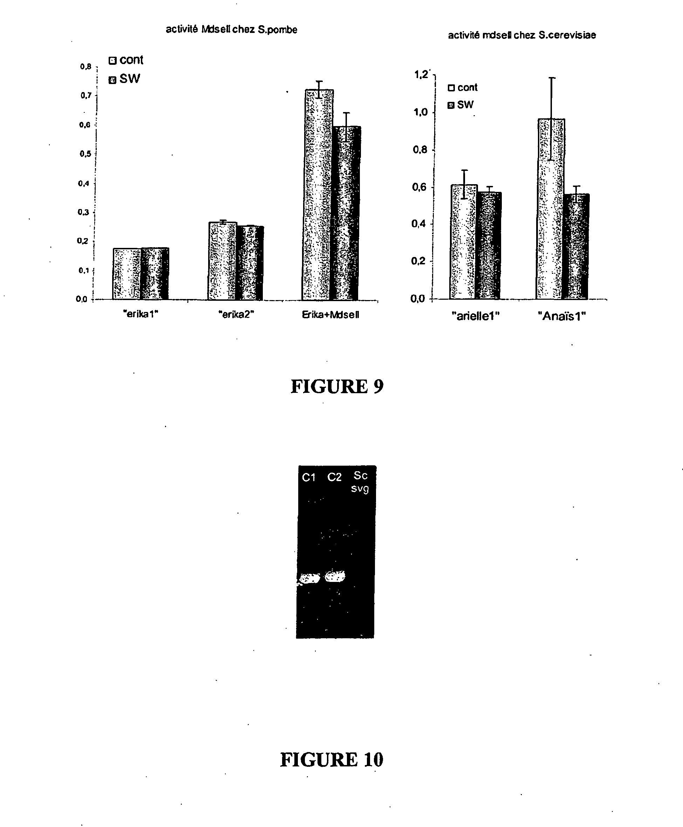

Les levures Adèle et Edgar positives pour l'insertion de la cassette d'expression de la mannosidase II sont testées pour leur activité biochimique mannosidase II.
D'après la **figure 9****,** nous pouvons observer dans les souches *S.cerevisiae* et *S. pombe* parentales une activité mannosidase insensible à l'action inhibitrice de la swainsonine. Inversement, les souches sélectionnées présentent une inhibition significative de l'activité mannosidase mesurée lors d'un traitement swainsonine. De plus, l'activité mannosidase II mesurée étant détectée dans les fractions golgiennes de levures, nous pouvons en déduire que cette enzyme est correctement exprimée dans la voie de sécrétion au niveau du système golgien.

Nomenclature des levures modifiées :
S.cerevisiae Arielle + Mannosidase II = Anaïs
S.pombe Erika + Mannosidase II = Enrique

### 14.6 Validation des clones ayant intégrés la cassette de la N-acétylglucosaminyl transférase II (GlcNAc transférase II) dans les levures modifiées :

### a- Validation par amplification PCR

Les clones sélectionnés pour *S.cerevisiae,* et *S.pombe* ont été testés par PCR pour vérifier la présence des cassettes d'expression de la GlcNAc transférase II dans le génome des levures (résultats non présentés).

### b- Expression de la GlcNAc transférase II :

L'expression de la GlcNAc transférase II a été analysée par RT-PCR sur des cultures de levures parentales ou modifiées. Après une étape de reverse transcription sur les ARN extraits, les cDNA ont été analysés par PCR en utilisant des primers spécifiques de la GlcNAc transférase II (PCR nichée). On observe donc dans les levures modifiées par la cassette d'expression de la GlcNAc transférase II une expression de l'ARNm transcrit (résultats non présentés).

Nomenclature des levures modifiées :
S.cerevisiae Anaïs + GlcNAc transférase II = Alice
S.pombe Enrique + GlcNAc transférase II = Elga

### 14.7 Validation des clones ayant intégrés la cassette Galactosyl transférase I :

### a- Validation par amplification PCR

Les clones sélectionnés pour *S.cerevisiae,* et *S.pombe* ont été testés par PCR pour vérifier la présence des cassettes d'expression de la GalTI dans le génome des levures (résultats non présentés).

### b- Expression de la Galactosyl transférase I :

L'expression de la GalTI a été analysée par RT-PCR sur des cultures de levures parentales ou modifiées. Après une étape de reverse transcription sur les ARN extraits, les cDNA ont été analysés par PCR en utilisant des primers spécifiques de la GalTI (PCR nichée). On observe donc dans les levures modifiées par la cassette d'expression de la GalTI une expression de l'ARNm transcrit (résultats non présentés).

### c- Activité de la GALTI

Après extraction des protéines totales des levures modifiées, 2µg de protéines sont déposées sur une membrane de nitrocellulose. La membrane est ensuite incubée avec la lectine erythrina cristagalli couplée à la biotine, lectine reconnaissant spécifiquement le galactose du motif Gal-β-1,4-GlcNAc présent sur les glycannes des glycoprotéines. La membrane est mise en contact avec de la steptavidine couplée à la horse radish peroxidase (HRP) afin de procéder à la détection par chimioluminescence (solution de détection Roche).

### 14.8 Validation des clones ayant intégrés la cassette du transporteur de GDP-fucose :

### a- Validation par amplification PCR

Les clones sélectionnés pour *S.cerevisiae,* et *S.pombe* ont été testés par PCR pour vérifier la présence des cassettes d'expression du transporteur de GDP-fucose dans le génome des levures.

### b- Expression du transporteur de GDP-fucose :

L'expression du transporteur de GDP-fucose a été analysée par RT-PCR sur des cultures de levures parentales ou modifiées. Après une étape de reverse transcription sur les ARN extraits, les cDNA ont été analysés par PCR en utilisant des primers spécifiques du transporteur de GDP-fucose (PCR nichée). On observe donc dans les levures modifiées par la cassette d'expression du transporteur de GDP-fucose une expression de l'ARNm transcrit **(****figure 10****).**
Nomenclature des levures modifiées :
S.cerevisiae Anaïs + GDP-fucose transporteur = Apoline
S.pombe Enrique + GDP-fucose transporteur = Epiphanie

### 14.9 Validation des clones ayant intégrés la cassette de la fucosyltransférase 8 (FUT8) :

### a- Validation par amplification PCR

Les clones sélectionnés pour *S.cerevisiae,* et *S.pombe* ont été testés par PCR pour vérifier la présence des cassettes d'expression de la FUT8 dans le génome des levures.

### b- Expression de la FUT8 :

L'expression de la FUT8 a été analysée par RT-PCR sur des cultures de levures parentales ou modifiées. Après une étape de reverse transcription sur les ARN extraits, les cDNA ont été analysés par PCR en utilisant des primers spécifiques de la FUT8 (PCR nichée). On observe donc dans les levures modifiées par la cassette d'expression de la FUT8 une expression de l'ARNm transcrit (résultats non présentés).
Nomenclature des levures modifiées :
S.cerevisiae Apoline + FUT8 = Ashley
S.pombe Epiphanie + FUT8 = Esther

### 14.10 Cas particulier de l'expression de l'EPO dans la souche Amélie.

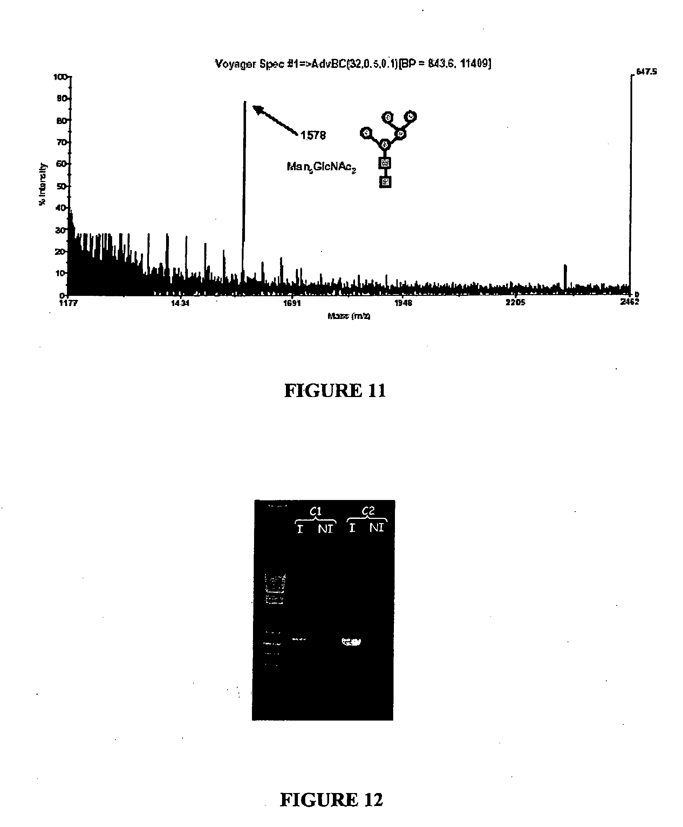

La souche Amélie possède la faculté de réaliser exclusivement le N-glycanne Man₅GlcNAc₂ **(****Figure 11****),** structure rencontrée chez les mammifères, qualifiée de glycanne de type simple ; et servant de base à l'élaboration de glycannes plus complexe porteurs de galactose, fucose ou acide sialique. La présence de chaque modification génomique dans cette souche est décrite ci-dessus. Chacune de ces étapes entre dans un « package » de vérifications consistant à choisir le meilleur clone producteur et à maximiser le pourcentage de chance d'obtenir un clone exploitable. La méthode utilisée permet un contrôle complet de la procédure de modification génétique : la séquence à intégrer est parfaitement connue, tout comme la région génomique cible de la future intégration. Ce dernier site fait par ailleurs l'objet de recherches approfondies quant aux effets d'une éventuelle cassure, c'est pourquoi l'ensemble des cibles est au final choisi pour l'absence d'effets phénotypiques obtenus à l'issue de leur cassure.

Toute une procédure de suivi de la stabilité génomique des clones producteurs est opérée : Après chaque production : repiquage régulier des clones sur les milieux drastiques initialement utilisés pour leur sélection, et remise en route de la procédure de validation. Toutes les cassettes d'expression sont clonées ce qui permet en cas de remaniement génomique d'une souche donnée, de procéder à une remise à niveau génétique de l'organisme. Les procédures d'intégration des cassettes sont à présent standardisées et l'on peut imaginer réaliser des souches « à la demande » afin de réaliser une glycosylation spécifique, commandée par l'utilisateur.
La souche Amélie est le clone qui doit servir de base à l'élaboration de toute autre souche destinée à produire des glycannes humanisés, hybrides ou complexes.

Le plasmide utilisé pour l'expression de l'EPO dans les levures modifiées contient le promoteur Gal1. Ce promoteur est l'un des promoteurs les plus forts connus chez S.cerevisiae, et est couramment utilisé pour les productions de protéines recombinantes. Ce promoteur est induit par le galactose et réprimé par le glucose. En effet, dans une culture de levures S.cerevisiae en glycérol, l'ajout de galactose permet l'induction des gènes GAL d'environ 1000 fois. Si du glucose est ajouté à cette culture en présence de galactose, les gènes GAL ne seront plus induit qu'à 1% du niveau obtenu avec le galactose seul (Johnston, M. (1987) Microbiol. Rev.). La séquence de l'EPO humaine intégrée dans notre plasmide a été modifiée en 5' par ajout d'un épitope V5 ainsi que d'un tag polyhistidine afin de facilité la détection et la purification de la protéine produite.

Les levures utilisées pour la production de l'EPO humaine sont tout d'abord mise en culture dans un milieu YNB drop out uracil, 2% glucose jusqu'à atteindre une DO>12. Au bout de 24 à 48 heures de culture, 2% de galactose sont rajoutés à la culture pour induire la production de notre protéine d'intérêt. Les prélèvements d'échantillons ont lieu à 0, 6, 24 et 48 heures d'induction.

### Expression de l'ARNm de l'EPO dans les levures modifiées:

L'analyse par RT-PCR des ARN totaux extraits montre une expression de l'ARN messager de l'EPO dans les clones de levures transformées après une induction par le galactose **(****figure 12** pistes 1 et 3) contrairement à ce qui est observé dans les levures modifiées sans induction par le galactose **(****figure 12** pistes 2 et 4). La présence de galactose provoque donc une induction de la transcription du gène EPO. Le séquençage de ce fragment amplifié confirme la production d'un ARNm correct.

### Purification de la protéine EPO exprimée dans les levures modifiées:

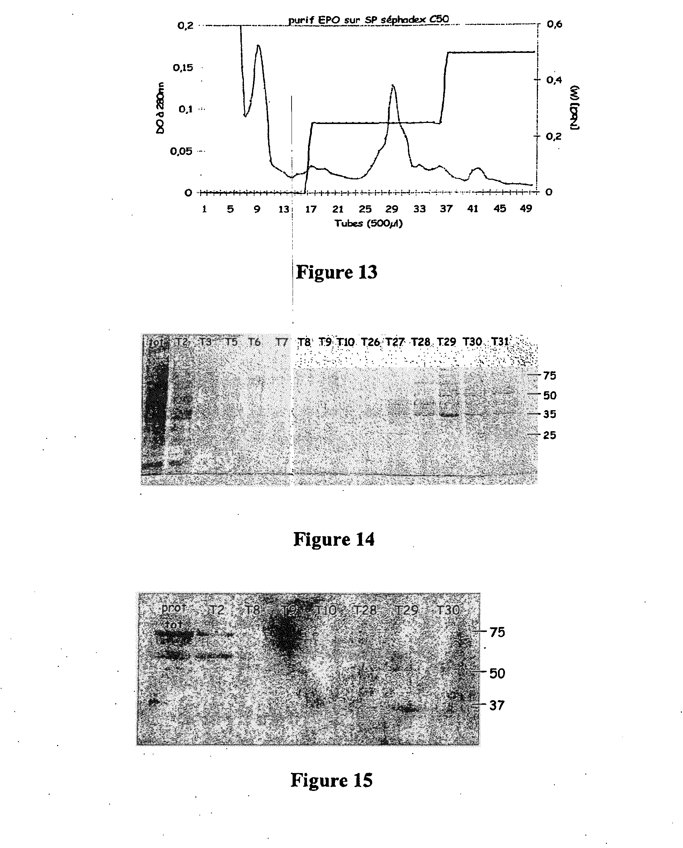

Les protéines totales obtenues après induction de l'expression de la protéine rhuEPO par le galactose sont ensuite déposées sur une résine de séphadex C50 équilibrée à pH 6. L'absorbance à 280 nm est déterminée en sortie de colonne **(****figure 13****).** Les protéines éluées de la colonne sont analysées par électrophorèse SDS-PAGE sur gel acrylamide 12%.
Après migration du gel SDS-PAGE, l'analyse des protéines se fait soit par coloration au bleu de coomassie **(****figure 14****)** soit par western blot. Dans ce cas, les protéines sont transférées sur une membrane de nitrocellulose afin de procéder à une détection par l'anticorps anti-EPO (R&D systems).

La **figure 15** nous montre la présence d'une protéine à environ 35 kDa. Cette protéine est majoritaire en coloration coomassie et révélée par un anticorps anti-EPO en analyse western blot (tube 29 en sortie de colonne).
Tous ces résultats indiquent donc la production de la protéine EPO par les levures génétiquement modifiées.

## Revendications

1. Levures génétiquement modifiées, capables de produire des glycoprotéines présentant des glycanes homogènes comprenant la structure Man₅GlcNAc₂, lesdites levures comprenant les modifications suivantes:
a) inactivation du gène *OCH1* codant l'alpha-1,6 mannosyl transférase par insertion par recombinaison homologue d'une séquence hétérologue codant pour un gène de résistance à une antibiotique (souche delta-*OCH1*), et
b) intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de S. *cerevisiae* ou S. *pombe,* et ADH2 possédant la séquence SEQ ID No 16-26 respectivement, une phase ouverte de lecture comprenant la séquence codante pour une alpha-1-2 mannosidase **I** comprenant une séquence de ciblage dans le réticulum endoplasmique ou l'appareil de Golgi et un terminateur de la transcription,
**caractérisées en ce que** lesdites levures sont choisies parmi *Saccharomyces cerevisiae* ou *Schizosaccharomyces pombe.*

2. Levures selon la revendication 1, **caractérisées en ce que** l'alpha-1-2 mannosidase I est l'alpha-1-2 mannosidase I de C. *elegans.*

3. Levures selon la revendication 2, **caractérisées en ce que** l'alpha-1-2 mannosidase I comprend la SEQ ID No 1.

4. Levures selon l'une des revendications 1 à 3, capables de produire des glycoprotéines présentant plus de 75% de la structure GlcMacMan₅GlcNAc₂, comprenant en outre l'intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement, une phase ouverte de lecture comprenant la séquence codante pour la N-acétyl-glucosaminyl transférase **I** humaine comprenant une séquence de ciblage dans le réticulum endoplasmique ou l'appareil de Golgi et un terminateur de la transcription.

5. Levures selon la revendication 4, **caractérisées en ce que** la N-acétyl-glucosaminyl transférase I humaine comprend la séquence SEQ ID No 2 sans la partie cytoplasmique de l'enzyme qui est remplacée par la partie cytoplasmique de mmn9 (SEQ ID No 13) pour une localisation golgienne de la protéine.

6. Levures selon la revendication 5, **caractérisées en ce qu'**elle comprennent en outre l'intégration par recombinaison homologue dans un marqueur d'auxotrophie d'une cassette d'expression comprenant un promoteur sélectionné parmi pGAP, pGAL1, PGK, 15 TEF, adh1, nmt1, SV40, PMA1, CaMV, pet56 de S. *cerevisiae* ou S. *pombe,* et ADH2 possédant la séquence SEQ ID No 16-26 respectivement, une phase ouverte de lecture comprenant la séquence codante pour le transporteur d'UDP-GlcNAc humain et un terminateur de la transcription.

7. Levures selon la revendication 5, **caractérisées en ce que** le transporteur d'UDPGlcNAc humain comprend la séquence SEQ ID No 3.

8. Levures selon l'une des revendications 5 à 7, capables de produire des glycoprotéines présentant plus de 75% de la structure GlcNacMan₃GlcNAc₂, comprenant en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de S. *cerevisiae* ou S. *pombe,* et ADH2 possédant la séquence SEQ ID No 16-26 respectivement, une phase ouverte de lecture comprenant la séquence codante pour une mannosidase Il comprenant une séquence de ciblage dans le réticulum endoplasmique ou l'appareil de Golgi et un terminateur de la transcription.

9. Levures selon la revendication 8, **caractérisées en ce que** la mannosidase II est la mannosidase II de souris et comprend la séquence SEQ ID No 4.

10. Levures selon l'une des revendications 8 ou 9, capables de produire des glycoprotéines présentant plus de 75% de la structure GlcNAc₂Man3GlcNAc₂, comprenant en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de S. *cerevisiae* ou S. *pombe,* et ADH2 possédant la séquence SEQ ID No 16-26 respectivement, une phase ouverte de lecture comprenant la séquence codante pour une *N*-acetyl-glucosaminyl transférase II, comprenant une séquence de ciblage dans le réticulum endoplasmique ou l'appareil de Golgi et un terminateur de la transcription.

11. Levures selon la revendication 10, **caractérisées en ce que** la N-acetyl-glucosaminyl transférase II est humaine, et comprend la séquence SEQ ID No 5.

12. Levures selon l'une des revendications 10 ou 11, capables de produire des glycoprotéines présentant plus de 75% de la structure Gal₂GlcNAc₂Man₃GlcNAc_{2,} comprenant en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement, une phase ouverte de lecture comprenant la séquence codante pour une galactosyltransférase I, comprenant une séquence de ciblage dans le réticulum endoplasmique ou l'appareil de Golgi et un terminateur de la transcription.

13. Levures selon la revendication 12, **caractérisées en ce que** la galactosyl transférase I est humaine, et comprend la séquence SEQ ID No 6, qui est dépourvue de la séquence de ciblage humaine.

14. Levures selon l'une des revendications 1 à 11, **caractérisées en ce que** le marqueur d'intégration est sélectionné parmi URA3, ADE2, LYS2, LEU2, TRP1, CAN1, ADO1, HIS5, HIS3, ARG3, MET17, LEM3, Mnn1, Mnn9, gma12.

15. Levures selon l'une des revendications 1 à 11, **caractérisées en ce que** la cassette d'expression de l'alpha-1-2 mannosidase I est intégrée dans le gène URA3, la cassette d'expression de la N acétyl-glucosaminyl transférase I est intégrée dans le gène ADE1 ou ADE2, la cassette d'expression du transporteur d'UDP-GlcNAc est intégrée dans le gène LYS2, la cassette d'expression de la-mannosidase II est intégrée dans le gène LEU2, et la cassette d'expression de la N-acetyl-glucosaminyl transférase II est intégrée dans le gène Lem3 ou TRP1.

16. Levures selon l'une des revendications 1 à 15, **caractérisées en ce que** la séquence de ciblage dans le réticulum endoplasmique ou l'appareil de Golgi est dérivée de la séquence de localisation du gène Mnt1 et comprend la séquence SEQ ID No 14.

17. Levures selon l'une des revendications 1 à 16, **caractérisées en ce que** le terminateur est dérivé du gène CYC1 et comprend la séquence SEQ ID No 15.

18. Levures selon l'une des revendications 4 à 11, capables de produire des glycoprotéines présentant plus de 75% d'une structure choisie parmi
Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GlcNac₂Man₃(Fuc)GlcNAc₂,
Gal₂GlcNac₂Man₃(Fuc)GlcNAc₂,
comprenant en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement ou le promoteur du gène MNT1, une phase ouverte de lecture comprenant la séquence codante pour une α-1,6-fucosyltransférase FUT8, comprenant une séquence de ciblage dans le réticulum endoplasmique ou l'appareil de Golgi et un terminateur de la transcription dérivé du gène CYC1 comprenant la séquence SEQ ID No 15.

19. Levures selon la revendication 18, **caractérisées en ce que** l'alpha-1,6-fucosyltransférase FUT8 est humaine et comprend la séquence SEQ ID No 7.

20. Levures selon l'une des revendications 18 et 19 comprenant en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement ou le promoteur SV40, une phase ouverte de lecture comprenant la séquence codante pour un transporteur de GDP fucose.

21. Levures selon la revendication 20, **caractérisées en ce que** le transporteur de GDP fucose comprend la SEQ ID No 8.

22. Levures selon l'une des revendications précédentes, **caractérisées en ce que** l'alpha-1-2 mannosidase I est exprimée sous le contrôle du promoteur pGAP et la glycoprotéine protéine exogène est exprimée sous le contrôle du promoteur pGAL1.

23. Levures selon l'une des revendications 12 à 22, capables de produire des glycoprotéines présentant plus de 75% d'une structure choisie parmi NANA₄Gal₄GlcNac₄Man₃GlcNAc₂ et NANA₄Gal₄GlcNac₄(Fuc)Man₃GlcNAc₂ **caractérisées en ce qu'**elles comprennent en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement ou le promoteur de la thymidine kinase du virus de l'herpès comprenant la séquence SEQ ID No 9, une phase ouverte de lecture comprenant la séquence codante pour une α-2,3 sialyltransférase et le terminateur dérivé du gène CYC1 comprenant la séquence SEQ ID No 15.

24. Levures selon la revendication 23 **caractérisées en ce que** la sialyltransférase est la ST4GAL4 humaine.

25. Levures selon la revendication 24, **caractérisées en ce que** la sialyltransférase comprend la SEQ ID No 10.

26. Levures selon l'une quelconque des revendications 23 à 25, comprenant en outre une intégration dans le génome d'une cassette d'expression comprenant le promoteur PET56 possédant la séquence SEQ ID No , la phase ouverte de lecture de l'acide sialique synthase de Neisseria meningitidis possédant la séquence SEQ ID No. , le terminateur CYC1 comprenant la séquence SEQ ID No 15, suivis du promoteur PET56, la phase ouverte de lecture de la CMP acide sialique synthase de N. meningitidis et du terminateur CYC1 comprenant la séquence SEQ ID No 15.

27. Levures selon la revendication 26, comprenant en outre une intégration par recombinaison homologue dans le gène MNN1 d'une cassette d'expression comprenant le promoteur CAMV possédant la séquence SEQ ID No. 21, le transporteur de CMP-acide sialique de Mus musculus , et le terminateur TEF de séquence 19.

28. Levures selon l'une quelconque des revendications 12 à 14 et 23 à 25, capables de produire des glycoprotéines présentant plus de 75%, voire 80% ou encore 95% ou 98% d'une structure choisie parmi Gal₂GlcNac₃Man₃GlcNAc₂ et NANA₂Gal₂GlcNac₃Man₃GlcNAc₂ **caractérisée en ce qu'**elles comprennent en outre une intégration par recombinaison homologue dans un marqueur d'auxotrophie d'une cassette d'expression comprenant un promoteur sélectionné parmi pGAP, pGAL1, PGK, TEF, adh1, nmt1, SV40, PMA1, CaMV, pet56 de *S*. *cerevisiae* ou *S*. *pombe,* et ADH2 possédant la séquence SEQ ID No 16-26 respectivement ou le promoteur de la thymidine kinase du virus de l'herpès comprenant la séquence SEQ ID No 9, une phase ouverte de lecture comprenant la séquence codante pour une β-1,4 N-acetylglucosaminyltransférase III et le terminateur dérivé du gène CYC1 comprenant la séquence SEQ ID No 15.

29. Levures selon la revendication 28, **caractérisées en ce que** la β-1,4 N-acetylglucosaminyltransférase III est murine.

30. Levures selon la revendication 29, **caractérisées en ce que** la β-1,4 N-acetylglucosaminyltransférase III comprend la séquence SEQ ID No. 27.

31. Levures selon l'une quelconque des revendications 1 à 25 et 28 à 30, **caractérisées en ce que** sont des levures de l'espèce S. *cerevisiae,* lesdites levures comprenant en outre une délétion du gène *MNN1.*

32. Levures selon l'une quelconque des revendications 1 à 25 et 28 à 30, **caractérisées en ce que** sont des levures de l'espèce *S*. *cerevisiae,* lesdites levures comprenant en outre une délétion du gène *MNN9.*

33. Levures selon l'une quelconque des revendications 1 à 25 et 28 à 30, **caractérisées en ce que** sont des levures de l'espèce *S*. *pombe,* lesdites levures comprenant en outre une délétion du gène *gma12.*

34. Levures selon l'une des revendications 1 à 33, **caractérisées en ce que** la glycoprotéine est choisie parmi les glycoprotéines à usage thérapeutique telles que des cytokines, des interleukines, des hormones de croissance, facteurs de croissance, des enzymes, et des anticorps monoclonaux, protéines vaccinales, récepteurs solubles et tous types de protéines recombinantes.

35. Levures selon la revendication 34, **caractérisées en ce que** la glycoprotéine est l'EPO, notamment une EPO ayant la SEQ ID No 12 comprenant l'épitope V5 et un motif poly-HIS en N-terminal pour purification.

36. Procédé de production d'une glycoprotéine possédant des structures glycanniques homogènes à plus de 75% d'une structure sélectionnée parmi :
Man₅GlcNAc₂,
GlcNacMan₅GlcNAc₂,
GlcNacMan₃GlcNAc₂,
GlcNaC₂Man₃GlcNAc₂.
Gal₂GlcNac₂Man₃GlcNAc₂
NANA2Gal2GlcNac2Man3GlcNAc2
GlcNac2Man3(Fuc)GlcNAc2
Gal2GlcNac2Man3(Fuc)GlcNAc2
NANA2Gal2GlcNac2Man3(Fuc)GlcNAc2
comprenant la mise en culture d'une levure selon l'une des revendications 1 à 33 dans un fermenteur, et l'extraction de ladite glycoprotéine du milieu de culture.

37. Utilisation d'une levure selon l'une des revendications 1 à 33 pour la production en fermenteur d'une glycoprotéine possédant des structures glycanniques homogènes à plus de 75% d'une structure sélectionnée parmi :
Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GlcNac₂Man₃(Fuc)GlcNAc₂,
Gal₂GlcNac₂Man₃(Fuc)GlcNAc₂.

## Patentansprüche

1. Gentechnisch veränderte Hefen, die in der Lage sind, Glykoproteine zu produzieren, die homogene Glykane zeigen und die die Struktur Man₅GlcNAc₂ aufweisen, wobei die Hefen die folgenden Modifikationen aufweisen:
a) Inaktivierung des Gens *OCH1,* das die alpha-1,6-Mannosyltransferase codiert, durch Einfügen mittels homologer Rekombination einer heterologen Sequenz, die ein Gen für die Resistenz gegen ein Antibiotikum codiert (Stamm Delta-*OCH1*), und
b) Integration einer Expressionskassette mittels homologer Rekombination in einen Marker für Auxotrophie, die einen Promotor aufweist, der aus pGAP, pGAL1, PGK, TEF, adh1, nmt1, SV40, PMA1, CaMV, pet56 von *S*. *cerevisiae* oder *S. pombe* und ADH2 ausgewählt ist, die jeweils die Sequenz SEQ ID No 16-26 besitzen, und die einen offenen Leserahmen mit der Sequenz für die Codierung einer alpha-1,2-Mannosidase I aufweist und eine Zielsequenz für das endoplasmatische Reticulum oder den Golgiapparat und einen Terminator für die Transkription aufweist,
**dadurch gekennzeichnet, dass** die Hefen unter *Saccharomyces cerevisiae* oder *Schizosaccharomyces pombe* ausgewählt sind.

2. Hefen nach Anspruch 1, **dadurch gekennzeichnet, dass** die alpha-1,2-Mannosidase I die alpha-1,2-Mannosidase I von *C. elegans* ist.

3. Hefen nach Anspruch 2, **dadurch gekennzeichnet, dass** die alpha-1,2-Mannosidase I die SEQ ID No 1 aufweist.

4. Hefen nach einem der Ansprüche 1 bis 3, die in der Lage sind, Glykoproteine zu erzeugen, die zu mehr als 75% die Struktur GlcNacMan₅GlcNAc₂ zeigen, die weiterhin die Integration einer Expressionskassette mittels homologer Rekombination in einen Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, und die einen offenen Leserahmen mit der Sequenz für die Kodierung von menschlicher N-Acetylglukosaminyltransferase I aufweist und eine Zielsequenz für das endoplasmatische Reticulum oder den Golgiapparat und einen Terminator für die Transkription aufweist.

5. Hefen nach Anspruch 4, **dadurch gekennzeichnet, dass** die menschliche N-Acetylglukosaminyltransferase I die Sequenz SEQ ID No 2 ohne den zytoplasmatischen Teil des Enzyms aufweist, der durch den zytoplasmatischen Teil von mmn9 (SEQ ID No 13) für eine Golgilokalisierung des Proteins ersetzt ist.

6. Hefen nach Anspruch 5, **dadurch gekennzeichnet, dass** diese weiterhin die Integration einer Expressionskassette mittels homologer Rekombination in einen Marker für Auxotrophie aufweisen, die einen Promotor aufweist, der aus pGAP, pGAL1, PGK, 15 TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, und die einen offenen Leserahmen mit der Sequenz für die Codierung von menschlichen UDP-GlcNAc und einen Terminator für die Transkription aufweist.

7. Hefen nach Anspruch 5, **dadurch gekennzeichnet, dass** der Transporter für menschliches UDPGlcNAc die Sequenz SEQ ID No 3 aufweist.

8. Hefen nach einem der Ansprüche 5 bis 7, die dazu geeignet sind, Glykoproteine zu erzeugen, die zu mehr als 75% die Struktur GlcNacMan₃GlcNAc₂ zeigen, die weiterhin die Integration einer Expressionskassette mittels homologer Rekombination in einem Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, und die ferner einen offenen Leserahmen mit der Sequenz für die Codierung einer Mannosidase II aufweist und eine Zielsequenz für das endoplasmatische Reticulum oder den Golgiapparat und einen Terminator für die Transkription aufweist.

9. Hefen nach Anspruch 8, **dadurch gekennzeichnet, dass** die Mannosidase II von Mäusen ist und die Sequenz SEQ ID No 4 aufweist.

10. Hefen nach einem der Ansprüche 8 oder 9, die in der Lage sind, Glykoproteine zu erzeugen, die zu mehr als 75% die Struktur GlcNAc₂Man3GlcNAc₂ zeigt, die weiterhin die Integration einer Expressionskassette mittels homologer Rekombination in einem Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, und die ferner einen offenen Leserahmen mit der Sequenz für die Codierung einer N-Acetylglukosaminyltransferase II aufweist und eine Zielsequenz für das endoplasmatische Reticulum oder den Golgiapparat und einen Terminator für die Transkription aufweist.

11. Hefen nach Anspruch 10, **dadurch gekennzeichnet, dass** die N-Acetylglukosaminyltransferase II menschlich ist und die Sequenz SEQ ID No 5 aufweist.

12. Hefen nach einem der Ansprüche 10 oder 11, die in der Lage sind, Glykoproteine zu erzeugen, die zu mehr als 75% die Struktur Gal₂GlcNAc₂Man₃GlcNAc₂ zeigen, und die weiterhin eine Integration einer Expressionskassette mittels homologer Rekombination in einen Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, und die einen offenen Leserahmen mit der Sequenz für die Codierung einer Galaktosyltransferase I aufweist und eine Zielsequenz für das endoplasmatische Reticulum oder den Golgiapparat und einen Terminator für die Transkription aufweist.

13. Hefen nach Anspruch 12, **dadurch gekennzeichnet, dass** die Galaktosyltransferase I menschlich ist und die Sequenz SEQ ID No 6 aufweist, von der die menschliche Zielsequenz entfernt worden ist.

14. Hefen nach einem der Ansprüche 1 bis 11, **dadurch gekennzeichnet, dass** der Marker für die Integration aus URA3, ADE2, LYS2, LEU2, TRP1, CAN1, AD01, HIS5, HIS3, ARG3, MET17, LEM3, Mnn1, Mnn9, gma12 ausgewählt ist.

15. Hefen nach einem der Ansprüche 1 bis 11, **dadurch gekennzeichnet, dass** die Expressionskassette für alpha-1,2-Mannosidase I in das Gen URA3 integriert ist, die Expressionskassette für N-Acetylglukosaminyltransferase I in das Gen ADE1 oder ADE2 integriert ist, die Expressionskassette für den Transporter UDP-GlcNAc in das Gen LYS2 integriert ist, die Expressionskassette für die *α-*Mannosidase II in das Gen LEU2 integriert ist und die Expressionskassette für N-Acetylglukosaminyltransferase II in das Gen Lem3 oder TRP1 integriert ist.

16. Hefen nach einem der Ansprüche 1 bis 15, **dadurch gekennzeichnet, dass** die Zielsequenz für das endoplasmatische Reticulum oder den Golgiapparat von der Lokalisierungssequenz des Gens Mnt1 abgeleitet ist und die Sequenz SEQ ID No 14 aufweist.

17. Hefen nach einem der Ansprüche 1 bis 16, **dadurch gekennzeichnet, dass** der Terminator vom Gen CYC1 abgeleitet ist und die Sequenz SEQ ID No 15 aufweist.

18. Hefen nach einem der Ansprüche 4 bis 11, die in der Lage sind, Glykoproteine zu erzeugen, die zu mehr als 75% eine Struktur zeigen, die ausgewählt ist aus Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GLcNac₂Man₃ (Fuc) GLcNAc₂,
Gal₂GLcNac₂Man₃ (Fuc) GlcNAc₂,
die ferner eine Integration einer Expressionskassette mittels homologer Rekombination in einen Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, oder den Promotor des Gens MNT1 aufweist und die einen offenen Leserahmen mit der Sequenz für die Codierung einer *α*-1,6-Fucosyltransferase FUT8, eine Zielsequenz für das endoplasmatische Reticulum oder den Golgiapparat und einen Terminator für die Transkription aufweist, der vom Gen CYC1 abgeleitet ist, das die Sequenz SEQ ID No 15 aufweist.

19. Hefen nach Anspruch 18, **dadurch gekennzeichnet, dass** die alpha-1,6-Fucosyltransferase FUT8 menschlich ist und die Sequenz SEQ ID No 7 aufweist.

20. Hefen nach einem der Ansprüche 18 und 19, die weiterhin eine Integration einer Expressionskassette mittels homologer Rekombination in einen Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, oder den Promotor SV40 aufweist und die einen offenen Leserahmen mit der Sequenz für die Codierung eines GDP Fucose Transporters aufweist.

21. Hefen nach Anspruch 20, **dadurch gekennzeichnet, dass** der GDP Fucose Transporter die SEQ ID No 8 aufweist.

22. Hefen nach einem der vorhergehenden Ansprüche, **dadurch gekennzeichnet, dass** die alpha-1,2-Mannosidase I unter der Kontrolle des Promotors pGAP exprimiert wird und das exogene Glykoprotein unter Kontrolle des Promotors pGAL1 exprimiert wird.

23. Hefen nach einem der Ansprüche 12 bis 22, die in der Lage sind, Glykoproteine zu erzeugen, die zu mehr als 75% eine Struktur zeigen, die aus NANA₄Gal₄GLcNac₄Man₃GlcNAc₂ und NANA₄Gal₄GlcNac₄(Fuc)Man₃GlcNAc₂, **dadurch gekennzeichnet, dass** diese weiterhin die Integration einer Expressionskassette mittels homologer Rekombination in einen Marker für Auxotrophie aufweisen, die einen Promotor einschließt, der aus pGAP, pGAL1, PGK, TEF, adh1, nmt1, SV40, PMA1, CaMV, pet56 von S. *cerevisiae* oder *S*. *pombe* und ADH2 ausgewählt ist, die jeweils die Sequenz SEQ ID No 16-26 besitzen, oder den Promotor für Thymidinkinase des Herpesvirus mit der Sequenz SEQ ID No 9 aufweist und die einen offenen Leserahmen mit der Sequenz für die Codierung einer *α*-2,3-Sialyltransferase und den Terminator aufweist, der vom Gen CYC1 mit der Sequenz SEQ ID No 15 abgeleitet ist.

24. Hefen nach Anspruch 23, **dadurch gekennzeichnet, dass** die Sialyltransferase die menschliche ST4GAL4 ist.

25. Hefen nach Anspruch 24, **dadurch gekennzeichnet, dass** die Sialyltransferase die SEQ ID No 10 aufweist.

26. Hefen nach einem der Ansprüche 23 bis 25, die weiterhin die Integration einer Expressionskassette in das Genom aufweist, die den Promotor PET56 aufweist, der die Sequenz SEQ ID No besitzt, und den offenen Leserahmen für Sialsäuresynthetase von Neisseria meningitidis aufweist, der die Sequenz SEQ ID No. besitzt, und den Terminator CYC1 mit der Sequenz SEQ ID No 15 aufweist, gefolgt vom Promotor PET56, dem offenen Leserahmen der CMP Sialinsäuresynthetase von N. meningitidis und dem Terminator CYC1 mit der Sequenz SEQ ID No 15.

27. Hefen nach Anspruch 26, die weiterhin eine Integration einer Expressionskassette mittels homologer Rekombination im Gen MNN1 aufweist, die den Promotor CAMV aufweist, der die Sequenz SEQ ID No. 21 besitzt, und den CMP Sialsäure Transporter von Mus musculus und den Terminator TEF der Sequenz 19 aufweist.

28. Hefen nach einem der Ansprüche 12 bis 14 und 23 bis 25, die in der Lage sind, Glykoproteine zu erzeugen, die zu mehr als 75%, sogar 80% oder weiter 95% oder 98% eine Struktur zeigen, die aus Gal₂GlcNac₃Man₃GlcNAc₂ und NANA₂Gal₂GlcNac₃Man₃GlcNAc₂ ausgewählt ist, **dadurch gekennzeichnet, dass** diese weiterhin die Integration einer Expressionskassette mittels homologer Rekombination in einen Marker für Auxotrophie aufweisen, die einen Promotor aufweist, der aus pGAP, pGAL1, PGK, TEF, adh1, nmt1, SV40, PMA1, CaMV, pet56 von *S. cerevisiae* oder *S. pombe* und ADH2 ausgewählt ist, die jeweils die Sequenz SEQ ID No 16-26 besitzen, oder den Promotor von Thymidinkinase des Herpesvirus mit der Sequenz SEQ ID No 9 aufweist und einen offenen Leserahmen mit der Sequenz für die Codierung einer β-1,4-N-Acetylglukosaminyltransferase III und den Terminator aufweist, der vom Gen CYC1 mit der Sequenz SEQ ID No 15 abgeleitet ist.

29. Hefen nach Anspruch 28, **dadurch gekennzeichnet, dass** die β-1,4-N-Acetylglukosaminyltransferase III murin ist.

30. Hefen nach Anspruch 29, **dadurch gekennzeichnet, dass** die β-1,4-N-Acetylglukosaminyltransferase III die Sequenz SEQ ID No. 27 ist.

31. Hefen nach einem der Ansprüche 1 bis 25 und 28 bis 30, **dadurch gekennzeichnet, dass** es sich um Hefen der Art *S*. *cerevisiae* handelt, wobei diese Hefen außerdem die Entfernung des Gens *MNN1* aufweisen.

32. Hefen nach einem der Ansprüche 1 bis 25 und 28 bis 30, **dadurch gekennzeichnet, dass** die Hefen von der Art S. *cerevisiae* sind, wobei die Hefen weiterhin die Entfernung des Gens *MNN9* aufweisen.

33. Hefen nach einem der Ansprüche 1 bis 25 und 28 bis 30, **dadurch gekennzeichnet, dass** die Hefen von der Art *S*. *pombe* sind, wobei die Hefen weiterhin die Entfernung des Gens *gma12* aufweisen.

34. Hefen nach einem der Ansprüche 1 bis 33, **dadurch gekennzeichnet, dass** die Glykoproteine aus den Glykoproteinen zur therapeutischen Verwendung, wie den Zytokinen, den Interleukinen, den Wachstumshormonen, den Wachstumsfaktoren, den Enzymen und den monoklonen Antikörpern, den Impfproteinen, den löslichen Rezeptoren und allen Arten von rekombinanten Proteinen ausgewählt sind.

35. Hefen nach Anspruch 34, **dadurch gekennzeichnet, dass** das Glykoprotein ein EPO ist, insbesondere ein EPO mit der SEQ ID No 12 mit dem Epitop V5 und einer Einheit N-terminales Poly-HIS für die Reinigung.

36. Verfahren zur Herstellung eines Glykoproteins mit homogenen Glykanstrukturen, das zu mehr als 75% eine Struktur aufweist, ausgewählt aus:
Man₅GlcNAc₂,
GlcNacMan₅GlcNAc₂,
GlcNacMan₃GlcNAc₂,
GlcNac₂Man₃GlcNAc₂.
Gal2GlcNac2Man3GlcNAc₂
NANA2Gal2GlcNac2Man3GlcNAc2
GlcNac2Man3(Fuc)GlcNAc2
Gal2GlcNac2Man3(Fuc)GlcNAc2
NANA2Gal2GlcNac2Man3(Fuc)GlcNAc2
wobei das Verfahren die Kultivierung einer Hefe nach einem der Ansprüche 1 bis 33 in einem Fermenter und die Extraktion des Glykoproteins aus dem Kulturmedium umfasst.

37. Verwendung einer Hefe nach einem der Ansprüche 1 bis 33 für die Herstellung eines Glykoproteins in einem Fermenter, das zu mehr als 75% eine Struktur aufweist, ausgewählt aus:
Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GlcNac₂Man₃ (Fuc) GlcNAc₂,
Gal₂GlcNac₂Man₃ (Fuc) GlcNAc₂.

## Claims

1. Genetically modified yeast, capable of producing glycoproteins having homogeneous glycans comprising the structure Man₅GlcNAc₂, said yeast comprising the following modifications:
a) inactivation of the *OCH1* gene encoding alpha-1,6 mannosyltransferase by insertion by homologous recombination of a heterologous sequence coding for an antibiotic-resistance gene (delta-*OCH1* strain), and
b) integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively, an open reading frame comprising the sequence coding for an alpha-1,2 mannosidase I comprising an endoplasmic reticulum- or Golgi apparatus-targeting sequence and a transcription terminator,
**characterised in that** said yeast is chosen from *Saccharomyces cerevisiae* or *Schizosaccharomyces pombe.*

2. Yeast according to claim 1, **characterised in that** the alpha-1,2 mannosidase I is the alpha-1,2 mannosidase I of C. *elegans.*

3. Yeast according to claim 2, **characterised in that** the alpha-1,2 mannosidase I comprises the sequence SEQ ID NO: 1.

4. Yeast according to one of claims 1 to 3, capable of producing glycoproteins having more than 75% of the structure GlcNacMan₅GlcNAc₂, further comprising the integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively, an open reading frame comprising the sequence coding for the human N-acetylglucosaminyltransferase I comprising an endoplasmic reticulum- or Golgi apparatus-targeting sequence and a transcription terminator.

5. Yeast according to claim 4, **characterised in that** the human N-acetylglucosaminyltransferase I comprises the sequence SEQ ID NO: 2 without the cytoplasmic part of the enzyme, which is replaced by the cytoplasmic part of mmn9 (SEQ ID NO: 13) for a golgian localisation of the protein.

6. Yeast according to claim 5, **characterised in that** it further comprises the integration by homologous recombination in an auxotrophy marker of an expression cassette comprising a promoter selected from pGAP, pGAL1, PGK, 15 TEF, adh1, nmt1, SV40, PMA1, CaMV, pet56 of *S. cerevisiae* or *S. pombe,* and ADH2 having the sequence SEQ ID NO: 16-26 respectively, an open reading frame comprising the sequence coding for the transporter of human UDP-GlcNAc and a transcription terminator.

7. Yeast according to claim 5, **characterised in that** the human UDP-GlcNAc transporter comprises the sequence SEQ ID NO: 3.

8. Yeast according to one of claims 5 to 7, capable of producing glycoproteins having more than 75% of the structure GlcNacMan₅GlcNAc₂, further comprising the integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively, an open reading frame comprising the sequence coding for a mannosidase II comprising an endoplasmic reticulum- or Golgi apparatus-targeting sequence and a transcription terminator.

9. Yeast according to claim 8, **characterised in that** the mannosidase II is mouse mannosidase II and comprises the sequence SEQ ID NO: 4.

10. Yeast according to one of claims 8 or 9, capable of producing glycoproteins having more than 75% of the structure GlcNAc₂Man3GlcNAc₂, further comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively, an open reading frame comprising the sequence coding for an N-acetyl-glucosaminyltransferase II, comprising an endoplasmic reticulum- or Golgi apparatus-targeting sequence and a transcription terminator.

11. Yeast according to claim 10, **characterised in that** the N-acetyl-glucosaminyltransferase II is human and comprises the sequence SEQ ID NO: 5.

12. Yeast according to claims 10 or 11, capable of producing glycoproteins having more than 75% of the structure Gal₂GlcNAc₂Man₃GlcNAc₂, further comprising the integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2. having the sequence SEQ ID NO: 16-26 respectively, an open reading frame comprising the sequence coding for a galactosyltransferase I, comprising an endoplasmic reticulum- or Golgi apparatus-targeting sequence and a transcription terminator.

13. Yeast according to claim 12, **characterised in that** the galactosyltransferase I is human, and comprises the sequence SEQ ID NO: 6, which is devoid of the human targeting sequence.

14. Yeast according to one of claims 1 to 11, **characterised in that** the integration marker is selected from URA3, ADE2, LYS2, LEU2, TRP1, CAN1, ADO1, HIS5, HIS3, ARG3, MET17, LEM3, Mnn1, Mnn9, gma12.

15. Yeast according to one of claims 1 to 11, **characterised in that** the expression cassette of alpha-1,2 mannosidase I is integrated in the URA3 gene, the expression cassette of N-acetylglucosaminyltransferase I is integrated in the ADE1 or ADE2 gene, the expression cassette of the UDP-GlcNAc transporter is integrated in the LYS2 gene, the expression cassette of α-mannosidase II is integrated in the LEU2 gene, and the expression cassette of N-acetylglucosaminyltransferase II is integrated in the Lem3 or TRP1 gene.

16. Yeast according to one of claims 1 to 15, **characterised in that** the endoplasmic reticulum- or Golgi apparatus-targeting sequence is derived from the localisation sequence of the Mnt1 gene and comprises the sequence SEQ ID NO: 14.

17. Yeast according to one of claims 1 to 16, **characterised in that** the terminator is derived from the CYC1 gene and comprises the sequence SEQ ID NO: 15.

18. Yeast according to one of claims 4 to 11, capable of producing glycoproteins having more than 75% of a structure chosen from
Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GlcNac₂Man₃ (Fuc) GlcNAc₂,
Gal₂GlcNac₂Man₃ (Fuc) GlcNAc₂,
also comprising integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively or the promoter of the MNT1 gene, an open reading frame comprising the sequence coding for an α-1,6-fucosyltransferase FUT8, comprising an endoplasmic reticulum- or Golgi apparatus-targeting sequence and a transcription terminator derived from the CYC1 gene comprising the sequence SEQ ID NO: 15.

19. Yeast according to claim 18, **characterised in that** the alpha-1,6-fucosyltransferase FUT8 is human and comprises the sequence SEQ ID NO: 7.

20. Yeast according to one of claims 18 and 19, further comprising an integration by homologous recombination in an auxotrophy marker of an expression cassette comprising a promoter selected from pGAP, pGAL1, PGK, TEF, adh1, nmt1, SV40, PMA1, CaMV, pet56 of S. *cerevisiae* or *S. pombe,* and ADH2 having the sequence SEQ ID NO: 16-26 respectively or the promoter SV40, an open reading frame comprising the sequence coding for a GDP fucose transporter.

21. Yeast according to claim 20, **characterised in that** the GDP fucose transporter comprises the sequence SEQ ID NO: 8.

22. Yeast according to one of the preceding claims, **characterised in that** the alpha-1,2 mannosidase I is expressed under the control of the pGAP promoter and the exogenous glycoprotein is expressed under the control of the pGAL1 promoter.

23. Yeast according to one of claims 12 to 22, capable of producing glycoproteins having more than 75% of a structure chosen from NANA₄Gal₄GlcNac₄Man₃GlcNAc₂ and NANA₄Gal₄GlcNac₄(Fuc)Man₃GlcNAc₂, **characterised in that** it further comprises the integration by homologous recombination in an auxotrophy marker of an expression cassette comprising a promoter selected from pGAP, pGAL1, PGK, TEF, adh1, nmt1, SV40, PMA1, CaMV, pet56 of *S*. *cerevisiae* or *S. pombe,* and ADH2 having the sequence SEQ ID NO: 16-26 respectively or the thymidine kinase promoter of the herpes virus comprising the sequence SEQ ID NO: 9, an open reading frame comprising the sequence coding for an α-2, 3 sialyltransferase and the terminator derived from the CYC1 gene comprising the sequence SEQ ID NO: 15.

24. Yeast according to claim 23, **characterised in that** the sialyltransferase is the human ST4GAL4.

25. Yeast according to claim 24, **characterised in that** the sialyltransferase comprises the sequence SEQ ID NO: 10.

26. Yeast according to any one of claims 23 to 25, further comprising an integration in the genome of an expression cassette comprising the promoter PET56 having the sequence SEQ ID NO: , the open reading frame of the sialic acid synthase of *Neisseria meningitidis* having the sequence SEQ ID NO: , the CYC1 terminator comprising the sequence SEQ ID NO: 15, followed by the PET56 promoter, the open reading frame of CMP-sialic acid synthase of *N. meningitidis,* and of the CYC1 terminator comprising the sequence SEQ ID NO: 15.

27. Yeast according to claim 26, further comprising an integration by homologous recombination in the MNN1 gene of an expression cassette comprising the CAMV promoter having the sequence SEQ ID NO: 21, the transporter of CMP-sialic acid of *Mus musculus,* and the TEF terminator of sequence 19.

28. Yeast according to any one of claims 12 to 14 and 23 to 25, capable of producing glycoproteins having more than 75% or even 80% or 95% or 98% of a structure chosen from Gal₂GlcNac₃Man₃GlcNAc₂ and NANA₂Gal₂GlcNac₃Man₃GlcNAc₂, **characterised in that** it further comprises the integration by homologous recombination in an auxotrophy marker of an expression cassette comprising a promoter selected from pGAP, pGAL1, PGK, TEF, adh1, nmt1, SV40, PMA1, CaMV, pet56 of *S. cerevisiae* or *S. pombe,* and ADH2 having the sequence SEQ ID NO: 16-26 respectively or the thymidine kinase promoter of the herpes virus comprising the sequence SEQ ID NO: 9, an open reading frame comprising the sequence coding for a β-1,4 N-acetylglucosaminyltransferase III and the terminator derived from the CYC1 gene comprising the sequence SEQ ID NO: 15.

29. Yeast according to claim 28, **characterised in that** the β-1,4 N-acetylglucosaminyltransferase III is murine.

30. Yeast according to claim 29, **characterised in that** the β-1,4 N-acetylglucosaminyltransferase III comprises the sequence SEQ ID NO: 27.

31. Yeast according to any one of claims 1 to 25 and 28 to 30, **characterised in that** it is yeast of the species *S. cerevisiae,* said yeast further comprising a deletion of the *MNN1* gene.

32. Yeast according to any one of claims 1 to 25 and 28 to 30, **characterised in that** it is yeast of the species *S. cerevisiae,* said yeast further comprising a deletion of the *MNN9* gene.

33. Yeast according to any one of claims 1 to 25 and 28 to 30, **characterised in that** it is yeast of the species *S. pombe,* said yeast further comprising a deletion of the *gma12* gene.

34. Yeast according to one of claims 1 to 33, **characterised in that** the glycoprotein is chosen from glycoproteins for therapeutic use such as cytokines, interleukins, growth hormones, growth factors, enzymes, monoclonal antibodies, vaccine proteins, soluble receptors and all types of recombinant protein.

35. Yeast according to claim 34, **characterised in that** the glycoprotein is EPO, in particular an EPO having SEQ ID NO: 12 comprising the V5 epitope and a poly-HIS motif at the N-terminus for purification.

36. A method for producing a glycoprotein having homogeneous glycan structures with more than 75% of a structure selected from:
Man₅GlcNAC₂,
GlcNacMan₅GlcNAc₂,
GlcNacMan₃GlcNAC₂,
GlcNac₂Man₃GlcNAc₂,
Gal2GlcNac2Man3GlcNAc₂,
NANA2Gal2GlcNac2Man3GlcNAc2,
GlcNac2Man3(Fuc)GlcNAc2,
Gal2GlcNac2Man3(Fuc)GlcNAc2,
NANA2Gal2GlcNac2Man3(Fuc)GlcNAc2
comprising the culturing of a yeast according to one of claims 1 to 33 in a fermenter, and the extraction of said glycoprotein from the culture medium.

37. Use of a yeast according to one of claims 1 to 33 for producing in a fermenter a glycoprotein having homogeneous glycan structures with more than 75% of a structure selected from:
Man₅GlcNAc₂,
GlcNacMan₅GlcNAc₂,
GlcNacMan₃GlcNAc₂,
GlcNac₂Man₃GlcNAc₂,
Gal₂GlcNac₂Man₃GlcNAc₂,
GlcNac₂Man₃ (Fuc) GlcNAc₂,
Gal₂GlcNac₂Man₃ (Fuc) GlcNAc₂.


### LISTE DE SEQUENCES

<110> Glycode SAS
<120> Levures génétiquement modifiées pour la production de glycoprotéines homogènes
<130> D25151
<150> FR0753050
   <151> 2007-02-02
<160> 27
<170> PatentIn version 3.3
<210> 1
   <211> 1644
   <212> ADN
   <213> Caenorhabditis elegans
<220>
   <223> alpha-1-2 mannosidase I
<400> 1
   aaagcaggca tgggcctccg atcacacgaa caacttgtcg tgtgtgtcgg agttatgttt      60
   cttctgactg tctgcatcac agcgtttttc tttcttccgt caggcggcgc tgatctgtat     120
   ttccgagaag aaaactccgt tcacgttaga gatgtgctta tcagagagga aattcgtcgt     180
   aaagagcaag atgagttacg gcggaaagcc gaagaagcca atcccattcc aattccaaaa     240
   cctgaaattg gagcatcaga tgatgcagaa ggacgaagaa ttttcgtgaa acaaatgatt     300
   aaattcgcat gggacggata tcggaaatat gcctgggggg agaatgaatt gaggcccaac     360
   agtagatcag gacattcttc atcgatattt gggtatggaa agacgggtgc aacaattatt     420
   gatgctattg atacattgta tttggttgga ttaaaagaag aatataaaga ggccagagac     480
   tggattgctg attttgattt caaaacgtct gcgaaaggag atctatcagt ttttgaaaca      540
   aatatccgat tcactggtgg cctactctcc gcatttgcac ttaccggaga caaaatgttc     600
   ttgaagaaag cagaagatgt ggcaactatt cttcttccgg cttttgaaac tccttctgga     660
   ataccaaatt cattaattga tgctcaaaca ggaagatcca aaacgtatag ttgggcaagc     720
   ggaaaggcaa ttctctcgga atacggttca attcaacttg aattcgatta tctctccaat     780
   ctgactggaa atccagtttt tgctcaaaaa gctgataaaa taagagatgt tttaactgca     840
   atggagaaac cagaaggact ttatccaatt tatattacta tggataatcc accaagatgg     900
   ggacaacatc ttttctcaat gggtgcaatg gctgacagtt ggtatgaata tctgctcaaa     960
   caatggattg ccactggtaa aaaagatgat cgcacgaaaa gagaatacga agaagcgata    1020
   tttgcaatgg aaaaacgaat gcttttcaaa tcggaacagt cgaatctttg gtatttcgca    1080
   aaaatgaacg gaaatcgcat ggaacattca tttgaacatc ttgcatgctt ttccggtgga    1140
   atggttgttc ttcatgcaat gaatgagaaa aataaaacaa tatcagatca ttatatgacg    1200
   ttgggaaaag aaattggtca tacatgtcat gaatcgtacg ctagatccac aactggaatc    1260
   ggcccagaat ccttccaatt cacatcgagt gtagaggcaa aaacagaacg tcgtcaggat    1320
   tcatattata ttcttcgtcc tgaagtcgtt gagacatggt tctacttgtg gagggctaca    1380
   aaagacgaga aatatcgaca atgggcttgg gatcatgttc aaaatttgga ggagtattgt    1440
   aagggcactg ccggatactc tggaatccga aacgtctacg aatcgagccc ggaacaagat    1500
   gatgtgcagc agtcattcct cttcgctgag ctcttcaaat atctgtattt aattttcagt    1560
   gaagataaca ttcttccact tgatcaatgg gttttcaata ccgaagctca tccattccgc    1620
   attcggcatc acgacgagtt gatt                                           1644
<210> 2
   <211> 1387
   <212> ADN
   <213> Homo sapiens
<220>
   <223> *N*-acétyl-glucosaminyl transférase I comprenant la partie cytoplasmique de Mnn9 pour une localisation golgienne
<400> 2
   atgtcacttt ctcttgtatc gtaccgccta agaaagaacc cgtgggttaa cgcagggctt     60
   gtgctgtggg gcgctatcct ctttgtggcc tggaatgccc tgctgctcct cttcttctgg    120
   acgcgcccag cacctggcag gccaccctca gtcagcgctc tcgatggcga ccccgccagc    180
   ctcacccggg aagtgattcg cctggcccaa gacgccgagg tggagctgga gcggcagcgt    240
   gggctgctgc agcagatcgg ggatgccctg tcgagccagc gggggagggt gcccaccgcg    300
   gcccctcccg cccagccgcg tgtgcctgtg acccccgcgc cggcggtgat tcccatcctg    360
   gtcatcgcct gtgaccgcag cactgttcgg cgctgcctgg acaagctgct gcattatcgg    420
   ccctcggctg agctcttccc catcatcgtt agccaggact gcgggcacga ggagacggcc    480
   caggccatcg cctcctacgg cagcgcggtc acgcacatcc ggcagcccga cctgagcagc    540
   attgcggtgc cgccggacca ccgcaagttc cagggctact acaagatcgc gcgccactac    600
   cgctgggcgc tgggccaggt cttccggcag tttcgcttcc ccgcggccgt ggtggtggag    660
   gatgacctgg aggtggcccc ggacttcttc gagtactttc gggccaccta tccgctgctg    720
   aaggccgacc cctccctgtg gtgcgtctcg gcctggaatg acaacggcaa ggagcagatg    780
   gtggacgcca gcaggcctga gctgctctac cgcaccgact ttttccctgg cctgggctgg    840
   ctgctgttgg ccgagctctg ggctgagctg gagcccaagt ggccaaaggc cttctgggac    900
   gactggatgc ggcggccgga gcagcggcag gggcgggcct gcatacgccc tgagatctca    960
   agaacgatga cctttggccg caagggtgtg agccacgggc agttctttga ccagcacctc   1020
   aagtttatca agctgaacca gcagtttgtg cacttcaccc agctggacct gtcttacctg   1080
   cagcgggagg cctatgaccg agatttcctc gcccgcgtct acggtgctcc ccagctgcag   1140
   gtggagaaag tgaggaccaa tgaccggaag gagctggggg aggtgcgggt gcagtatacg   1200
   ggcagggaca gcttcaaggc tttcgccaag gctctgggtg tcatggatga ccttaagtcg   1260
   ggggttccga gagctggcta ccggggtatt gtcaccttcc agttccgggg ccgccgtgtc   1320
   cacctggcgc ccccactgac gtgggagggc tatgatccta gctggaatta gcacctgcct   1380
   gtccttc                                                             1387
<210> 3
   <211> 916
   <212> ADN
   <213> Homo sapiens
<220>
   <223> Transporteur d'UDP-GlcNAc
<400> 3
   atgttcgcca acctaaaata cgtttccctg ggaattttgg tctttcagac taccagtttg     60
   gttctaacaa tgcgttattc cagaacttta aaagaagaag gacctcgtta tctatcttct    120
   acagcagtgg ttgttgctga acttttgaag ataatggcct gcattttatt ggtctacaaa    180
   gacagcaaat gtagtctaag agcactgaat cgagtactac atgatgaaat tcttaataaa    240
   cctatggaaa cacttaaact tgctattcca tcagggatct atactcttca gaataattta    300
   ctgtatgtgg cactatcaaa tctagatgca gctacttatc aggtcacgta tcagttgaaa    360
   attcttacaa cagcattatt ttctgtgtct atgcttagta aaaaattggg tgtataccag    420
   tggctgtccc tagtaatttt gatgacagga gttgcttttg tacagtggcc ctcagattct    480
   cagcttgatt ctaaggaact ttcagctggt tctcaatttg taggactcat ggcagttctc    540
   acagcatgtt tttcaagtgg ctttgctggg gtttactttg agaaaatctt aaaagaaaca    600
   aaacaatcag tgtggataag aaatattcag cttggtttct ttggaagtat atttggatta    660
   atgggtgtat acatttatga tggagaactg gtatcaaaga atggattttt tcagggatat    720
   aaccgactga cctggatagt agttgttctt caggcacttg gaggccttgt aatagctgct    780
   gttattaagt atgcagataa tattttaaaa ggatttgcaa cctctttatc gataatatta    840
   tcaacattga tctcctattt ttggcttcaa gattttgtgc caaccagtgt ctttttcctt    900
   ggagccatcc ttgtaa                                                    916
<210> 4
   <211> 3453
   <212> ADN
   <213> Mus musculus
<220>
   <223> Mannosidase II
<400> 4
   atgaagttaa gtcgccagtt caccgtgttt ggcagcgcga tcttctgcgt cgtaatcttc     60
   tcactctacc tgatgctgga caggggtcac ttggactacc ctcggggccc gcgccaggag    120
   ggctcctttc cgcagggcca gctttcaata ttgcaagaaa agattgacca tttggagcgt    180
   ttgctcgctg agaacaacga gatcatctca aatatcagag actcagtcat caacctgagc    240
   gagtctgtgg aggacggccc gcgggggtca ccaggcaacg ccagccaagg ctccatccac    300
   ctccactcgc cacagttggc cctgcaggct gaccccagag actgtttgtt tgcttcacag    360
   agtgggagtc agccccggga tgtgcagatg ttggatgttt acgatctgat tccttttgat    420
   aatccagatg gtggagtttg gaagcaagga tttgacatta agtatgaagc ggatgagtgg    480
   gaccatgagc ccctgcaagt gtttgtggtg cctcactccc ataatgaccc aggttggttg    540
   aagactttca atgactactt tagagacaag actcagtata tttttaataa catggtccta    600
   aagctgaaag aagactcaag caggaagttt atgtggtctg agatctctta ccttgcaaaa    660
   tggtgggata ttatagatat tccgaagaag gaagctgtta aaagtttact acagaatggt    720
   cagctggaaa ttgtgaccgg tggctgggtt atgcctgatg aagccactcc acattatttt    780
   gccttaattg accaactaat tgaagggcac caatggctgg aaaaaaatct aggagtgaaa    840
   cctcgatcgg gctgggccat agatcccttt ggtcattcac ccacaatggc ttatcttcta    900
   aagcgtgctg gattttcaca catgctcatc cagagagtcc attatgcaat caaaaaacac    960
   ttctctttgc ataaaacgct ggagtttttc tggagacaga attgggatct tggatctgct   1020
   acagacattt tgtgccatat gatgcccttc tacagctacg acatccctca cacctgtggg   1080
   cctgatccta aaatatgctg ccagtttgat tttaaacggc ttcctggagg cagatatggt   1140
   tgtccctggg gagttccccc agaagcaata tctcctggaa atgtccaaag cagggctcag   1200
   atgctattgg atcagtaccg gaaaaagtca aaacttttcc gcactaaagt tctgctggct   1260
   ccactgggag acgactttcg gttcagtgaa tacacagagt gggatctgca gtgcaggaac   1320
   tacgagcaac tgttcagtta catgaactcg cagcctcatc tgaaagtgaa gatccagttt   1380
   ggaaccttgt cagattattt cgacgcattg gagaaagcgg tggcagccga gaagaagagt   1440
   agccagtctg tgttccctgc cctgagtgga gacttcttca cgtacgctga cagagacgac   1500
   cattactgga gtggctactt cacgtccaga cctttctaca aacgaatgga cagaataatg   1560
   gaatctcgta taagggctgc tgaaattctt taccagttgg ccttgaaaca agctcagaaa   1620
   tacaagataa ataaatttct ttcatcacct cattacacaa cactgacaga agccagaagg   1680
   aacttaggac tatttcagca tcatgatgcc atcacaggaa ccgcgaaaga ctgggtggtt   1740
   gtggactatg gtaccagact ctttcagtca ttaaattctt tggagaagat aattggagat   1800
   tctgcatttc ttctcatttt aaaggacaaa aagctgtacc agtcagatcc ttccaaagcc   1860
   ttcttagaga tggatacgaa gcaaagttca caagattctc tgccccaaaa aattataata   1920
   caactgagcg cacaggagcc aaggtacctt gtggtctaca atccctttga acaagaacgg   1980
   cattcagtgg tgtccatccg ggtaaactcc gccacaggga aagtgctgtc tgattcggga   2040
   aaaccggtgg aggttcaagt cagtgcagtt tggaacgaca tgaggacaat ttcacaagca   2100
   gcctatgagg tttcttttct agctcatata ccaccactgg gactgaaagt gtttaagatc   2160
   ttagagtcac aaagttcaag ctcacacttg gctgattatg tcctatataa taatgatgga   2220
   ctagcagaaa atggaatatt ccacgtgaag aacatggtgg atgctggaga tgccataaca   2280
   atagagaatc ccttcctggc gatttggttt gaccgatctg ggctgatgga gaaagtgaga   2340
   aggaaagaag acagtagaca gcatgaactg aaggtccagt tcctgtggta cggaaccacc   2400
   aacaaaaggg acaagagcgg tgcctacctc ttcctgcctg acgggcaggg ccagccatat   2460
   gtttccctaa gaccgccctt tgtcagagtg acacgtggaa ggatctactc agatgtgacc   2520
   tgtttcctcg aacacgttac tcacaaagtc cgcctgtaca acattcaggg aatagaaggt   2580
   cagtccatgg aagtttctaa tattgtaaac atcaggaatg tgcataaccg tgagattgta   2640
   atgagaattt catctaaaat aaacaaccaa aatagatatt atactgacct aaatggatat   2700
   cagattcagc ctagaaggac catgagcaaa ttgcctcttc aagccaacgt ttacccgatg   2760
   tgcacaatgg cgtatatcca ggatgctgag caccggctca cgctgctctc tgctcagtct   2820
   ctaggtgctt ccagcatggc ttctggtcag attgaagtct tcatggatcg aaggctcatg   2880
   caggatgata accgtggcct tgggcaaggc gtccatgaca ataagattac agctaatttg   2940
   tttcgaatcc tcctcgagaa gagaagcgct gtgaacatgg aagaagaaaa gaagagccct   3000
   gtcagctacc cttccctcct cagccacatg acttcgtcct tcctcaacca tccctttctc  3060
   cccatggtac taagtggcca gctcccctcc cctgcctttg agctgctgag tgaatttcct   3120
   ctgctgcagt cctctctacc ttgtgatatc catctggtca acctgcggac aatacaatca   3180
   aagatgggca aaggctattc ggatgaggca gccttgatcc tccacaggaa agggtttgat   3240
   tgccagttct ccagcagagg catcgggcta ccctgttcca ctactcaggg aaagatgtca   3300
   gttctgaaac ttttcaacaa gtttgctgtg gagagtctcg tcccttcctc tctgtccttg   3360
   atgcactccc ctccagatgc ccagaacatg agtgaagtca gcctgagccc catggagatc   3420
   agcacgttcc gtatccgctt gcgttggacc tga                                3453
<210> 5
   <211> 1344
   <212> ADN
   <213> Homo sapiens
<220>
   <223> *N*-acetyl-glucosaminyl transférase II
<400> 5
   atgaggttcc gcatctacaa acggaaggtg ctaatcctga cgctcgtggt ggccgcctgc     60
   ggcttcgtcc tctggagcag caatgggcga caaaggaaga acgaggccct cgccccaccg    120
   ttgctggacg ccgaacccgc gcggggtgcc ggcggccgcg gtggggacca cccctctgtg    180
   gctgtgggca tccgcagggt ctccaacgtg tcggcggctt ccctggtccc ggcggtcccc    240
   cagcccgagg cggacaacct gacgctgcgg taccggtccc tggtgtacca gctgaacttt    300
   gatcagaccc tgaggaatgt agataaggct ggcacctggg ccccccggga gctggtgctg    360
   gtggtccagg tgcataaccg gcccgaatac ctcagactgc tgctggactc acttcgaaaa    420
   gcccagggaa ttgacaacgt cctcgtcatc tttagccatg acttctggtc gaccgagatc    480
   aatcagctga tcgccggggt gaatttctgt ccggttctgc aggtgttctt tcctttcagc    540
   attcagttgt accctaacga gtttccaggt agtgacccta gagattgtcc cagagacctg    600
   ccgaagaatg ccgctttgaa attggggtgc atcaatgctg agtatcccga ctccttcggc    660
   cattatagag aggccaaatt ctcccagacc aaacatcact ggtggtggaa gctgcatttt    720
   gtgtgggaaa gagtgaaaat tcttcgagat tatgctggcc ttatactttt cctagaagag    780
   gatcactact tagccccaga cttttaccat gtcttcaaaa agatgtggaa actgaagcag    840
   caagagtgcc ctgaatgtga tgttctctcc ctggggacct atagtgccag tcgcagtttc    900
   tatggcatgg ctgacaaggt agatgtgaaa acttggaaat ccacagagca caatatgggt    960
   ctagccttga cccggaatgc ctatcagaag ctgatcgagt gcacagacac tttctgtact   1020
   tatgatgatt ataactggga ctggactctt caatacttga ctgtatcttg tcttccaaaa   1080
   ttctggaaag tgctggttcc tcaaattcct aggatctttc atgctggaga ctgtggtatg   1140
   catcacaaga aaacctgtag accatccact cagagtgccc aaattgagtc actcttaaat   1200
   aataacaaac aatacatgtt tccagaaact ctaactatca gtgaaaagtt tactgtggta   1260
   gccatttccc cacctagaaa aaatggaggg tggggagata ttagggacca tgaactctgt   1320
   aaaagttata gaagactgca gtga                                          1344
<210> 6
   <211> 1047
   <212> ADN
   <213> Séquence artificielle
<220>
   <223> Galactosyl transférase I dépourvue de la séquence de ciblage humaine
<400> 6
   ccccaactgg tcggagtctc cacaccgctg cagggcggct cgaacagtgc cgccgccatc     60
   gggcagtcct ccggggagct ccggaccgga ggggcccggc cgccgcctcc tctaggcgcc    120
   tcctcccagc cgcgcccggg tggcgactcc agcccagtcg tggattctgg ccctggcccc    180
   gctagcaact tgacctcggt cccagtgccc cacaccaccg cactgtcgct gcccgcctgc    240
   cctgaggagt ccccgctgct tgtgggcccc atgctgattg agtttaacat gcctgtggac    300
   ctggagctcg tggcaaagca gaacccaaat gtgaagatgg gcggccgcta tgcccccagg    360
   gactgcgtct ctcctcacaa ggtggccatc atcattccat tccgcaaccg gcaggagcac    420
   ctcaagtact ggctatatta tttgcaccca gtcctgcagc gccagcagct ggactatggc    480
   atctatgtta tcaaccaggc gggagacact atattcaatc gtgctaagct cctcaatgtt    540
   ggctttcaag aagccttgaa ggactatgac tacacctgct ttgtgtttag tgacgtggac    600
   ctcattccaa tgaatgacca taatgcgtac aggtgttttt cacagccacg gcacatttcc    660
   gttgcaatgg ataagtttgg attcagccta ccttatgttc agtattttgg aggtgtctct    720
   gctctaagta aacaacagtt tctaaccatc aatggatttc ctaataatta ttggggctgg    780
   ggaggagaag atgatgacat ttttaacaga ttagttttta gaggcatgtc tatatctcgc    840
   ccaaatgctg tggtcgggag gtgtcgcatg atccgccact caagagacaa gaaaaatgaa    900
   cccaatcctc agaggtttga ccgaattgca cacacaaagg agacaatgct ctctgatggt    960
   ttgaactcac tcacctacca ggtgctggat gtacagagat acccattgta tacccaaatc   1020
   acagtggaca tcgggacacc gagctag                                       1047
<210> 7
   <211> 1801
   <212> ADN
   <213> Homo sapiens
<220>
   <223> alpha-1,6-fucosyltransférase FUT8
<400> 7
   caggactcca gggaagtgag ttgaaaatct gaaaatgcgg ccatggactg gttcctggcg     60
   ttggattatg ctcattcttt ttgcctgggg gaccttgctg ttttatatag gtggtcactt    120
   ggtacgagat aatgaccatc ctgatcactc tagccgagaa ctgtccaaga ttctggcaaa    180
   gcttgaacgc ttaaaacagc agaatgaaga cttgaggcga atggccgaat ctctccggat    240
   accagaaggc cctattgatc aggggccagc tataggaaga gtacgcgttt tagaagagca    300
   gcttgttaag gccaaagaac agattgaaaa ttacaagaaa cagaccagaa atggtctggg    360
   gaaggatcat gaaatcctga ggaggaggat tgaaaatgga gctaaagagc tctggttttt    420
   cctacagagt gaattgaaga aattaaagaa cttagaagga aatgaactcc aaagacatgc    480
   agatgaattt cttttggatt taggacatca tgaaaggtct ataatgacgg atctatacta    540
   cctcagtcag acagatggag caggtgattg gcgggaaaaa gaggccaaag atctgacaga    600
   actggttcag cggagaataa catatcttca gaatcccaag gactgcagca aagccaaaaa    660
   gctggtgtgt aatatcaaca aaggctgtgg ctatggctgt cagctccatc atgtggtcta    720
   ctgcttcatg attgcatatg gcacccagcg aacactcatc ttggaatctc agaattggcg    780
   ctatgctact ggtggatggg agactgtatt taggcctgta agtgagacat gcacagacag    840
   atctggcatc tccactggac actggtcagg tgaagtgaag gacaaaaatg ttcaagtggt    900
   cgagcttccc attgtagaca gtcttcatcc ccgtcctcca tatttaccct tggctgtacc    960
   agaagacctc gcagatcgac ttgtacgagt gcatggtgac cctgcagtgt ggtgggtgtc   1020
   tcagtttgtc aaatacttga tccgcccaca gccttggcta gaaaaagaaa tagaagaagc   1080
   caccaagaag cttggcttca aacatccagt tattggagtc catgtcagac gcacagacaa   1140
   agtgggaaca gaagctgcct tccatcccat tgaagagtac atggtgcatg ttgaagaaca   1200
   ttttcagctt cttgcacgca gaatgcaagt ggacaaaaaa agagtgtatt tggccacaga   1260
   tgacccttct ttattaaagg aggcaaaaac aaagtacccc aattatgaat ttattagtga   1320
   taactctatt tcctggtcag ctggactgca caatcgatac acagaaaatt cacttcgtgg   1380
   agtgatcctg gatatacatt ttctctctca ggcagacttc ctagtgtgta ctttttcatc   1440
   ccaggtctgt cgagttgctt atgaaattat gcaaacacta catcctgatg cctctgcaaa   1500
   cttccattct ttagatgaca tctactattt tgggggccag aatgcccaca atcaaattgc   1560
   catttatgct caccaacccc gaactgcaga tgaaattccc atggaacctg gagatatcat   1620
   tggtgtggct ggaaatcatt gggatggcta ttctaaaggt gtcaacagga aattgggaag   1680
   gacgggccta tatccctcct acaaagttcg agagaagata gaaacggtca agtaccccac   1740
   atatcctgag gctgagaaat aaagctcaga tggaagagat aaacgaccaa actcagttcg   1800
   a                                                                   1801
<210> 8
   <211> 1136
   <212> ADN
   <213> Homo sapiens
<220>
   <223> Transporteur de GDP-fucose
<400> 8
   tgacccagct cctctgctac catgaatagg gcccctctga agcggtccag gatcctgcac     60
   atggcgctga ccggggcctc agacccctct gcagaggcag aggccaacgg ggagaagccc    120
   tttctgctgc gggcattgca gatcgcgctg gtggtctccc tctactgggt cacctccatc    180
   tccatggtgt tccttaataa gtacctgctg gacagcccct ccctgcggct ggacaccccc    240
   atcttcgtca ccttctacca gtgcctggtg accacgctgc tgtgcaaagg cctcagcgct    300
   ctggccgcct gctgccctgg tgccgtggac ttccccagct tgcgcctgga cctcagggtg    360
   gcccgcagcg tcctgcccct gtcggtggtc ttcatcggca tgatcacctt caataacctc    420
   tgcctcaagt acgtcggtgt ggccttctac aatgtgggcc gctcactcac caccgtcttc    480
   aacgtgctgc tctcctacct gctgctcaag cagaccacct ccttctatgc cctgctcacc    540
   tgcggtatca tcatcggggg cttctggctt ggtgtggacc aggagggggc agaaggcacc    600
   ctgtcgtggc tgggcaccgt cttcggcgtg ctggctagcc tctgtgtctc gctcaacgcc    660
   atctacacca cgaaggtgct cccggcggtg gacggcagca tctggcgcct gactttctac    720
   aacaacgtca acgcctgcat cctcttcctg cccctgctcc tgctgctcgg ggagcttcag    780
   gccctgcgtg actttgccca gctgggcagt gcccacttct gggggatgat gacgctgggc    840
   ggcctgtttg gctttgccat cggctacgtg acaggactgc agatcaagtt caccagtccg    900
   ctgacccaca atgtgtcggg cacggccaag gcctgtgccc agacagtgct ggccgtgctc    960
   tactacgagg agaccaagag cttcctctgg tggacgagca acatgatggt gctgggcggc   1020
   tcctccgcct acacctgggt caggggctgg gagatgaaga agactccgga ggagcccagc   1080
   cccaaagaca gcgagaagag cgccatgggg gtgtgagcac cacaggcacc ctggat       1136
<210> 9
   <211> 268
   <212> ADN
   <213> herpes simplex virus
<220>
   <223> Promoteur de la thymidine kinase
<400> 9
   gcagtgtggt tttgcaagag gaagcaaaaa gcctctccac ccaggcctgg aatgtttcca     60
   cccaatgtcg agcagtgtgg ttttgcaaga ggaagcaaaa agcctctcca cccaggcctg    120
   gaatgtttcc acccaatgtc gagcaaaccc cgcccagcgt cttgtcattg gcgaattcga    180
   acacgcagat gcagtcgggg cggcgcggtc ccaggtccac ttcgcatatt aaggtgacgc    240
   gtgtggcctc gaacaccgag cgaccctg                                       268
<210> 10
   <211> 990
   <212> ADN
   <213> Homo sapiens
<220>
   <223> Sialyltransférase (NM_006278)
<400> 10
   atggtcagca agtcccgctg gaagctcctg gccatgttgg ctctggtcct ggtcgtcatg     60
   gtgtggtatt ccatctcccg ggaagacagt ttttattttc ccatcccaga gaagaaggag    120
   ccgtgcctcc agggtgaggc agagagcaag gcctctaagc tctttggcaa ctactcccgg    180
   gatcagccca tcttcctgcg gcttgaggat tatttctggg tcaagacgcc atctgcttac    240
   gagctgccct atgggaccaa ggggagtgag gatctgctcc tccgggtgct agccatcacc    300
   agctcctcca tccccaagaa catccagagc ctcaggtgcc gccgctgtgt ggtcgtgggg    360
   aacgggcacc ggctgcggaa cagctcactg ggagatgcca tcaacaagta cgatgtggtc    420
   atcagattga acaatgcccc agtggctggc tatgagggtg acgtgggctc caagaccacc    480
   atgcgtctct tctaccctga atctgcccac ttcgacccca aagtagaaaa caacccagac    540
   acactcctcg tcctggtagc tttcaaggca atggacttcc actggattga gaccatcctg    600
   agtgataaga agcgggtgcg aaagggtttc tggaaacagc ctcccctcat ctgggatgtc    660
   aatcctaaac agattcggat tctcaacccc ttcttcatgg agattgcagc tgacaaactg    720
   ctgagcctgc caatgcaaca gccacggaag attaagcaga agcccaccac gggcctgttg    780
   gccatcacgc tggccctcca cctctgtgac ttggtgcaca ttgccggctt tggctaccca    840
   gacgcctaca acaagaagca gaccattcac tactatgagc agatcacgct caagtccatg    900
   gcggggtcag gccataatgt ctcccaagag gccctggcca ttaagcggat gctggagatg    960
   ggagctatca agaacctcac gtccttctga                                     990
<210> 11
   <211> 681
   <212> ADN
   <213> Séquence artificielle
<220>
   <223> Séquence de l'EPO modifiée
<400> 11
   atgggggtgc acgaatgtcc tgcctggctg tggcttctcc tgtccctgct gtcgctccct     60
   ctgggcctcc cagtcctggg cgccccacca cgcctcatct gtgacagccg agtcctggag    120
   aggtacctct tggaggccaa ggaggccgag aatatcacga cgggctgtgc tgaacactgc    180
   agcttgaatg agaatatcac tgtcccagac accaaagtta atttctatgc ctggaagagg    240
   atggaggtcg ggcagcaggc cgtagaagtc tggcagggcc tggccctgct gtcggaagct    300
   gtcctgcggg gccaggccct gttggtcaac tcttcccagc cgtgggagcc cctgcagctg    360
   catgtggata aagccgtcag tggccttcgc agcctcacca ctctgcttcg ggctctggga    420
   gcccagaagg aagccatctc ccctccagat gcagcctcag ctgctccgct ccgaacaatc    480
   actgctgaca ctttccgcaa actcttccga gtctactcca atttcctccg gggaaagctg    540
   aagctgtaca caggggaggc ctgcaggaca ggcgacagaa agggcgagct tcgaggtcac    600
   ccattcgaag gtaagcctat ccctaaccct ctcctcggtc tcgattctac gcgtaccggt    660
   catcatcacc atcaccattg a                                              681
<210> 12
   <211> 225
   <212> PRT
   <213> Séquence artificielle
<220>
   <223> EPO séquencée dans le plasmide d'expression
<400> 12
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Pro Trp Glu Pro Gln Leu His Val Asp Lys Ala Val Ser Gly Leu
           115                 120                 125
   Arg Ser Leu Thr Thr Leu Leu Arg Ala Leu Gly Ala Gln Lys Glu Ala
       130                 135                 140
   Ile Ser Pro Pro Asp Ala Ala Ser Ala Ala Pro Leu Arg Thr Ile Thr
   145                 150                 155                 160
   Ala Asp Thr Phe Arg Lys Leu Phe Arg Val Tyr Ser Asn Phe Leu Arg
                   165                 170                 175
   Gly Lys Leu Lys Leu Tyr Thr Gly Glu Ala Cys Arg Thr Gly Asp Arg
               180                 185                 190
   Lys Gly Glu Leu Arg Gly His Pro Phe Glu Gly Lys Pro Ile Pro Asn
           195                 200                 205
   Pro Leu Leu Gly Leu Asp Ser Thr Arg Thr Gly His His His His His
               210                 215                 220
   His
   225
<210> 13
   <211> 51
   <212> ADN
   <213> Séquence artificielle
<220>
   <223> Partie cytoplasmique de mmn9
<400> 13
   atgtcacttt ctcttgtatc gtaccgccta agaaagaacc cgtgggttaa c 51
<210> 14
   <211> 246
   <212> ADN
   <213> Saccharomyces cerevisiae
<400> 14
   atggccctct ttctcagtaa gagactgttg agatttaccg tcattgcagg tgcggttatt    60
   gttctcctcc taacattgaa ttccaacagt agaactcagc aatatattcc gagttccatc   120
   tccgctgcat ttgattttac ctcaggatct atatcccctg aacaacaagt catctctgag   180
   gaaaatgatg ctaaaaaatt agagcaaagt gctctgaatt cagaggcaag cgaagactcc   240
   gaagcc                                                              246
<210> 15
   <211> 274
   <212> ADN
   <213> Séquence artificielle
<220>
   <223> CYC1 terminateur seul
<400> 15
   gaattggtcg atcaggtatt catgtaatta gttatgtcac gcttacattc acgccctccc      60
   cccacatccg ctctaaccga aaaggaagga gttagacaac ctgaagtcta ggtccctatt     120
   tattttttta tagttatgtt agtattaaga acgttattta tatttcaaat ttttcttttt     180
   tttctgtaca gacgcgtgta cgcatgtaac attatactga aaaccttgct tgagaaggtt     240
   ttgggacgct cgaaggcttt aatttgcaag ctgc                                 274
<210> 16
   <211> 679
   <212> ADN
   <213> Saccharomyces cerevisiae
<220>
   <223> Promoteur pGAP
<400> 16
   tcgagtttat cattatcaat actcgccatt tcaaagaata cgtaaataat taatagtagt      60
   gattttccta actttattta gtcaaaaaat tagcctttta attctgctgt aacccgtaca     120
   tgccaaaata gggggcgggt tacacagaat atataacact gatggtgctt gggtgaacag     180
   gtttattcct ggcatccact aaatataatg gagcccgctt tttaagctgg catccagaaa     240
   aaaaaagaat cccagcacca aaatattgtt ttcttcacca accatcagtt cataggtcca     300
   ttctcttagc gcaactacag agaacagggc acaaacaggc aaaaaacggg cacaacctca     360
   atggagtgat gcaacctgcc tggagtaaat gatgacacaa ggcaattgac ccacgcatgt     420
   atctatctca ttttcttaca ccttctatta ccttctgctc tctctgattt ggaaaaagct     480
   gaaaaaaaag gttgaaacca gttccctgaa attattcccc tacttgacta ataagtatat     540
   aaagacggta ggtattgatt gtaattctgt aaatctattt cttaaacttc ttaaattcta     600
   cttttatagt tagtcttttt tttagtttta aaacaccaag aacttagttt cgaataaaca     660
   cacataaata aacaaaatg                                                  679
<210> 17
   <211> 451
   <212> ADN
   <213> Saccharomyces cerevisiae
<220>
   <223> Promoteur pGAL1
<400> 17
   acggattaga agccgccgag cgggtgacag ccctccgaag gaagactctc ctccgtgcgt      60
   cctcgtcttc accggtcgcg ttcctgaaac gcagatgtgc ctcgcgccgc actgctccga     120
   acaataaaga ttctacaata ctagctttta tggttatgaa gaggaaaaat tggcagtaac     180
   ctggccccac aaaccttcaa atgaacgaat caaattaaca accataggat gataatgcga     240
   ttagtttttt agccttattt ctggggtaat taatcagcga agcgatgatt tttgatctat     300
   taacagatat ataaatgcaa aaactgcata accactttaa ctaatacttt caacattttc     360
   ggtttgtatt acttcttatt caaatgtaat aaaagtatca acaaaaaatt gttaatatac     420
   ctctatactt taacgtcaag gagaaaaaac c                                    451
<210> 18
   <211> 603
   <212> ADN
   <213> Saccharomyces cerevisiae
<220>
   <223> Promoteur PGK
<400> 18
   cccaagctta cctgctgcgc attgttttat atttgttgta aaaagtagat aattacttcc    60
   ttgatgatct gtaaaaaaga gaaaaagaaa gcatctaaga acttgaaaaa ctacgaatta   120
   gaaaagacca aatatgtatt tcttgcattg accaatttat gcaagtttat atatatgtaa   180
   atgtaagttt cacgaggttc tactaaacta aaccaccccc ttggttagaa gaaaagagtg   240
   tgtgagaaca ggctgttgtt gtcacacgat tcggacaatt ctgtttgaaa gagagagagt   300
   aacagtacga tcgaacgaac tttgctctgg agatcacagt gggcatcata gcatgtggta   360
   ctaaaccctt tcccgccatt ccagaacctt cgattgcttg ttacaaaacc tgtgagccgt   420
   cgctaggacc ttgttgtgtg acgaaattgg aagctgcaat caataggaag acaggaagtc   480
   gagcgtgtct gggttttttc agttttgttc tttttgcaaa caaatcacga gcgacggtaa   540
   tttctttctc gataagaggc cacgtgcttt atgagggtaa catcaattca agatctgaat   600
   tcc                                                                 603
<210> 19
   <211> 409
   <212> ADN
   <213> Saccharomyces cerevisiae
<220>
   <223> Promoteur TEF
<400> 19
   cccacacacc atagcttcaa aatgtttcta ctcctttttt actcttccag attttctcgg    60
   actccgcgca tcgccgtacc acttcaaaac acccaagcac agcatactaa attttccctc   120
   tttcttcctc tagggtgtcg ttaattaccc gtactaaagg tttggaaaag aaaaaagaga   180
   ccgcctcgtt tctttttctt cgtcgaaaaa ggcaataaaa atttttatca cgtttctttt   240
   tcttgaaatt ttttttttta gtttttttct ctttcagtga cctccattga tatttaagtt   300
   aataaacggt cttcaatttc tcaagtttca gtttcatttt tcttgttcta ttacaacttt   360
   ttttacttct tgttcattag aaagaaagca tagcaatcta atctaaggg               409
<210> 20
   <211> 936
   <212> ADN
   <213> Saccharomyces cerevisiae
<220>
   <223> Promoteur PMA1
<400> 20
   aagcttcctg aaacggagaa acataaacag gcattgctgg gatcacccat acatcactct     60
   gttttgcctg accttttccg gtaatttgaa aacaaacccg gtctcgaagc ggagatccgg    120
   cgataattac cgcagaaata aacccataca cgagacgtag aaccagccgc acatggccgg    180
   agaaactcct gcgagaattt cgtaaactcg cgcgcattgc atctgtattt cctaatgcgg    240
   cacttccagg cctcgagacc tctgacatgc ttttgacagg aatagacatt ttcagaatgt    300
   tatccatatg cctttcgggt ttttttcctt ccttttccat catgaaaaat ctctcgagac    360
   cgtttatcca ttgctttttt gttgtctttt tccctcgttc acagaaagtc tgaagaagct    420
   atagtagaac tatgagcttt ttttgtttct gttttccttt tttttttttt tacctctgtg    480
   gaaattgtta ctctcacact ctttagttcg tttgtttgtt ttgtttattc caattatgac    540
   cggtgacgaa acgtggtcga tggtgggtac cgcttatgct cccctccatt agtttcgatt    600
   atataaaaag gccaaatatt gtattatttt caaatgtcct atcattatcg tctaacatct    660
   aatttctctt aaattttttc tctttctttc ctataacacc aatagtgaaa atcttttttt    720
   cttctatatc tacaaaaact ttttttttct atcaacctcg ttgataaatt ttttctttaa    780
   caatcgttaa taattaatta attggaaaat aaccattttt tctctctttt atacacacat    840
   tcaaaagaaa gaaaaaaaat ataccccagc tagttaaaga aaatcattga aaagaataag    900
   aagataagaa agatttaatt atcaaacaat atcaat                              936
<210> 21
   <211> 504
   <212> ADN
   <213> Cauliflower mosaic virus
<400> 21
   caacagaact cgccgtgaag actggcgaac agttcataca gagtctttta cgactcaatg     60
   acaagaagaa aatcttcgtc aacatggtgg agcacgacac tctggtctac tccaaaaatg    120
   tcaaagatac agtctcagaa gaccaaaggg ctattgagac ttttcaacaa aggataattt    180
   cgggaaacct cctcggattc cattgcccag ctatctgtca cttcatcgaa aggacagtag    240
   aaaaggaagg tggctcctac aaatgccatc attgcgataa aggaaaggct atcattcaag    300
   atctgcctct gccgacagtg gtcccaaaga tggaccccca cccacgagga gcatcgtgga    360
   aaaagaagac gttccaacca cgtcttcaaa gcaagtggat tgatgtgaca tctccactga    420
   cgtaagggat gacgcacaat cccactatcc ttcgcaagac ccttcctcta tataaggaag    480
   ttcatttcat ttggagagga cacg                                           504
<210> 22
   <211> 623
   <212> ADN
   <213> Saccharomyces cerevisiae
<220>
   <223> Promoteur Pho5
<400> 22
   gatccgaaag ttgtattcaa caagaatgcg caaatatgtc aacgtatttg gaagtcatct     60
   tatgtgcgct gctttaatgt tttctcatgt aagcggacgt cgtctataaa cttcaaacga    120
   aggtaaaagg ttcatagcgc tttttctttg tctgcacaaa gaaatatata ttaaattagc    180
   acgttttcgc atagaacgca actgcacaat gccaaaaaaa gtaaaagtga ttaaaagagt    240
   taattgaata ggcaatctct aaatgaatcg atacaacctt ggcactcaca cgtgggacta    300
   gcacagacta aatttatgat tctggtccct gttttcgaag agatcgcaca tgccaaatta    360
   tcaaattggt caccttactt ggcaaggcat atacccattt gggataaggg taaacatctt    420
   tgaattgtcg aaatgaaacg tatataagcg ctgatgtttt gctaagtcga ggttagtatg    480
   gcttcatctc tcatgagaat aagaacaaca acaaatagag caagcaaatt cgagattacc    540
   aatgtttaaa tctgttgttt attcaatttt agccgcttct ttggccaatg caggtaccat    600
   tcccttaggc aaactagccg atg                                            623
<210> 23
   <211> 1501
   <212> ADN
   <213> Saccharomyces cerevisiae
<220>
   <223> Promoteur d'une protéine de liaison à l'actine
<400> 23
   gaattcggaa gactcagact tcccaacagg cagaaaattc agaagtcata aaggaccttt     60
   acgagtacct ctgtaacgta cgtgtacata aaagctacga ggatgattct gggttgtggt    120
   tcgacatctc gcagggcacc cactcagggg gatcttccga tgattattcg ataatggact    180
   ataaactcgg atttgtcaag ggccaggcca agtcacagaa gtcatatatg cgcccgttct    240
   taaacagcga tccaccgagg aactatactc gctacagtcg aaactaccgg aatacctctt    300
   cgaaacgttg agtttccccc tctcgtcgct aaaccaattc tataacaaaa tcgctaaaag    360
   cctgaataag aaaagagaga aaaaagatga aaccgagtaa gctgctacat aatgtctata    420
   tatctacaca taaaattccg attattcctt tgcatacctg atttgcccct cagaatccac    480
   aaccagactt ttcaagaagg tcttttttgc ccctttatcc ttcatggttt tcaaattttg    540
   tacaacgact tgcccttgtg aagctcgata ccgttcgagg tcttttccac tttctctgcc    600
   ttcttgatca caggcttctc cactttcttt gcaggagttg ccactgctgg tgctggtgct    660
   gcttctgctg ctgcttctgc tgctgcttct gcttctgctt cttgttgccc ttgtacaacg    720
   ctttctggta cttctcgtcc tcgctgatgc aagacgtgtg attcttgtaa ctcacgccat    780
   cttcaaacgt cttggagcaa tctatgcatg tatagtacgc gttaggacat ctataataat    840
   gcttttcggt attcttcttg ggcacagtat cattgcacac ctcacagttg aacgtaacca    900
   tcctgtaata acaaatattt cttgactgag accgtttgct gttgtataca gaatactctt    960
   agagctcatc gcaagttaaa aattttcaat tttttttcac tttttcccgt caaggcaaaa    1020
   agcaaccaaa agagaatgaa cctttatttt tgatttattt attatgagat gctgctagtc   1080
   cactcatctg catcaatgta gtagtcacaa agctaatatt tagacgttac tttgatatct   1140
   ctgtccatga gagcacttat tttaggaagt taaatgagac agtcaatagt tcacaatatc   1200
   ccgtcagcaa tggagggaaa aggcattcct tttccatagg atttaatcgt ttttcaagca    1260
   tcatacgccc tcgaggaact cttgttttcg ctttactatg caaccattga tgtatttctg   1320
   tataaatgtg cgtcacgtgg ccttgtgtct cttatttcca cttgtttttt cacaatgcgg   1380
   aaaacctcga ttaaagtaga aaaaaaggat ataataggag tataccatat tggatagttc   1440
   aatctataaa caaacaatcg cataaccgca cgtatataca cgcacacacc tatcaatcac   1500
   a                                                                    1501
<210> 24
   <211> 384
   <212> ADN
   <213> Saccharomyces cerevisiae
<220>
   <223> Promoteur putatif CYC1
<400> 24
   cccgggagca agatcaagat gttttcaccg atctttccgg tctctttggc cggggtttac     60
   ggacgatgac cgaagaccaa gcgccagctc atttggcgag cgttggttgg tggatcaagc    120
   ccacgcgtag gcaatcctcg agcagatccg ccaggcgtgt atatagcgtg gatggccagg    180
   caactttagt gctgacacat acaggcatat atatatgtgt gcgacgacac atgatcatat    240
   ggcatgcatg tgctctgtat gtatataaaa ctcttgtttt cttcttttct ctaaatattc    300
   tttccttata cattaggtcc tttgtagcat aaattactat acttctatag acacgcaaac    360
   acaaatacac acactaaatt aata                                           384
<210> 25
   <211> 444
   <212> ADN
   <213> Schizosaccharomyces pombe
<220>
   <223> Promoteur CYC1
<400> 25
   atcataccaa gaccaaccac acatgctgaa gatgcattgc atgccacgaa atcattgcat     60
   atcagccaat tccagttttc caacgattac tgcactctaa cgatatctat tttccttatt    120
   ttcagacacg gtatacaacc ttaattatgt ttaatacggc tgctcgtcat caccaaatcc    180
   tgtttttact ttaacgaagg ttatcgcacg caaaaaaggt aaacattgga ttggctcgcc    240
   atgtcattcc gcggagagct catgaaccaa tgaaataagg gcgaaaaaat aaatttaaag    300
   gtcgacttcc accgcatccc aacttactct caacgtacat cttctcacta acttcgcggt    360
   gaatttgtgg tttttaaatc ttcttctcta caaatactaa actcttaagc ctatttcctt    420
   tccttagcaa tttattaatt caaa                                           444
<210> 26
   <211> 159
   <212> ADN
   <213> Saccharomyces cerevisiae
<220>
   <223> promoteur ADH2
<400> 26
   cctatcacat ataaatagag tgccagtagc gacttttttc acactcgaga tactcttact     60
   actgctctct tgttgttttt atcacttctt gtttcttctt ggtaaataga atatcaagct    120
   acaaaaagca tacaatcaac tatcaactat taactatat                           159
<210> 27
   <211> 1617
   <212> ADN
   <213> Mus musculus
<220>
   <223> beta-1,4 N-acetylglucosaminyltransferase III
<400> 27
   atgaagatga gacgctacaa gctctttctc atgttctgta tggctggcct gtgcctcata     60
   tccttcctgc acttctttaa gaccttatcc tatgtcacct tcccgagaga actggcctcc    120
   ctcagcccta acctcgtatc cagcttcttc tggaacaatg cccctgtcac tccccaggcc    180
   agtccggagc cgggtggccc cgacctattg cggacacccc tctactccca ctctcccctg    240
   ctccagccac tgtccccgag caaggccaca gaggaactgc accgggtgga cttcgtgttg    300
   ccggaggaca ccacggagta ttttgtgcgc accaaagctg gtggtgtgtg cttcaaacca    360
   ggtaccagga tgctggagaa accttcgcca gggcggacag aggagaagcc cgaagtgtct    420
   gagggctcct cagcccgggg acctgctcgg aggcccatga ggcacgtgtt gagtacgcgg    480
   gagcgcctgg gcagccgggg cactaggcgc aagtgggttg agtgtgtgtg cctgccaggc    540
   tggcacgggc ccagttgcgg ggtgcccacg gtggtgcagt attccaacct gcccaccaag    600
   gaacgcctgg tacccaggga ggtaccgagg cgggttatca acgccatcaa catcaaccac    660
   gagttcgacc tgctggatgt gcgtttccat gagctgggag atgttgtgga cgccttcgtg    720
   gtctgtgaat ctaatttcac cgcctacggg gagcctcggc cgctcaagtt ccgagagatg    780
   ctgaccaatg gcaccttcga gtacatccgc cacaaggtgc tctatgtctt cctggaccat    840
   ttcccacctg gtggccgtca ggacggctgg attgcggatg actacctgcg caccttcctc     900
   acccaggatg gcgtctcccg cctgcgcaac ctgcggcccg atgacgtctt tatcatcgac     960
   gatgcggacg agatccctgc gcgtgatggt gtgctgttcc tcaaactcta cgatggctgg    1020
   acagagccct tcgccttcca catgcggaag tccctgtatg gtttcttctg gaagcagccg    1080
   ggcacactgg aggtggtgtc aggctgcacc atggacatgc tgcaggccgt gtatgggctg    1140
   gatggcatcc gcctgcgccg ccgccagtac tacaccatgc ccaacttccg gcagtatgag    1200
   aaccgcaccg gccacatcct agtgcagtgg tctctcggca gccccctgca cttcgcgggc    1260
   tggcattgct cctggtgctt cacacccgag ggcatctact ttaaactcgt gtcagcccag    1320
   aatggcgact tcccccgctg gggtgactat gaggacaaga gggacctcaa ttacatccgc    1380
   agcttgatcc gcactggggg atggttcgac ggaacgcagc aggagtaccc tcctgcggac    1440
   cccagtgagc acatgtatgc tcctaaatac ctgctcaaga actatgacca gttccgctac    1500
   ttgctggaaa atccctaccg ggagcccaag agcactgaag agggtgggcg ccggaaccag    1560
   ggctcagatg gaaggccatc tgctgtcagg ggcaagttgg atacagtgga gggctag       1617